# EUROPEAN PATENT SPECIFICATION

(11) **EP 3 416 673 B1**
(45) Date of publication and mention of the grant of the patent: **02.11.2022**
(21) Application number: 17708103.1
(22) Date of filing: 16.02.2017
(51) Int. Cl.: A61K 38/16, A61P 9/10, A61P 25/00

(54) **MODULATION OF HYPOXIA ASSOCIATED WITH STROKE**
MODULATION VON SCHLAGANFALL-ASSOZIIERTER HYPOXIE
MODULATION DE L'HYPOXIE ASSOCIÉE À UN ACCIDENT VASCULAIRE CÉRÉBRAL

(30) Priority: 16.02.2016 US 201662296009 P
(43) Date of publication of application: 26.12.2018
(73) Proprietor: Omniox, Inc., San Carlos, CA 94070-2727 (US)
(72) Inventor: LE MOAN, Natacha, San Carlos CA 94070-2727 (US); KRTOLICA, Ana, San Carlos CA 94070-2727 (US); LEUNG, Philberta, San Carlos CA 94070-2727 (US); CARY, Stephen, P.L., San Carlos CA 94070-2727 (US)
(74) Representative: Hoffmann Eitle
(86) International application number: PCT/US2017/018233
(87) International publication number: WO 2017/143104

(56) References cited:
- WO-A1-2014/107171
- WO-A2-2007/139767
- US-A1- 2015 273 024
- KRTOLICA ANA ET AL: "OMX-4.80P, a novel H-NOX oxygen carrier that oxygenates hypoxic tumors in multiple tumor models and canine cancer patients, downregulates HIF-1 pathway and increases response to radiation therapy leading to cures", CANCER RESEARCH, AMERICAN ASSOCIATION FOR CANCER RESEARCH, US, vol. 75, no. Supplement 15, 1 August 2015 (2015-08-01), pages 1-5, XP008180963, ISSN: 0008-5472, DOI: 10.1158/1538-7445.AM2015-3003
- NATACHA LE MOAN: "A New Paradigm in Protecting Ischemic Brain: Preserving the Neurovascular Unit Before Reperfusion", 1 January 2017 (2017-01-01), NEUROPROTECTIVE THERAPY FOR STROKE AND ISCHEMIC DISEASE,, PAGE(S) 641 - 664, XP009194076, ISBN: 978-3-319-45344-6 the whole document

## Description

### CROSS-REFERENCE TO RELATED APPLICATIONS

This application claims the benefit of U.S. Provisional Patent Application No. 62/296,009, filed February 16, 2016.

### SUBMISSION OF SEQUENCE LISTING ON ASCII TEXT FILE

The content of the following submission on ASCII text file is in its entirety: a computer readable form (CRF) of the Sequence Listing (file name: 627042001040SeqList.txt, date recorded: February 16, 2017, size: 9 KB).

### STATEMENT REGARDING FEDERALLY SPONSORED RESEARCH OR DEVELOPMENT

This work was supported by 1R43NS076272-01A1. The U.S. government has rights in any patent issuing on this application.

### TECHNICAL FIELD

This application pertains to methods to modulate hypoxia associated with injury to organs using H-NOX proteins.

### BACKGROUND OF THE INVENTION

H-NOX proteins (named for Heme-Nitric oxide and OXygen binding domain) are members of a highly-conserved, well-characterized family of hemoproteins (Iyer, LM et al. (2003) BMC Genomics 4(1):5; Karow, DS et al. (2004) Biochemistry 43(31):10203-10211; Boon, EM et al. (2005) Nature Chem. Biol. 1:53-59; Boon, EM et al. (2005) Curr. Opin. Chem. Biol. 9(5):441-446; Boon, EM et al. (2005) J. Inorg. Biochem. 99(4):892-902; Cary, SP et al. (2005) Proc Natl Acad Sci USA 102(37): 13064-9; Karow DS et al. (2005) Biochemistry 44(49):16266-74; Cary, SP et al. (2006) Trends Biochem Sci 31(4):231-9; Boon, EM et al. (2006) J Biol Chem 281(31):21892-902; Winger, JA et al. (2007) J Biol Chem. 282(2):897-907). H-NOX proteins are nitric-oxide-neutral, unlike previous hemoglobin-based oxygen carriers, H-NOX do not scavenge circulating nitric oxide, and thus are not associated with hypertensive or renal side effects. The intrinsic low NO reactivity (and high NO stability) makes wild-type and mutant H-NOX proteins desirable blood substitutes because of the lower probability of inactivation of H-NOX proteins by endogenous NO and the lower probability of scavenging of endogenous NO by H-NOX proteins. Importantly, the presence of a distal pocket tyrosine in some H-NOX proteins (Pellicena, P. et al. (2004) Proc Natl. Acad Sci USA 101(35):12854-12859) is suggestive of undesirable, high NO reactivity, contraindicating use as a blood substitute. For example, by analogy, a *Mycobacterium tuberculosis* hemoglobin protein, with a structurally analogous distal pocket tyrosine, reacts extremely rapidly with NO, and is used by the Mycobacterium to effectively scavenge and avoid defensive NO produced by an infected host (Ouellet, H. et al. (2002) Proc. Natl. Acad. Sci.U S A 99(9):5902-5907). However, it was surprisingly discovered that H-NOX proteins actually have a much lower NO reactivity than that of hemoglobin making their use as blood substitutes possible.

It was discovered that H-NOX proteins that bind NO but not O₂ can be converted to H-NOX proteins that bind both NO and O₂ by the introduction of a single amino acid mutation (see WO 2007/139791 and WO 2007/139767). Thus, the affinity of H-NOX proteins for O₂ and NO and the ability of H-NOX proteins to discriminate between O₂ and NO ligands can be altered by the introduction of one or more amino acid mutations, allowing H-NOX proteins to be tailored to bind O₂ or NO with desired affinities. Additional mutations can be introduced to further alter the affinity for O₂ and/or NO. The H-NOX protein family can therefore be manipulated to exhibit improved or optimal kinetic and thermodynamic properties for O₂ delivery. For example, mutant H-NOX proteins have been generated with altered dissociation constants and/or off rates for O₂ binding that improve the usefulness of H-NOX proteins for a variety of clinical and industrial applications. H-NOX oxygen binding proteins with different "tuned" oxygen affinities has enabled the construction of a panel of H-NOX oxygen carriers with properties that are acceptable to a wide range of specific hypoxic/ischemic conditions such as cancer and stroke. The ability to tune H-NOX proteins to bind and deliver O₂ is a therapeutic avenue that addresses and overcomes the central shortcomings of current O₂ carriers. Polymeric H-NOX proteins and methods to use polymeric H-NOX proteins are provided by WO 2014/107171 and US 20150273024A1.

After vascular occlusion, there are two major zones of injury: the infarct core that rapidly dies, and the surrounding penumbra, which is hypoxic and at risk for infarction. For example, restoring blood flow by vascular recanalization *(i.e.,* endovascular +/- IV tPA) to prevent the penumbra from infarcting has become the new standard of care for acute ischemic stroke patients presenting with salvageable tissue. However, not all patients benefit from recanalization and the penumbra is still lost to infarction before recanalization can occur. Regardless of successful or partial reperfusion, secondary hypoxic events within the rescued penumbra can cause selective neuronal loss (SNL) that may account for suboptimal clinical recovery. H-NOX proteins may be tuned to release oxygen specifically in hypoxic tissue and reduce hypoxia to prevent unwanted consequences within the reperfused penumbra. What is needed for certain therapeutic uses is an H-NOX that may permeate hypoxic penumbra associated with organ injury (e.g., brain injury) to deliver O₂ and improve function of the organ *(e.g.,* the brain) as well as the overall well-being of the individual with an organ injury.

### BRIEF SUMMARY OF THE INVENTION

The invention is set out in the claims. In some aspects, the disclosure provides methods for treating stroke in an individual, the method comprising administering a therapeutically effective amount of an H-NOX to the individual, wherein the H-NOX comprises H-NOX covalently bound to polyethylene glycol (PEG) and H-NOX that is not covalently bound to polyethylene glycol. In some aspects, the disclosure disclosure provides methods for delivering oxygen to an individual following a stroke, the method comprising administering a therapeutically effective amount of an H-NOX to the individual, wherein N-NOX comprises H-NOX covalently bound to polyethylene glycol and H-NOX that is not covalently bound to PEG. In some embodiments, the stroke is an ischemic stroke or a hemorrhagic stroke.

In some aspects, the disclosure provides methods for treating a transient ischemic attack in an individual, the method comprising administering a therapeutically effective amount of H-NOX to the individual, wherein the H-NOX comprises H-NOX covalently bound to PEG and H-NOX that is not covalently bound to PEG. In some aspects, the disclosure provides methods for delivering oxygen to an individual following a transient ischemic attack, the method comprising administering a therapeutically effective amount of an H-NOX to the individual, wherein the H-NOX comprises H-NOX covalently bound to PEG and H-NOX that is not covalently bound to PEG.

In some aspects, the disclosure provides methods for treating hypoxic brain injury in an individual, the method comprising administering a therapeutically effective amount of an H-NOX to the individual, wherein the H-NOX comprises H-NOX covalently bound to PEG and H-NOX that is not covalently bound to PEG. In some aspects, the disclosure provides methods for delivering oxygen to an individual following a hypoxic brain injury, the method comprising administering a therapeutically effective amount of an H-NOX to the individual, wherein the H-NOX comprises H-NOX covalently bound to PEG and H-NOX that is not covalently bound to PEG.

In some embodiments of the above aspects, the H-NOX delivers O₂ to a hypoxic penumbra associated with the stroke. In some embodiments, the H-NOX delivers O₂ to a hypoxic penumbra associated with the transient ischemic attack. In some embodiments, the H-NOX delivers O₂ to a hypoxic penumbra associated with the hypoxic brain injury. In some embodiments, administration of the H-NOX reduces inflammation in the hypoxic penumbra. In some embodiments, the individual displays sustained hypoperfusion of the brain as a result of the stroke, transient ischemic attack or hypoxic brain injury. In some embodiments, delivery of H-NOX to a site of occlusion or the hypoxic penumbra associated with stroke, transient ischemic attack or hypoxic brain injury reduces the activation of astrocytes, microglia and/or macrophages at the site of occlusion or the penumbra. In some embodiments, delivery of H-NOX to a site of occlusion or the hypoxic penumbra associated with stroke, transient ischemic attack or hypoxic brain injury reduces sensorimotor deficits associated with stroke, hypoxic brain injury or transient ischemic attack.

In some embodiments of the above aspects and embodiments, the H-NOX covalently bound to PEG is administered with the H-NOX that is not covalently bound to PEG. In some embodiments, the ratio of H-NOX covalently bound to PEG to H-NOX not covalently bound to PEG is about 9:1, about 8:2, about 7:3, about 6:4; about 5:5; about 4:6; about 3:7; about 2:8; or about 1:9. In some embodiments, the H-NOX covalently bound to PEG and the H-NOX not covalently bound to PEG are in a composition.

In some embodiments of the above aspects and embodiments, the H-NOX covalently bound to PEG and the H-NOX not covalently bound to PEG of the methods are delivered simultaneously or sequentially. In some embodiments, the H-NOX covalently bound to PEG and the H-NOX not covalently bound to PEG are delivered simultaneously. In some embodiments, the H-NOX covalently bound to PEG and the H-NOX not covalently bound to PEG are delivered sequentially. In some embodiments, the H-NOX covalently bound to PEG is administered before administration of the H-NOX not covalently bound to PEG. In some embodiments, the H-NOX covalently bound to PEG is administered after administration of the H-NOX not covalently bound to PEG. In some embodiments, the H-NOX covalently bound to PEG is administered about 1 hour, about 2 hours, about 4 hours, about 6 hours, about 8 hours, about 10 hours, about12 hours, about 24 hours, or more than about 24 hours after administration of the H-NOX not covalently bound to PEG. In some embodiments, the H-NOX bound to PEG is administered one or more of one hour, one day, two days or three days after administration of H-NOX not bound to PEG. In some embodiments, the H-NOX bound to PEG is administered about one hour, about one day, and about two days after administration of H-NOX not bound to PEG.

In some embodiments of the above aspects and embodiments, the individual of the methods is a mammal. In some embodiments, the mammal is a human.

In some embodiments of the above aspects and embodiments, the H-NOX protein covalently bound to PEG and the H-NOX protein not covalently bound to PEG of the methods are derived from the same H-NOX protein. In some embodiments, the H-NOX protein covalently bound to PEG and the H-NOX protein not covalently bound to PEG are derived from a different H-NOX protein. In some embodiments, the H-NOX protein covalently bound to PEG and/or the H-NOX protein not covalently bound to PEG is a *T. tengcongensis* H-NOX, a *L*. *pneumophilia* 2 H-NOX, a *H. sapiens* β1, a *R. norvegicus* β1, a C. *lupus* H-NOX, *a D.* melanogaster β1, *a D.* melanogaster CG14885-PA, a C. *elegans* GCY-35, a *N. punctiforme* H-NOX, C. *crescentus* H-NOX, a S. *oneidensis* H-NOX, or C. *acetobutylicum* H-NOX.

In some embodiments, the H-NOX protein covalently bound to PEG and/or the H-NOX protein not covalently bound to PEG of the methods comprises a H-NOX domain corresponding to the H-NOX domain of *T. tengcongensis* set forth in SEQ ID NO:2. In some embodiments, the H-NOX protein covalently bound to PEG and/or the H-NOX protein not covalently bound to PEG comprises one or more distal pocket mutations. In some embodiments, the distal pocket mutation is an amino acid substitution at a site corresponding to L144 of *T. tengcongensis* H-NOX. In some embodiments, the H-NOX is a *T. tengcongensis* H-NOX comprising an amino acid substitution at position 144. In some embodiments, the amino acid substitution at position 144 is an L144F substitution.

In some embodiments of the above aspects and embodiments, the H-NOX protein covalently bound to PEG and/or the H-NOX protein not covalently bound to PEG of the methods is a polymeric H-NOX protein. In some embodiments, the polymeric H-NOX protein comprises monomers, wherein the monomers comprise an H-NOX domain and a polymerization domain. In some embodiments, the H-NOX domain is covalently linked to the polymerization domain. In some embodiments, the polymeric H-NOX protein is a trimeric H-NOX protein. In some embodiments, the trimeric H-NOX protein comprises one or more trimerization domains. In some embodiments, the trimeric H-NOX protein comprises three monomers, wherein the monomers comprise an H-NOX domain and a trimerization domain, wherein the trimerization domain is a bacteriophage T4 trimerization domain. In some embodiments, the trimerization domain is a foldon domain. In some embodiments, the foldon domain comprises the amino acid sequence of SEQ ID NO:4. In some embodiments the H-NOX covalently bound to PEG and/or the H-NOX not covalently bound to PEG is a trimeric *T. tengcongensis* L144F H-NOX protein. In some embodiments the H-NOX covalently bound to PEG and/or the H-NOX not covalently bound to PEG is a trimeric *T. tengcongensis* L144F H-NOX protein wherein one, two or all three monomers of the trimer comprise the sequence set forth in SEQ ID NO:8. In some embodiments, the H-NOX protein is fused to an Fc domain of an immunoglobulin.

In some embodiments of the above aspects and embodiments, the disclosure provides methods wherein the O₂ dissociation constant of the H-NOX protein is within 2 orders of magnitude of that of hemoglobin, and wherein the NO reactivity of the H-NOX protein is at least 10-fold lower than that of hemoglobin. In some embodiments, the O₂ dissociation constant of the polymeric H-NOX protein is between about 1 nM and about 1000 nM at 20°C. In some embodiments, the O₂ dissociation constant of the H-NOX protein is between about 1 µM and about 10 µM at 20°C. In some embodiments, the O₂ dissociation constant of the H-NOX protein is between about 10 µM and about 50 µM at 20°C. In some embodiments, the NO reactivity of the H-NOX protein is less than about 700 _{S}⁻¹ at 20°C. In some embodiments, the NO reactivity of the H-NOX protein is at least 100-fold lower than that of hemoglobin. In some embodiments, the NO reactivity of the H-NOX protein is at least 1,000-fold lower than that of hemoglobin. In some embodiments, the k_{off} for oxygen of the H-NOX protein is less than or equal to about 0.65 s⁻¹at 20°C. In some embodiments, the k_{off} for oxygen of the H-NOX protein is between about 0.21 s⁻¹and about 0.65 _{S}⁻¹ at 20°C. In some embodiments, the k_{off} for oxygen of the H-NOX protein is between about 1.35 s⁻¹and about 2.9 _{S}⁻¹ at 20°C. In some embodiments, the rate of heme autoxidation of the H-NOX protein is less than about 1 h⁻¹at 37 °C.

In some embodiments of the above aspects and embodiments, the disclosure provides methods wherein the H-NOX is administered in combination with another therapy. In some embodiments, the other therapy is treatment with tissue plasminogen activator and/or recanalization or a neuroprotectant. In some embodiments, the H-NOX administration is before, during or after the other treatment.

In some aspects, the disclosure provides a composition comprising a therapeutically effective amount of an H-NOX, wherein the H-NOX comprises H-NOX covalently bound to polyethylene glycol (PEG) and H-NOX that is not covalently bound to polyethylene glycol. In some embodiments, the ratio of H-NOX covalently bound to PEG to H-NOX not covalently bound to PEG is about 9:1, about 8:2, about 7:3, about 6:4; about 5:5; about 4:6; about 3:7; about 2:8; or about 1:9.

In some embodiments of the disclosure, the H-NOX protein covalently bound to PEG and the H-NOX protein not covalently bound to PEG of the composition are derived from the same H-NOX protein. In some embodiments! the H-NOX protein covalently bound to PEG and the H-NOX protein not covalently bound to PEG are derived from the different H-NOX protein. In some embodiments of the disclosure, the H-NOX protein covalently bound to PEG and/or the H-NOX protein not covalently bound to PEG is a *T. tengcongensis* H-NOX, a *L. pneumophilia* 2 H-NOX, a *H. sapiens* β1, a *R*. *norvegicus* β1, a *C*. *lupus* H-NOX, a *D.* melanogaster β1, a *D*. melanogaster CG14885-PA, a C. *elegans* GCY-35, a *N*. *punctiforme* H-NOX, *C. crescentus* H-NOX, a S. *oneidensis* H-NOX, or C. *acetobutylicum* H-NOX.

In some embodiments, the H-NOX protein covalently bound to PEG and/or the H-NOX protein not covalently bound to PEG of the composition comprises a H-NOX domain corresponding to the H-NOX domain of T. tengcongensis set forth in SEQ ID NO:2. In some embodiments of the disclosure, the H-NOX protein covalently bound to PEG and/or the H-NOX protein not covalently bound to PEG comprises one or more distal pocket mutations. In some embodiments of the disclosure, the distal pocket mutation is an amino acid substitution at a site corresponding to L144 of *T. tengcongensis* H-NOX. In some embodiments of the disclosure, the H-NOX is a *T. tengcongensis* H-NOX comprising an amino acid substitution at position 144. In some embodiments, the amino acid substitution at position 144 is an L144F substitution.

In some embodiments of the above aspects and embodiments, the H-NOX protein covalently bound to PEG and/or the H-NOX protein not covalently bound to PEG is a polymeric H-NOX protein. In some embodiments of the disclosure, the polymeric H-NOX protein comprises monomers, wherein the monomers comprise an H-NOX domain and a polymerization domain. In some embodiments, the H-NOX domain is covalently linked to the polymerization domain. In some embodiments, the polymeric H-NOX protein is a trimeric H-NOX protein. In some embodiments, the trimeric H-NOX protein comprises one or more trimerization domains. In some embodiments, the trimeric H-NOX protein comprises three monomers, wherein the monomers comprise an H-NOX domain and a trimerization domain, wherein the trimerization domain is a bacteriophage T4 trimerization domain. In some embodiments, the trimerization domain is a foldon domain. In some embodiments, the foldon domain comprises the amino acid sequence of SEQ ID NO:4. In some embodiments the H-NOX covalently bound to PEG and/or the H-NOX not covalently bound to PEG is a trimeric *T. tengcongensis* L144F H-NOX protein. In some embodiments the H-NOX covalently bound to PEG and/or the H-NOX not covalently bound to PEG is a trimeric *T. tengcongensis* L144F H-NOX protein wherein one, two or all three monomers of the trimer comprise the sequence set forth in SEQ ID NO:8. In some embodiments, the H-NOX protein is fused to an Fc domain of an immunoglobulin.

In some embodiments of the above aspects and embodiments, the invention provides compositions as claimed wherein the O₂ dissociation constant of the H-NOX protein is within 2 orders of magnitude of that of hemoglobin, and wherein the NO reactivity of the H-NOX protein is at least 10-fold lower than that of hemoglobin. In some embodiments, the O₂ dissociation constant of the polymeric H-NOX protein is between about 1 nM and about 1000 nM at 20°C. In some embodiments, the O₂ dissociation constant of the H-NOX protein is between about 1 µM and about 10 µM at 20°C. In some embodiments, the O₂ dissociation constant of the H-NOX protein is between about 10 µM and about 50 µM at 20°C. In some embodiments, the NO reactivity of the H-NOX protein is less than about 700 _{S}⁻¹ at 20°C. In some embodiments, the NO reactivity of the H-NOX protein is at least 100-fold lower than that of hemoglobin. In some embodiments, the NO reactivity of the H-NOX protein is at least 1,000-fold lower than that of hemoglobin. In some embodiments, the k_{off} for oxygen of the H-NOX protein is less than or equal to about 0.65 s⁻¹at 20°C. In some embodiments, the k_{off} for oxygen of the H-NOX protein is between about 0.21 s⁻¹and about 0.65 _{S}⁻¹ at 20°C. In some embodiments, the k_{off} for oxygen of the H-NOX protein is between about 1.35 s⁻¹and about 2.9 s⁻¹ at 20°C. In some embodiments, the rate of heme autoxidation of the H-NOX protein is less than about 1 h⁻¹at 37°C

In some embodiments, the invention provides pharmaceutical compositions as claimed comprising H-NOX for the treatment of a stroke, hypoxic brain injury or transient ischemic attack in an individual according to the methods described herein. In some embodiments, the invention provides pharmaceutical compositions comprising H-NOX for the treatment of a stroke, hypoxic brain injury or transient ischemic attack in an individual, wherein the pharmaceutical composition comprises the composition as claimed.

In some embodiments, of the disclosure, provided is the use of a pharmaceutical composition comprising H-NOX for the treatment of a stroke, hypoxic brain injury or transient ischemic attack in an individual, wherein the composition comprises any of the compositions described herein. In some embodiments, the use of a composition comprising H-NOX in the manufacture of a medicament for the treatment of a stroke, hypoxic brain injury or transient ischemic attack in an individual, wherein the composition comprises any of the compositions described herein.

In some embodiments, of the disclosure, provided is kits comprising a therapeutically effective amount of an H-NOX covalently bound to polyethylene glycol (PEG) and a therapeutically effective amount of an H-NOX that is not covalently bound to polyethylene glycol. In some embodiments, the ratio of H-NOX covalently bound to PEG to H-NOX not covalently bound to PEG of the kit is about 9:1, about 8:2, about 7:3, about 6:4; about 5:5; about 4:6; about 3:7; about 2:8; or about 1:9. In some embodiments, the kit is used in a method to delivering oxygen to an individual following a stroke, a transient ischemic attack or a hypoxic brain injury. In some embodiments, the kit is used in a method to treat a stroke, a transient ischemic attack or a hypoxic brain injury in an individual. In some embodiments, the H-NOX delivers O₂ to a hypoxic penumbra associated with the stroke, transient ischemic attack or hypoxic brain injury. In some embodiments, the individual displays sustained hypoperfusion of the brain as a result of the stroke, transient ischemic attack or hypoxic brain injury. In some embodiments, delivery of H-NOX to a site of occlusion or the hypoxic penumbra associated with stroke, transient ischemic attack or hypoxic brain injury reduces the activation of astrocytes, microglia and/or macrophages at the site of occlusion or the penumbra. In some embodiments, delivery of H-NOX to a site of occlusion or the hypoxic penumbra associated with stroke, transient ischemic attack or hypoxic brain injury reduces sensorimotor deficits associated with stroke, hypoxic brain injury or transient ischemic attack. In some embodiments, the H-NOX covalently bound to PEG is administered with the H-NOX that is not covalently bound to PEG.

In some embodiments, the H-NOX of the kit is delivered at a ratio of H-NOX covalently bound to PEG to H-NOX not covalently bound to PEG is about 9:1, about 8:2, about 7:3, about 6:4; about 5:5; about 4:6; about 3:7; about 2:8; or about 1:9. In some embodiments, the H-NOX covalently bound to PEG and the H-NOX not covalently bound to PEG are in a composition

In some embodiments, the H-NOX covalently bound to PEG and the H-NOX not covalently bound to PEG of the kit are delivered simultaneously or sequentially. In some embodiments, the H-NOX covalently bound to PEG and the H-NOX not covalently bound to PEG are delivered simultaneously. In some embodiments, the H-NOX covalently bound to PEG and the H-NOX not covalently bound to PEG are delivered sequentially. In some embodiments, the H-NOX covalently bound to PEG is administered before administration of the H-NOX not covalently bound to PEG. In some embodiments, the H-NOX covalently bound to PEG is administered after administration of the H-NOX not covalently bound to PEG. In some embodiments, the H-NOX covalently bound to PEG is administered about 1hour, about 2 hours, about 4 hours, about 6 hours, about 8 hours, about 10 hours, about 12 hours, about 24 hours, or more than about 24 hours after administration of the H-NOX not covalently bound to PEG. In some embodiments, the H-NOX bound to PEG is administered one or more of one hour, one day, two days or three days after administration of H-NOX not bound to PEG. In some embodiments, the H-NOX bound to PEG is administered about one hour, about one day, and about two days after administration of H-NOX not bound to PEG.

In some embodiments, the kit is for use with an individual wherein the individual is a mammal. In some embodiments, the mammal is a human.

In some embodiments, the H-NOX protein covalently bound to PEG and the H-NOX protein not covalently bound to PEG of the kit are derived from the same H-NOX protein. In some embodiments, the H-NOX protein covalently bound to PEG and the H-NOX protein not covalently bound to PEG are derived from the different H-NOX protein. In some embodiments, of the disclosure the H-NOX protein covalently bound to PEG and/or the H-NOX protein not covalently bound to PEG is a *T. tengcongensis* H-NOX, a *L. pneumophilia* 2 H-NOX, a *H. sapiens* β1, a *R. norvegicus* β1, a C. *lupus* H-NOX, a *D.* melanogaster β1, a *D.* melanogaster CG14885-PA, a C. *elegans* GCY-35, a *N. punctiforme* H-NOX, C. *crescentus* H-NOX, a S. *oneidensis* H-NOX, or *C. acetobutylicum* H-NOX.

In some embodiments, the H-NOX protein covalently bound to PEG and/or the H-NOX protein not covalently bound to PEG of the kit comprises a H-NOX domain corresponding to the H-NOX domain of T. tengcongensis set forth in SEQ ID NO:2. In some embodiments of the disclosure, the H-NOX protein covalently bound to PEG and/or the H-NOX protein not covalently bound to PEG comprises one or more distal pocket mutations. In some embodiments, of the disclosure the distal pocket mutation is an amino acid substitution at a site corresponding to L144 of *T. tengcongensis* H-NOX. In some embodiments of the disclosure, the H-NOX is a *T. tengcongensis* H-NOX comprising an amino acid substitution at position 144. In some embodiments, the amino acid substitution at position 144 is an L144F substitution.

In some embodiments of the above aspects and embodiments, the H-NOX protein covalently bound to PEG and/or the H-NOX protein not covalently bound to PEG of the kit is a polymeric H-NOX protein. In some embodiments, the polymeric H-NOX protein comprises monomers, wherein the monomers comprise an H-NOX domain and a polymerization domain. In some embodiments, the H-NOX domain is covalently linked to the polymerization domain. In some embodiments, the polymeric H-NOX protein is a trimeric H-NOX protein. In some embodiments, the trimeric H-NOX protein comprises one or more trimerization domains. In some embodiments, the trimeric H-NOX protein comprises three monomers, wherein the monomers comprise an H-NOX domain and a trimerization domain, wherein the trimerization domain is a bacteriophage T4 trimerization domain. In some embodiments, the trimerization domain is a foldon domain. In some embodiments, the foldon domain comprises the amino acid sequence of SEQ ID NO:4. In some embodiments the H-NOX covalently bound to PEG and/or the H-NOX not covalently bound to PEG is a trimeric *T. tengcongensis* L144F H-NOX protein. In some embodiments the H-NOX covalently bound to PEG and/or the H-NOX not covalently bound to PEG is a trimeric *T. tengcongensis* L144F H-NOX protein wherein one, two or all three monomers of the trimer comprise the sequence set forth in SEQ ID NO:8. In some embodiments, the H-NOX protein is fused to an Fc domain of an immunoglobulin.

In some embodiments of the above aspects and embodiments, the disclosure provides kits wherein the O₂ dissociation constant of the H-NOX protein is within 2 orders of magnitude of that of hemoglobin, and wherein the NO reactivity of the H-NOX protein is at least 10-fold lower than that of hemoglobin. In some embodiments, the O₂ dissociation constant of the polymeric H-NOX protein is between about 1 nM and about 1000 nM at 20°C. In some embodiments, the O₂ dissociation constant of the H-NOX protein is between about 1 µM and about 10 µM at 20°C. In some embodiments, the O₂ dissociation constant of the H-NOX protein is between about 10 µM and about 50 µM at 20°C. In some embodiments, the NO reactivity of the H-NOX protein is less than about 700 s⁻¹ at 20°C. In some embodiments, the NO reactivity of the H-NOX protein is at least 100-fold lower than that of hemoglobin. In some embodiments, the NO reactivity of the H-NOX protein is at least 1,000-fold lower than that of hemoglobin. In some embodiments, the k_{off} for oxygen of the H-NOX protein is less than or equal to about 0.65 s⁻¹at 20°C. In some embodiments, the k_{off} for oxygen of the H-NOX protein is between about 0.21 s⁻¹and about 0.65 _{S}⁻¹ at 20°C. In some embodiments, the k_{off} for oxygen of the H-NOX protein is between about 1.35 s⁻¹and about 2.9 _{S}⁻¹ at 20°C. In some embodiments, the rate of heme autoxidation of the H-NOX protein is less than about 1 h⁻¹at 37 °C.

In some aspects, the disclosure provides methods for treating tissue hypoxia in an individual comprising administering a therapeutically effective amount of an H-NOX protein to the individual. In some aspects, the disclosure provides method for delivering O₂ to a hypoxic tissue in an individual under hypoxic stress comprising administering a therapeutically effective amount of an H-NOX protein to the individual. In some aspects, the disclosure provides methods for reducing consequences of tissue hypoxia in an individual comprising administering a therapeutically effective amount of an H-NOX protein to the individual. In some embodiments, the consequence of hypoxic stress is loss of cellular function and/or cell death. In some embodiments, the loss of cellular function and/or cell death leads to organ (e.g., brain) and/or body dysfunction.

In some embodiments, the H-NOX protein is administered to the individual by infusion. In some embodiments, the H-NOX is administered at a dose of about 10 mg/kg to about 400 mg/kg. In some embodiments, the H-NOX is delivered in a volume of about 10 ml to about 1 L. In some embodiments, the H-NOX is delivered as a bolus over a period of less than about 30 minutes. In some embodiments, the H-NOX is delivered by infusion over a period of about 30 minutes to about 7 days. In some embodiments, the H-NOX is administered to directly to the hypoxic tissue or is administered systemically (e.g., intravenously). In some embodiments, the H-NOX protein is administered to the individual by a bolus followed by administration to the individual by infusion over a period of about 30 minutes to about 7 days. In some embodiments, the H-NOX protein is administered to the individual by infusion over more than about 1 hour, 3 hours, 6 hours, 12 hours, 1 day, 2 days, 3 days, 4 days, 5 days, 6 days , or 7 days.

In some embodiments, the hypoxic tissue displays hypoperfusion. In some embodiments, the hypoxic tissue is associated with a vascular occlusion or hemorrhages. In some embodiments, administration of the H-NOX protein reduces hypoxia in a hypoxic penumbra associated with an organ injury (e.g., brain injury) or limb injury. In some embodiments, administration of the H-NOX protein reduces consequences of hypoxia in the injured organ (e.g., brain) or limb. In some embodiments, administration of the H-NOX protein reduces cell death and dysfunction associated with hypoxia in the injured organ or limb. In some embodiments, administration of the H-NOX protein reduces inflammation of the hypoxic tissue. In some embodiments, administration of the H-NOX protein reduces inflammation of a hypoxic penumbra associated with an injured organ or limb. In some embodiments, administration of the H-NOX protein reduces activation of inflammatory cells in the injured organ and/or hypoxic penumbra associated with the injured organ or limb. In some embodiments, the inflammatory cells are macrophages, T cells, B cells, NK cells, dendritic cells, neutrophils, monocytes, eosinophils, basophils and/or microglia.

In some embodiments, the organ is an organ of the musculoskeletal system, the respiratory system, the digestive system, the urinary system, the circulatory system, the reproductive system, the nervous system, or is skin or an endocrine gland. In some embodiments, the organ is a brain, a heart, a liver, a kidney, spleen, or a lung. In some embodiments, the hypoxic tissue is a result of a stroke, ischemic stroke, hemorrhagic stroke, emerging stroke, or a transient ischemic attack.

In some embodiments, the individual is a mammal. In some embodiments, the mammal is a human. In some embodiments, the mammal is a pet, a laboratory research animal, or a farm animal. In some embodiments, the pet, research animal or farm animal is a dog, a cat, a horse, a monkey, a rabbit, a rat, a mouse, a guinea pig, a hamster, a pig, or a cow.

In some embodiments of the disclosure, the H-NOX protein is a *T. tengcongensis* H-NOX, a *L. pneumophilia* 2 H-NOX, a *H. sapiens* β1, a *R. norvegicus* β1, a C. *lupus* H-NOX, *a D.* melanogaster β1, *a D.* melanogaster CG14885-PA, a C. *elegans* GCY-35, a *N*. *punctiforme* H-NOX, C. *crescentus* H-NOX, a S. *oneidensis* H-NOX, or C. *acetobutylicum* H-NOX. In some embodiments, the H-NOX protein comprises an H-NOX domain corresponding to the H-NOX domain of *T. tengcongensis* set forth in SEQ ID NO:2. In some embodiments, of the disclosure the H-NOX comprises one or more distal pocket mutations. In some embodiments! the distal pocket mutation is an amino acid substitution at a site corresponding to L144 of *T. tengcongensis* H-NOX. In some embodiments, the H-NOX is a *T. tengcongensis* H-NOX comprising an amino acid substitution at position 144. In some embodiments, the amino acid substitution at position 144 is an L144F substitution. In some embodiments, the H-NOX protein is a polymeric H-NOX protein. In some embodiments, the polymeric H-NOX protein comprises monomers, wherein the monomers comprise an H-NOX domain and a polymerization domain. In some embodiments, the H-NOX domain is covalently linked to the polymerization domain. In some embodiments, the polymeric H-NOX protein is a trimeric H-NOX protein. In some embodiments, the trimeric H-NOX protein comprises one or more trimerization domains. In some embodiments, the trimeric H-NOX protein comprises three monomers, wherein the monomers comprise an H-NOX domain and a trimerization domain, wherein the trimerization domain is a bacteriophage T4 trimerization domain. In some embodiments, the trimerization domain is a foldon domain. In some embodiments, wherein the foldon domain comprises the amino acid sequence of SEQ ID NO:4. In some embodiments, the H-NOX protein is fused to an Fc domain of an immunoglobulin. In some embodiments, the H-NOX protein is covalently bound to polyethylene glycol.

In some embodiments, the O₂ dissociation constant of the H-NOX protein is within 2 orders of magnitude of that of hemoglobin, and wherein the NO reactivity of the H-NOX protein is at least 10-fold lower than that of hemoglobin. In some embodiments, the O₂ dissociation constant of the polymeric H-NOX protein is between about 1 nM and about 1000 nM at 20 °C. In some embodiments, the O₂ dissociation constant of the H-NOX protein is between about 1 µM and about 10 µM at 20 °C. In some embodiments, the O₂ dissociation constant of the H-NOX protein is between about 10 µM and about 50 µM at 20 °C. In some embodiments, the NO reactivity of the H-NOX protein is less than about 700 _{S}⁻¹ at 20 °C. In some embodiments, the NO reactivity of the H-NOX protein is at least 100-fold lower than that of hemoglobin. In some embodiments, the NO reactivity of the H-NOX protein is at least 1,000-fold lower than that of hemoglobin. In some embodiments, the k_{off} for oxygen of the H-NOX protein is less than or equal to about 0.65 s⁻¹at 20 °C. In some embodiments, the k_{off} for oxygen of the H-NOX protein is between about 0.21 s⁻¹and about 0.65 _{S}⁻¹ at 20 °C. In some embodiments, the k_{off} for oxygen of the H-NOX protein is between about 1.35 s⁻¹and about 2.9 _{S}⁻¹ at 20 °C. In some embodiments, the rate of heme autoxidation of the H-NOX protein is less than about 1 h⁻¹at 37 °C.

In some embodiments, the H-NOX protein is in a pharmaceutical composition. In some embodiments, the pharmaceutical composition further comprises a pharmaceutically acceptable carrier. In some embodiments, the invention provides a pharmaceutical composition as claimed comprising an H-NOX protein for the treatment of a hypoxic injury to an organ (e.g., brain) in an individual according to any of the methods described herein.

Disclosed is furthemore a use of a pharmaceutical composition comprising an H-NOX protein for the treatment of a hypoxic injury to an organ (e.g., brain) in an individual according to any of the methods described herein.

Disclosed is furthemore a kit comprising a pharmaceutical composition comprising an H-NOX protein, wherein the H-NOX protein is for use in any of the methods described herein.

Disclosed are furthemore methods to tissue hypoxia in the brain of an individual, the method comprising administering a therapeutically effective amount of an H-NOX protein to the individual in conjunction with another therapy. In some embodiments, the H-NOX is delivered before, during, or after the other therapy. In some embodiments, the hypoxic tissue is associated with a stroke in the individual. In some embodiments, the other therapy chemical or mechanical recanalization of an occluded vessel. In some embodiments, the other therapy is administration of tissue plasminogen activator.

Disclosed is furthemore a method to treat stroke in an individual, the method comprising administering a bolus of an H-NOX protein to the individual followed by an infusion of H-NOX to the individual. In some embodiments, the H-NOX is delivered by infusion over a period of about over more than about 1 hour, 3 hours, 6 hours, 12 hours, 1 day, 2 days, 3 days, 4 days, 5 days, 6 days , or 7 days. In some embodiments, the infusion is initiated immediately after or about 1, 2, 3, 4, 5, or 6 hours after the bolus of H-NOX is administered to the individual. In some embodiments, the H-NOX is delivered in conjunction with another therapy. In some embodiments, the H-NOX is delivered by bolus and/or infusion before, during, or after the other therapy. In some embodiments, the other therapy is administration of tissue plasminogen activator or recanalization of an occluded vessel using a mechanical device. In some embodiments, the other therapy is administration of tissue plasminogen activator. In some embodiments, the H-NOX protein is delivered systemically (e.g., intravenously, intramuscularly, subcutaneously), to a site of occlusion (e.g., intracranial), to the brain, or to the CSF.

Disclosed are furthemore methods for delivering O₂ to the penumbra following a stroke in an individual comprising administering a therapeutically effective amount of H-NOX to the individual as a bolus and/or by infusion.

Disclosed are furthemore methods for treating a hypoxic brain injury in an individual, the method comprising administering a therapeutically effective amount of an H-NOX protein to the individual as a bolus and/or by infusion. Disclosed are furthemore methods for delivering oxygen to an individual with a hypoxic brain injury, the method comprising administering a therapeutically effective amount of an H-NOX protein to the individual as a bolus and/or by infusion.

Disclosure are furthemore methods for treating a transient ischemic attack in an individual, the method comprising administering a therapeutically effective amount of an H-NOX protein to the individual as a bolus and/or by infusion. Disclosure are furthemore methods for delivering oxygen to an individual following a transient ischemic attack, the method comprising administering a therapeutically effective amount of an H-NOX protein to the individual as a bolus and/or by infusion.

Disclosed are furthemore methods for reducing inflammation at the hypoxic penumbra associated with a vascular occlusion or hemorrhage in an individual following a stroke, the method comprising administering a therapeutically effective amount of an H-NOX protein to the individual.

In some embodiments, the individual displays sustained hypoperfusion of the brain as a result of the stroke, hypoxic brain injury, transient ischemic attack, a cerebral vasospasm or brain aneurysm repair.

In some embodiments, delivery of H-NOX to the site of occlusion or the hypoxic penumbra associated with a site of occlusion reduces the activation of astrocytes, microglia, macrophages and neuronal damage/death at the site of occlusion or the penumbra associated with a site of occlusion. In some embodiments, delivery of the H-NOX to the site of the occlusion or the hypoxic penumbra associated with a site of occlusion reduces infarct volume. In some embodiments, delivery of H-NOX to the site of occlusion or the hypoxic penumbra associated with a site of occlusion reduces sensorimotor deficits associated with stroke, hypoxic brain injury, transient ischemic attack, cerebral vasospasm, or brain aneurysm repair.

Disclosed are furthemore methods for reducing the risk of hypoxic tissue in the brain of in an individual, the method comprising administering a therapeutically effective amount of an H-NOX protein as a bolus and/or by infusion to the individual at risk for developing hypoxic tissue in the brain. Disclosed are furthemore methods for delivering oxygen to an individual at risk of having hypoxic tissue in the brain, the method comprising administering a therapeutically effective amount of an H-NOX protein to the individual as a bolus and/or by infusion. In some embodiments the individual at risk of having hypoxic tissue in the brain is at risk for a stroke. In some embodiments, the individual at risk of having a stroke has had a transient ischemic attack or cerebral vasospasm, or brain aneurysm repair.

In some embodiments, the individual is a mammal. In some embodiments, the mammal is a human. In some embodiments, the mammal is a pet, a laboratory research animal, or a farm animal. In some embodiments, the pet, research animal or farm animal is a dog, a cat, a horse, a monkey, a rabbit, a rat, a mouse, a guinea pig, a hamster, a pig, or a cow.

In some embodiments of the disclosure, the H-NOX protein is a *T. tengcongensis* H-NOX, a *L. pneumophilia* 2 H-NOX, a *H. sapiens* β1, a *R. norvegicus* β1, a C. *lupus* H-NOX, a *D.* melanogaster β1, a *D.* melanogaster CG14885-PA, a C. *elegans* GCY-35, a *N. punctiforme* H-NOX, C. *crescentus* H-NOX, a S. *oneidensis* H-NOX, or C. *acetobutylicum* H-NOX. In some embodiments, the H-NOX protein comprises an H-NOX domain corresponding to the H-NOX domain of *T. tengcongensis* set forth in SEQ ID NO:2. In some embodiments of the disclosure, the H-NOX comprises one or more distal pocket mutations. In some embodiments of the disclosure, the distal pocket mutation is an amino acid substitution at a site corresponding to L144 of *T. tengcongensis* H-NOX. In some embodiments, the H-NOX is a *T. tengcongensis* H-NOX comprising an amino acid substitution at position 144. In some embodiments, the amino acid substitution at position 144 is an L144F substitution. In some embodiments of the disclosure, the H-NOX protein is a polymeric H-NOX protein. In some embodiments, the polymeric H-NOX protein comprises monomers, wherein the monomers comprise an H-NOX domain and a polymerization domain. In some embodiments, the H-NOX domain is covalently linked to the polymerization domain. In some embodiments, the polymeric H-NOX protein is a trimeric H-NOX protein. In some embodiments, the trimeric H-NOX protein comprises one or more trimerization domains. In some embodiments, the trimeric H-NOX protein comprises three monomers, wherein the monomers comprise an H-NOX domain and a trimerization domain, wherein the trimerization domain is a bacteriophage T4 trimerization domain. In some embodiments, the trimerization domain is a foldon domain. In some embodiments, wherein the foldon domain comprises the amino acid sequence of SEQ ID NO:4. In some embodiments, the H-NOX protein is fused to an Fc domain of an immunoglobulin. In some embodiments, the H-NOX protein is covalently bound to polyethylene glycol.

In some embodiments, the O₂ dissociation constant of the H-NOX protein is within 2 orders of magnitude of that of hemoglobin, and wherein the NO reactivity of the H-NOX protein is at least 10-fold lower than that of hemoglobin. In some embodiments, the O₂ dissociation constant of the polymeric H-NOX protein is between about 1 nM and about 1000 nM at 20 °C. In some embodiments, the O₂ dissociation constant of the H-NOX protein is between about 1 µM and about 10 µM at 20 °C. In some embodiments, the O₂ dissociation constant of the H-NOX protein is between about 10 µM and about 50 µM at 20 °C. In some embodiments, the NO reactivity of the H-NOX protein is less than about 700 _{S}⁻¹ at 20 °C. In some embodiments, the NO reactivity of the H-NOX protein is at least 100-fold lower than that of hemoglobin. In some embodiments, the NO reactivity of the H-NOX protein is at least 1,000-fold lower than that of hemoglobin. In some embodiments, the k_{off} for oxygen of the H-NOX protein is less than or equal to about 0.65 s⁻¹at 20 °C. In some embodiments, the k_{off} for oxygen of the H-NOX protein is between about 0.21 s⁻¹and about 0.65 _{S}⁻¹ at 20 °C. In some embodiments, the k_{off} for oxygen of the H-NOX protein is between about 1.35 s⁻¹and about 2.9 _{S}⁻¹ at 20 °C. In some embodiments, the rate of heme autoxidation of the H-NOX protein is less than about 1 h⁻¹at 37 °C.

In some embodiments, the H-NOX protein is in a pharmaceutical composition. In some embodiments, the pharmaceutical composition further comprises a pharmaceutically acceptable carrier. In some embodiments, the invention provides a pharmaceutical composition as claimed comprising an H-NOX protein for the treatment of a hypoxia in the brain of an individual according to any of the methods to treat hypoxia in the brain as described herein.

Disclosed is furthemore a use of a pharmaceutical composition comprising an H-NOX protein for the treatment of a hypoxia in the brain of an individual according to any of the methods described herein.

Disclosed is furthemore a kit comprising a pharmaceutical composition comprising an H-NOX protein, wherein the H-NOX protein is for use in any of the methods to treat hypoxia in the brain as described herein.

### BRIEF DESCRIPTION OF THE DRAWINGS

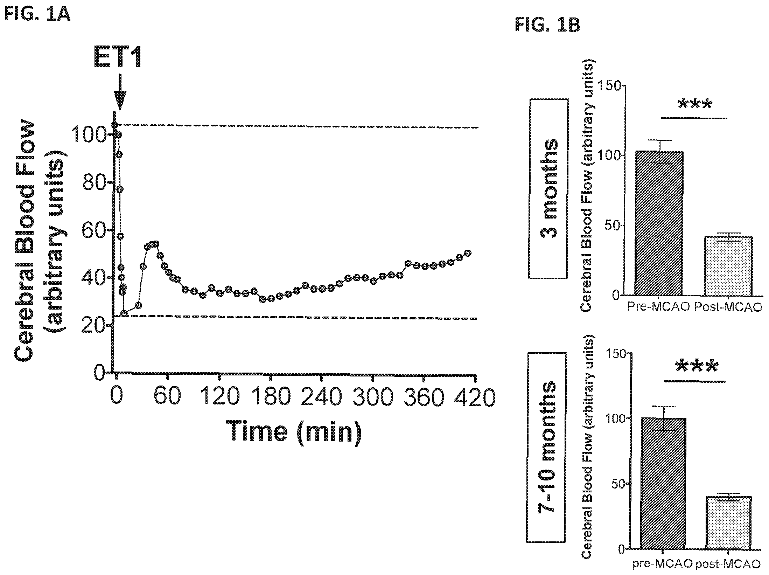

FIG 1A shows a representative example of alterations in rCBF after intracranial injection of ET1 close to the MCA. Laser Doppler flowmetry was performed on anesthetized animals using a non-invasive VP5 probe and measure for ~ 7 hours.
FIG. 1B shows rCBF measurements after ET1 injection before administering vehicle or H-NOX (trimeric H-NOX L144F) in young (3 months) or aged (7-10 months) rats. ET1 injection resulted in an average ~ 60% rCBF decrease relative to baseline values. Mean ± SEM, n=15-25/group, ***, p<0.001 by unpaired t-test.

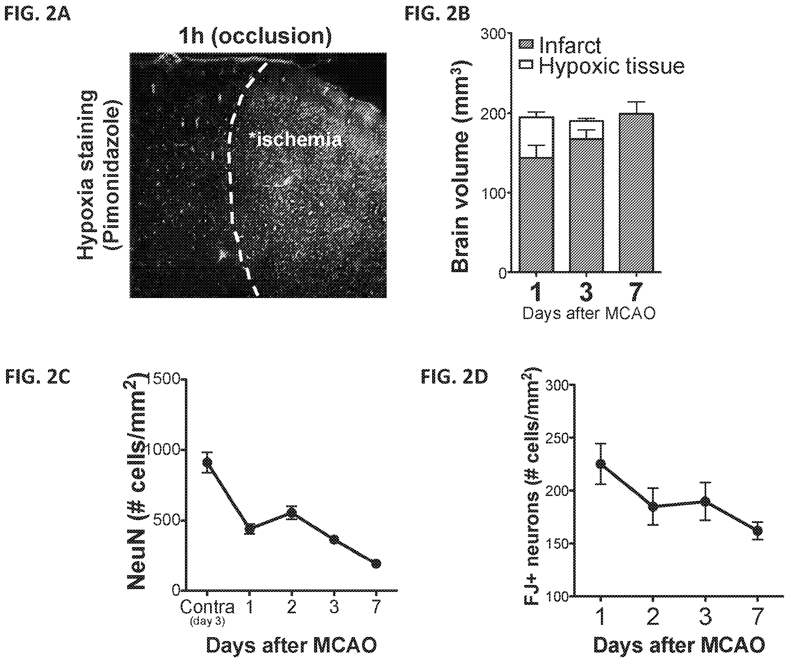

FIG. 2A shows the detection of hypoxic tissue evolution in a rat ET1-induced tMCAO model using pimonidazole staining. n = 3-4 rats per condition.

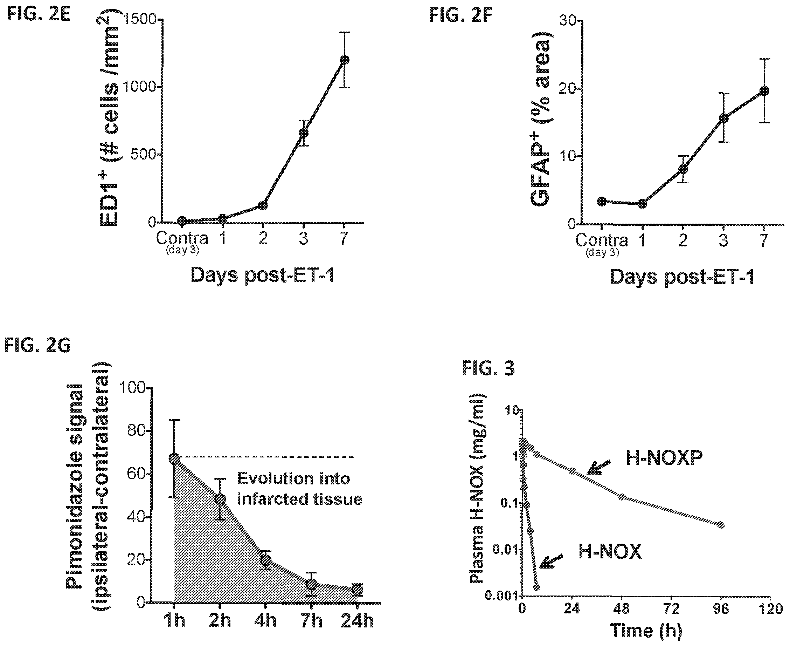

FIGS. 2B and 2G show the evolution of hypoxia and infarct formation post-tMCAO. Animals were sacrificed at 1 hour to 7 days after tMCAO and brains were processed for histology (FIG. 2B) or ELISA (FIG. 2C) using an anti-pimonidazole antibody (hypoxia volume) or cresyl violet staining (infarct volume). Cresyl violet-negative and pimonidazole-positive areas were quantified in sections over 9 coordinate levels every 1 mm. Mean ± SEM, n=5/group for histology; n=10 per group for ELISA.
FIGS. 2C-2F show the evolution of neuronal death and gliosis in the ET1 tMCAO model. Animals were sacrificed at 1-7 days after tMCAO. Brains sections were stained using NeuN (neurons), FluoroJade (FJ; neurodegeneration), ED1 (activated microglia/macrophages) and GFAP (astrocytes). The number of NeuN⁺ (FIG. 2C), FJ⁺ (FIG. 2D) and ED1⁺ (FIG. 2E) cells were counted and the percent of area covered by GFAP signal (FIG. 2F) was measured in 8 sections per coordinate level in 2 non-overlapping regions of the cortex located in the peri-infarct area. Mean±SEM. n=6-10/group.
FIG. 3 shows plasma levels of H-NOX (trimeric H-NOX L144F) and H-NOXP (PEGylated trimeric H-NOX L144F) following intravenous injection in rats, as quantified by ELISA using a 5-parameter fitting model.

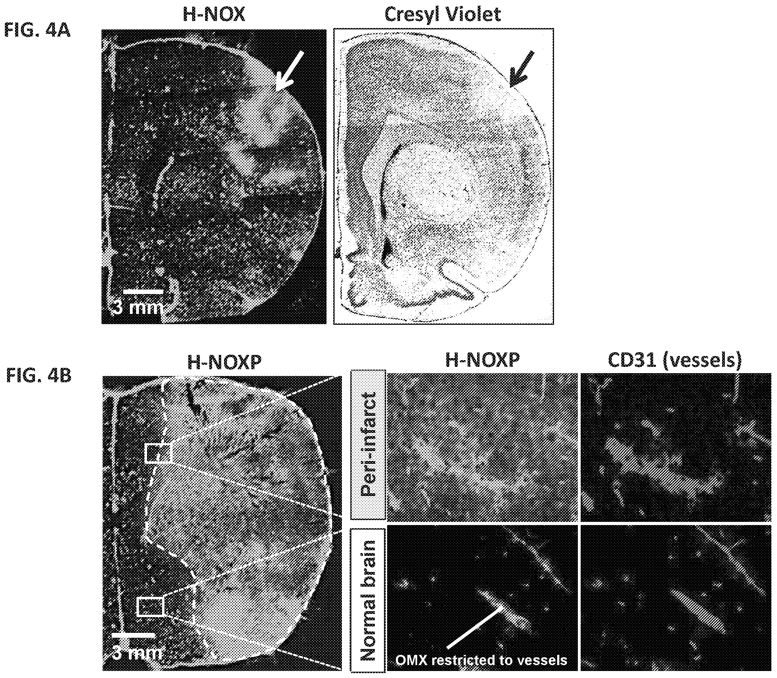

FIGS. 4A and 4B show the extravasation of H-NOX (trimeric H-NOX L144F) and H-NOXP (PEGylated trimeric H-NOX L144F) into the brain parenchyma through disrupted vasculature after ET1-induced tMCAO in rats. A single dose of H-NOX or H-NOXP (400 mg/kg) was administered 1h after tMCAO onset, and animals were euthanized 2 hours (FIG. 4A) to 24 hours (FIG. 4B) post-occlusion. Sections were stained with anti-H-NOX and CD31 (endothelial cells) antibodies. H-NOX leaked outside the vasculature 2h after onset. H-NOXP accumulated in penumbra for at least 1d after tMCAO, extravasating across a leaky BBB and persisting up to 1d (peri-infarct panel). In contrast, H-NOXP remained in the intact vasculature in normal brain tissue (normal brain panel).

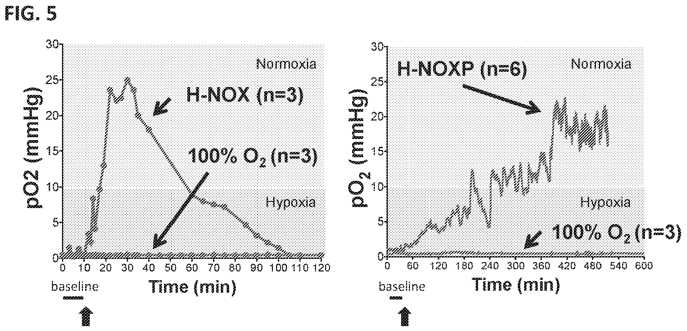

FIG. 5 shows detection of oxygen in hypoxic tissue following administration of H-NOX (trimeric H-NOX L144F) (left panel) or H-NOXP (PEGylated trimeric H-NOX L144F) (right panel). Mice bearing H460 subcutaneous xenograft tumors were used when tumor volume reached ~400-600 mm3 (~10-18 days after tumor cell subcutaneous implantation). pO2 was measured in H460 tumors using a four-channel fiber-optic oxygen-sensing device (OxyLite^{™}, Oxford Optronix, UK) consisting of the ruthenium chloride dye held in a polymer matrix with 230 µm in diameter at the tip. After probe implantation, H-NOX or PEGylated H-NOX L144F was injected IV or intratumor and fluorescent quenching was recorded as a function of time.

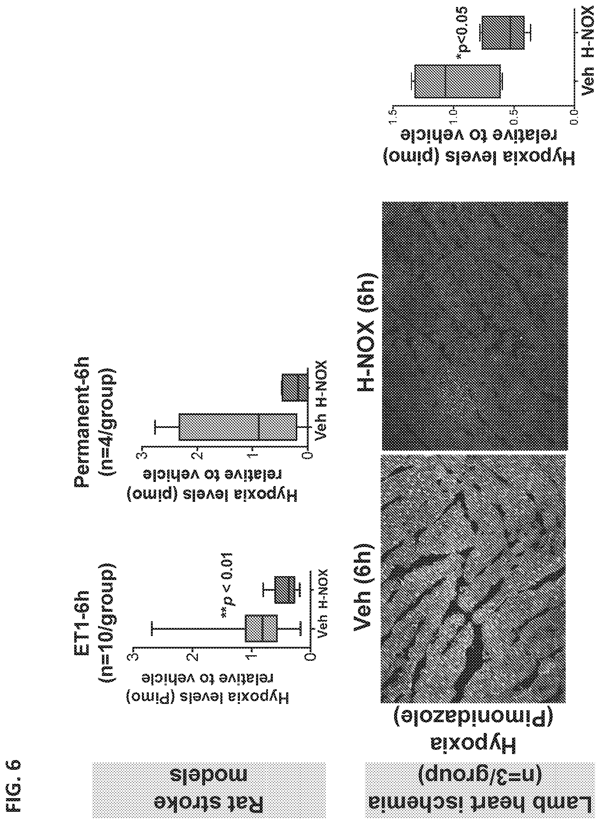

FIG. 6 shows the results of hypoxia levels quantified by pimonidazole ELISA in the rat ET1 tMCAO and permanent MCAO models (upper panels) and a lamb myocardial hypoxia model (lower panels) 6 hours after a administering vehicle (Veh) or 1:1 mixture of H-NOX (trimeric H-NOX L144F) + H-NOXP (PEGylated trimeric H-NOX L144F). Representative pictures of immunohistochemical staining of pimonidazole for the lambs are shown on the lower panels.

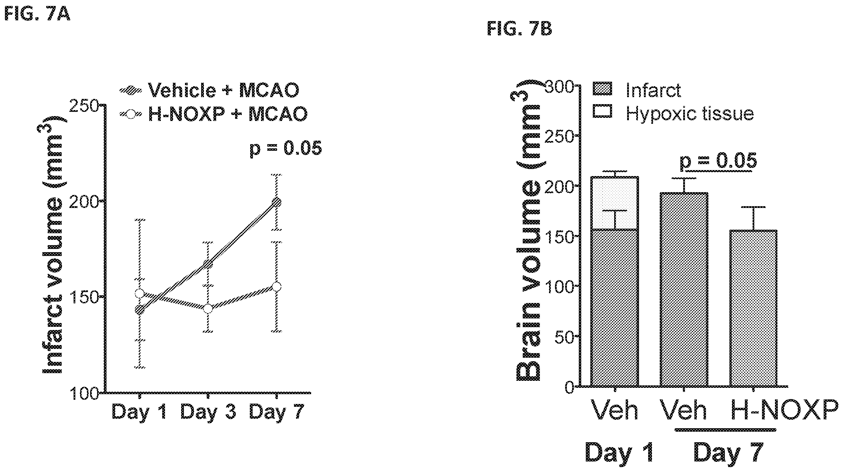

FIGS. 7A and 7B show the prevention of hypoxic tissue loss after ET1-induced tMCAO. H-NOXP (PEGylated trimeric H-NOX L144F) (240 mg/kg) or vehicle (Veh) was administered 1h after occlusion and repeated every 24 hours for 3 days. Infarct volume at 1-7 days post-tMCAO was quantified by histology using cresyl violet staining (FIG. 7A). For each section the total area of each hemisphere and infarct area were measured using ImageJ, with infarct volume corrected for edema. Mean± SEM. n=8-10, *p*=0.05 by unpaired t-test at day 7. Infarct and hypoxic tissue volume at 1-7 days post-tMCAO were quantified by cresyl violet and pimonidazole staining (FIG. 7B). Mean ± SEM. Veh=12/group; H-NOXP=6/group. Day 7: Veh vs H-NOXP, p=0.05 by unpaired t-test.

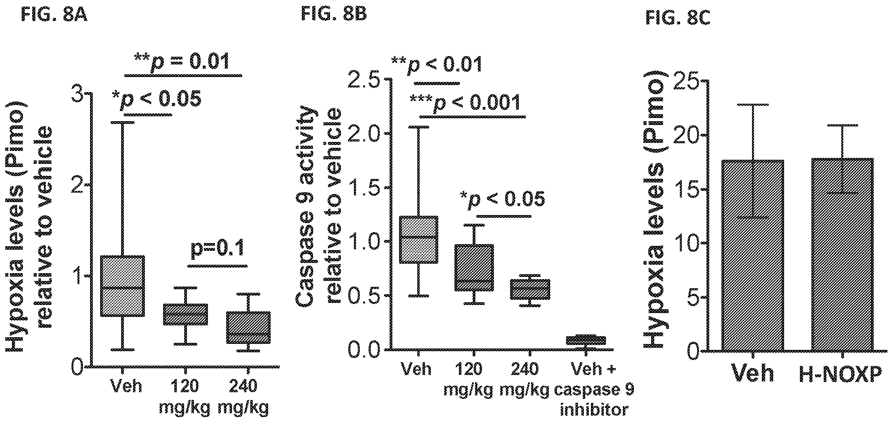

FIGS. 8A-8C show the effect of H-NOX on hypoxia and cell death 6 hours after ET1-induced tMCAO. A 1:1 mixture of H-NOX (trimeric H-NOX L144F) + H-NOXP (PEGylated trimeric H-NOX L144F) (120 mg/kg or 240 mg/kg) or vehicle (Veh) was administered 30 min post-tMCAO. Rats were assayed for pimonidazole by ELISA (FIG. 8A) or caspase 9 activity by luminometry assay (Promega) (FIG. 8B). Veh: n=20; 120 mg/kg: n=10; 240 mg/kg: n=8. Mean±SEM. ^{∗∗∗}*p*<0.001, ^{∗∗}*p*<0.01, ^{∗}p<0.05 by one-way ANOVA and Student's t tests. H-NOXP alone (240 mg/kg) was administered 1 hour post-tMCAO and brains were harvested 6 hours post-occlusion and assayed for pimonidazole levels by ELISA (FIG. 8C). Mean ± SEM. n=6-7/group.

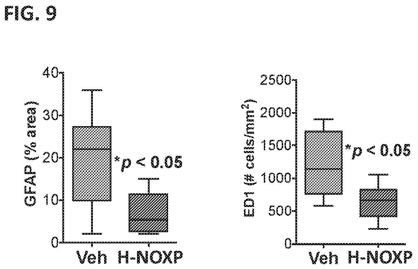

FIG. 9 shows H-NOX-mediated decrease in inflammation at 7 days post-ETl-induced tMCAO. Vehicle (Veh) or H-NOXP (PEGylated trimeric H-NOX L144F) (240 mg/kg) was administered 1h after occlusion and administration was repeated every 24h for 3d. Rats were sacrificed 7 days post-tMCAO. Sections were stained for anti-GFAP (astrocytes) and anti-EDl (activated microglia and macrophages). ED1⁺ cells were counted and GFAP percent of area was measured across sections. Mean ± SEM. n=6-7/group. ^{∗}*p*<0.05 by the Student's t test.

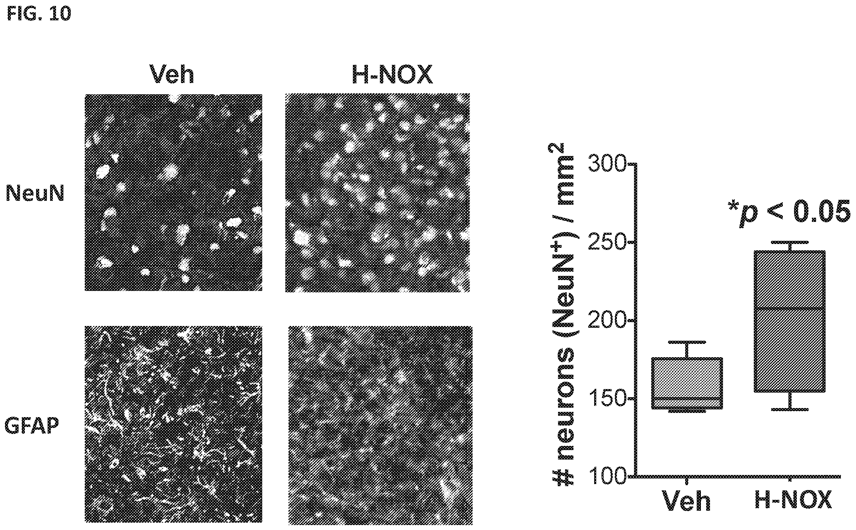

FIG. 10 shows immunohistochemical staining for NeuN and GFAP in the peri-infarct area at 29 days following MCAO in aged rats administered either H-NOX (trimeric H-NOX L144F) (400 mg/kg) or vehicle control (Veh) 1 hour after tMCAO, with administration repeated every 24 hours for 3 days. The neuronal density (indicated by NeuN+ cells) was quantified in sections over 8 coordinates per level (every 1 mm) in 2 non-overlapping regions of the cortex located in the peri-infarct area where there is GFAP signal. Mean ± SEM. n=6-7/group. ^{∗}*p*<0.05 by the Student's t test.

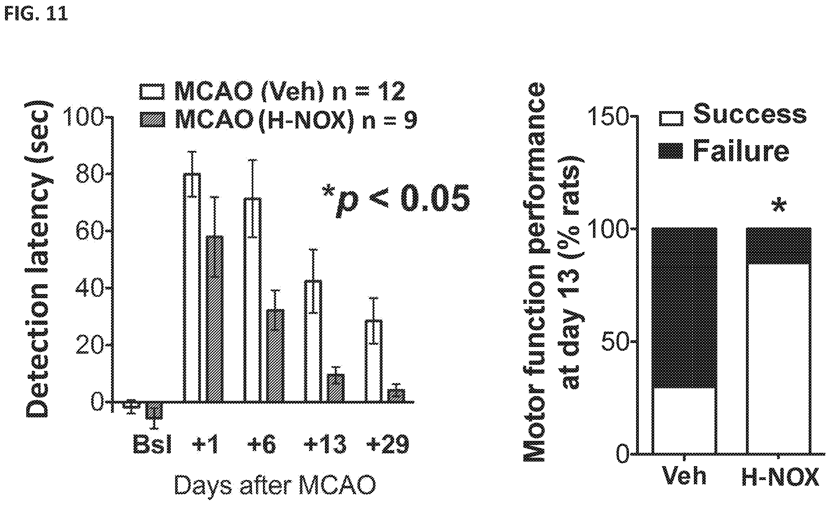

FIG. 11 shows the results of an adhesive removal assay probing sensory and motor function in aged rats administered H-NOX (trimeric H-NOX L144F) (400 mg/kg) or vehicle (Veh) 1 hour after tMCAO, with administration repeated every 24 hours for 3 days. The detection latency was recorded on day 1,6, 13, and 29 after tMCAO. ^{∗}*p*<0.05 by two way repeated measures ANOVA. Bsl = baseline. Bonferroni post-test analysis showed that the H-NOX and vehicle groups on days 6 and 13 were significantly different. Motor function performance was assessed by the percentage of rats that successfully removed the tape within 75 sec at 13 days after tMCAO. All included rats successfully removed the tape on the unimpaired paw (not shown). Veh (n=12), H-NOX (n=9). Mean ± SEM. ^{∗}*p*<0.05 by Fisher's exact test with two-sided hypothesis. Sham animals did not present significant sensory-motor deficits (not shown).

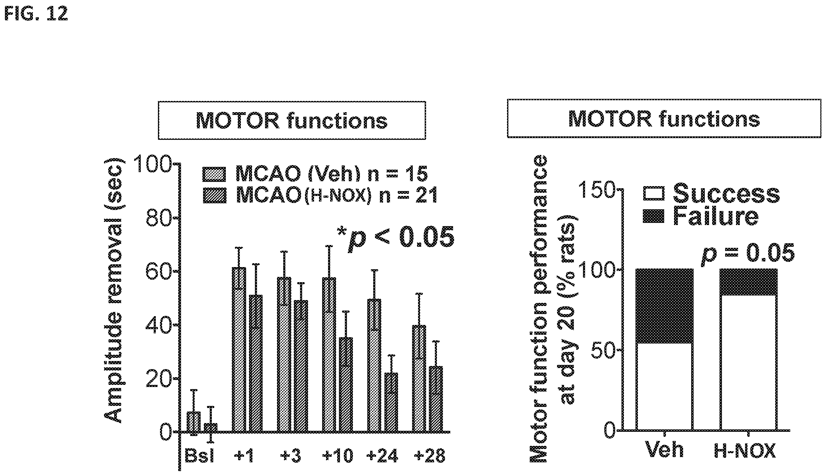

FIG. 12 shows the results of an adhesive removal assay probing motor function in aged rats administered H-NOX (trimeric H-NOX L144F) (240 mg/kg) or vehicle control (Veh) 2 hours after tMCAO, with administration repeated every 24h for 3 days. The removal latency (sec) was recorded on day 1, 3, 10,24 and 28 after tMCAO. ^{∗}*p*<0.05 by two-way repeated measures ANOVA. Motor function performance was also assessed by the percentage of rats that successfully removed the tape within 60 sec at 20 days after tMCAO. All included rats successfully removed the tape on the unimpaired paw (not shown). Vehicle (n=15), H-NOX (n=21). Mean ± SEM. ^{∗}*p*<0.05 by two way ANOVA repeated measures (removal latency) and ^{∗}*p*<0.05 by Fisher's exact test with the two-sided hypothesis (removal success). Sham-operated animals did not present any significant sensory-motor deficits (not shown).

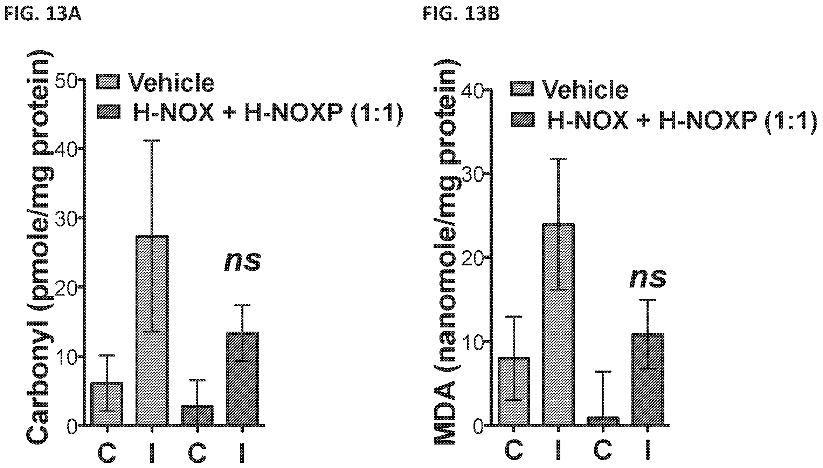

FIGS. 13A and 13B show the effect of H-NOX on free radicals 6 hours post-tMCAO during spontaneous reperfusion. A 1:1 mixture of H-NOX (trimeric H-NOX L144F) + H-NOXP (PEGylated trimeric H-NOX L144F) (400 mg/kg) was administered 1 hour post-tMCAO and brains were assayed 6 hours post-tMCAO for protein carbonyl content (FIG. 13A) by ELISA (OxiSelect^{™}) and lipid peroxydation (FIG. 13B) with the TBARs assay (Cayman), two markers widely accepted as indicators of oxidative stress. Mean ± SEM, n=6-8 rats/group. ns: not significant by Student's t test. C: contralateral, I: ipsilateral.

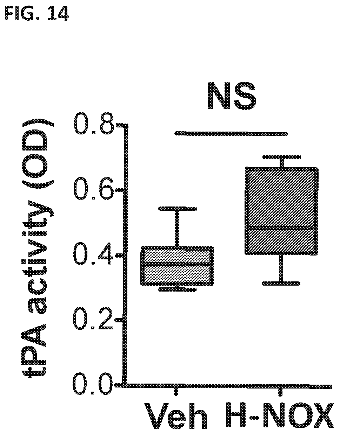

FIG. 14 shows an *in vitro* tPA activity assay with vehicle control (Veh) or an excess of a 1:1 mixture of H-NOX (trimeric H-NOX L144F) + H-NOXP (PEGylated trimeric H-NOX L144F) (100X the amount of tPA) added.

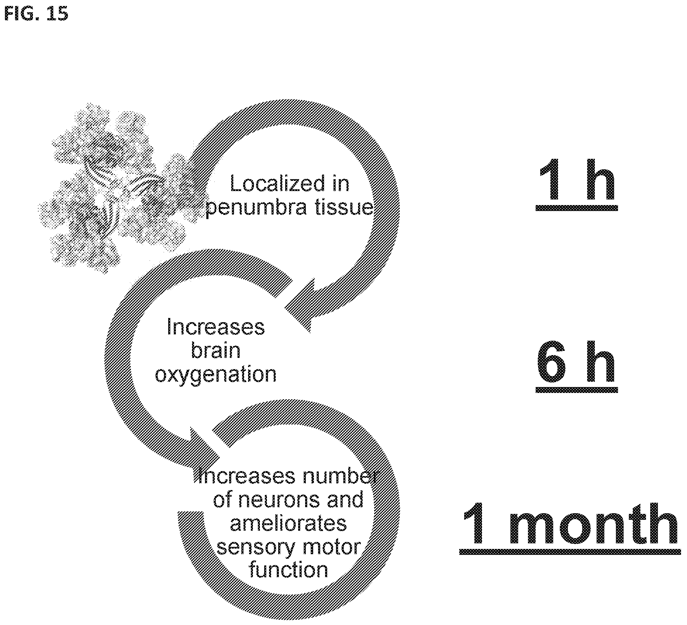

FIG. 15 shows a schematic demonstrating the potential for H-NOX to treat hypoxia in the brain associated with stroke.

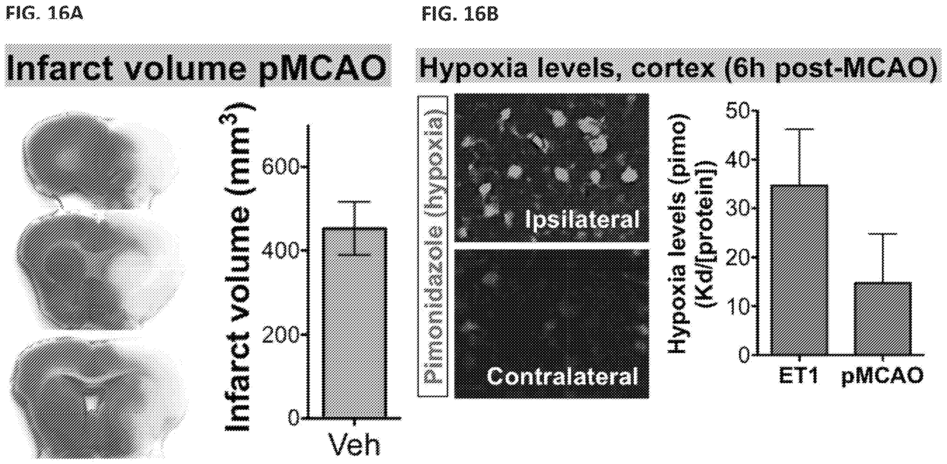

FIGS. 16A and 16B show infarct volume and hypoxia in a permanent MCAO (pMCAO) model. Infarct volume was assayed by TTC staining (FIG. 16A), and hypoxia was measured by pimonidazole staining (FIG. 16B). For each TTC-stained section (6 sections every 2 mm), the total area of each hemisphere and the infarct area were measured using ImageJ. Infarct volume corrected for edema was calculated. Mean +/- SEM (n=4). Pimonidazole (60 mg/kg) was injected i.v. 2 hours before sacrifice and brain sections were stained with an anti-pimonoidazole antibody or tissue was analyzed by ELISA.

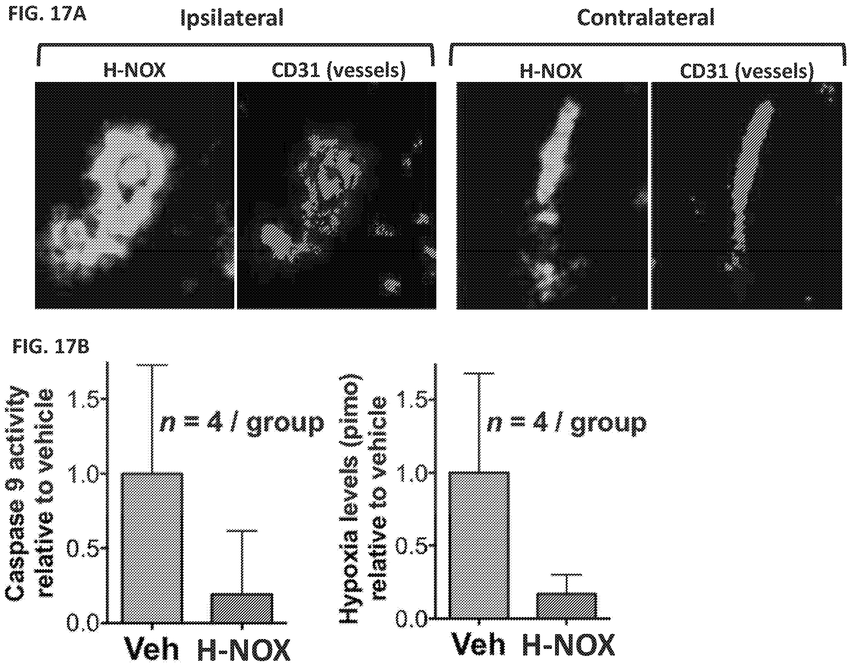

FIGS. 17A and 17B show results for administration of H-NOX (trimeric H-NOX L144F) in a pMCAO model. H-NOX (240 mg/kg) was administered 1 hour after occlusion. Animals were either euthanized 2 hours post-pMCAO and brain sections were stained with anti-H-NOX and anti-CD31 antibodies (FIG. 17A), or euthanized 6 hours post-pMCAO and brains were harvested and assayed for pimonidazole by ELISA and caspase 9 activity by luminometry.

### DETAILED DESCRIPTION OF THE INVENTION

Disclosed herein are methods for treating stroke in an individual, the method comprising administering a therapeutically effective amount of an H-NOX to the individual, wherein the H-NOX comprises H-NOX covalently bound to polyethylene glycol (PEG) and H-NOX that is not covalently bound to polyethylene glycol. In some embodiments, the disclosure provides for treating a transient ischemic attack in an individual, the method comprising administering a therapeutically effective amount of H-NOX to the individual, wherein the H-NOX comprises H-NOX covalently bound to PEG and H-NOX that is not covalently bound to PEG. In some embodiments, the disclosure provides methods for treating a hypoxic brain injury in an individual, the method comprising administering a therapeutically effective amount of an H-NOX to the individual, wherein the H-NOX comprises H-NOX covalently bound to PEG and H-NOX that is not covalently bound to PEG. Hypoxic brain injury includes but is not limited to hypoxic brain injury, vessel occlusion, and hemorrhage of the brain. Without being bound by theory, the H-NOX that is not bound to PEG provides rapid delivery of O₂ to sites of hypoxia following ischemic injury whereas the H-NOX that is bound to PEG accumulates in hypoxic tissue and provides longer term delivery of O₂ to reduce infarct of hypoxic tissue. Surprisingly the mixture of PEGylated H-NOX and non-PEGylated H-NOX does not increase reactive oxygen species (ROS) in the brain. Previous attempts to deliver O₂ to the brain following stroke have shown to increase ROS in a reperfusion setting (Thom, SR, 1985, J Appl Physiol. 106(3):988-995).

Disclosed are methods for delivering oxygen to an individual following a stroke, the method comprising administering a therapeutically effective amount of an H-NOX to the individual, wherein N-NOX comprises H-NOX covalently bound to polyethylene glycol and H-NOX that is not covalently bound to PEG. Disclosed is furthemore a method for delivering oxygen to an individual following a transient ischemic attack, the method comprising administering a therapeutically effective amount of an H-NOX to the individual, wherein the H-NOX comprises H-NOX covalently bound to PEG and H-NOX that is not covalently bound to PEG. Disclosed are furthemore methods for delivering oxygen to an individual following a hypoxic brain injury, the method comprising administering a therapeutically effective amount of an H-NOX to the individual, wherein the H-NOX comprises H-NOX covalently bound to PEG and H-NOX that is not covalently bound to PEG.

The present invention is based at least in part on the surprising discovery that H-NOX proteins extravasate through the blood brain barrier and preferentially accumulate in hypoxic tissues thereby providing methods to oxygenate hypoxic tissue associated with organ injury *(e.g.,* brain injury). This was surprising as most agents do not cross the blood brain barrier. Disclosed are methods for treating hypoxia associated with injury to an organ (e.g., brain) in an individual comprising administering a therapeutically effective amount of an H-NOX protein to the individual. Disclosed are furthemore methods for delivering O₂ to hypoxic tissue associated with injury to an organ in an individual under hypoxic stress comprising administering a therapeutically effective amount of an H-NOX protein to the individual. Disclosed are furthemore methods for reducing consequences of hypoxia associated with injury to an organ (e.g., brain) in an individual comprising administering a therapeutically effective amount of an H-NOX protein to the individual (e.g., sensorimotor deficits, locomotion deficits, cognition deficits, speech deficits, *etc*.). Disclosed are furthemore methods to treat stroke in an individual, the method comprising administering a administering a therapeutically effective amount of an H-NOX protein to the individual in conjunction with another therapy. Disclosed are furthemore methods to treat stroke in an individual, the method comprising administering a bolus of an H-NOX protein to the individual followed by an infusion of H-NOX to the individual. Accordingly, disclosed are proteins, compositions, kits and methods for the delivery of oxygen following hypoxia associated with injury to an organ *(e.g.,* brain).

### Definitions

Unless defined otherwise, the meanings of all technical and scientific terms used herein are those commonly understood by one of skill in the art to which this invention belongs. One of skill in the art will also appreciate that any methods and materials similar or equivalent to those described herein can also be used to practice or test the invention.

For use herein, unless clearly indicated otherwise, use of the terms "a", "an," and the like refers to one or more.

In this application, the use of "or" means "and/or" unless expressly stated or understood by one skilled in the art. In the context of a multiple dependent claim, the use of "or" refers back to more than one preceding independent or dependent claim.

Reference to "about" a value or parameter herein includes (and describes) embodiments that are directed to that value or parameter per se. For example, description referring to "about X" includes description of "X."

It is understood that aspect and embodiments of the invention described herein include "comprising," "consisting," and "consisting essentially of' aspects and embodiments.

The terms "polypeptide" and "protein" are used interchangeably to refer to a polymer of amino acid residues, and are not limited to a minimum length. Such polymers of amino acid residues may contain natural or non-natural amino acid residues, and include, but are not limited to, peptides, oligopeptides, dimers, trimers, and polymers of amino acid residues. Both full-length proteins and fragments thereof are encompassed by the definition. The terms also include post-expression modifications of the polypeptide, for example, glycosylation, sialylation, acetylation, phosphorylation, and the like. Furthermore, for purposes of the present invention, a "polypeptide" refers to a protein which includes modifications, such as deletions, additions, and substitutions (generally conservative in nature), to the native sequence, as long as the protein maintains the desired activity. These modifications may be deliberate, as through site-directed mutagenesis, or may be accidental, such as through mutations of hosts which produce the proteins or errors due to PCR amplification. As used herein, a protein may include two or more subunits, covalently or non-covalently associated; for example, a protein may include two or more associated monomers.

The terms "nucleic acid molecule", "nucleic acid" and "polynucleotide" may be used interchangeably, and refer to a polymer of nucleotides. Such polymers of nucleotides may contain natural and/or non-natural nucleotides, and include, but are not limited to, DNA, RNA, and PNA. "Nucleic acid sequence" refers to the linear sequence of nucleotides that comprise the nucleic acid molecule or polynucleotide.

As used herein, an "H-NOX protein" means a protein that has an H-NOX domain (named for Heme-Nitric oxide and OXygen binding domain). An H-NOX protein may or may not contain one or more other domains in addition to the H-NOX domain. In some examples, an H-NOX protein does not comprise a guanylyl cyclase domain. An H-NOX protein may or may not comprise a polymerization domain.

As used herein, a "polymeric H-NOX protein" is an H-NOX protein comprising two or more H-NOX domains. The H-NOX domains may be covalently or non-covalently associated.

As used herein, an "H-NOX domain" is all or a portion of a protein that binds nitric oxide and/or oxygen by way of heme. The H-NOX domain may comprise heme or may be found as an apoproprotein that is capable of binding heme. In some examples, an H-NOX domain includes six alpha-helices, followed by two beta-strands, followed by one alpha-helix, followed by two beta strands. In some examples, an H-NOX domain corresponds to the H-NOX domain of *Thermoanaerobacter tengcongensis* H-NOX set forth in SEQ ID NO:2. For example, the H-NOX domain may be at least about 10%, 15%, 20%, 25%, 30%, 40%, 50%, 60%, 70%, 80%, 90%, 95%, or 99% identical to the H-NOX domain of *Thermoanaerobacter tengcongensis* H-NOX set forth in SEQ ID NO:2. In some embodiments, the H-NOX domain may be 10%-20%, 20%-30%, 30%-40%, 40%-50%, 50%-60%, 60%-70%, 70%-80%, 80%-90%, 90%-95%, 95%-99% or 100% identical to the H-NOX domain of *Thermoanaerobacter tengcongensis* H-NOX set forth in SEQ ID NO:2.

As used herein, a "polymerization domain" is a domain *(e.g.* a polypeptide domain) that promotes the association of monomeric moieties to form a polymeric structure. For example, a polymerization domain may promote the association of monomeric H-NOX domains to generate a polymeric H-NOX protein. An exemplary polymerization domain is the foldon domain of T4 bacteriophage, which promotes the formation of trimeric polypeptides. Other examples of polymerization domains include, but are not limited to, Arc, POZ, coiled coil domains (including GCN4, leucine zippers, Velcro), uteroglobin, collagen, 3-stranded coiled colis (matrilin-1), thrombosporins, TRPV1-C, P53, Mnt, avadin, streptavidin, Bcr-Abl, COMP, verotoxin subunit B, CamKII, RCK, and domains from N ethylmaleimide-sensitive fusion protein, STM3548, KaiC, TyrR, Hcp1, CcmK4, GP41, anthrax protective antigen, aerolysin, a-hemolysin, C4b-binding protein, Mi-CK, arylsurfatase A, and viral capsid proteins.

As used herein, an "amino acid linker sequence" or an "amino acid spacer sequence" is a short polypeptide sequence that may be used to link two domains of a protein. In some embodiments, the amino acid linker sequence is one, two, three, four, five, six, seven, eight, nine, ten or more than ten amino acids in length. Exemplary amino acid linker sequences include but are not limited to a Gly-Ser-Gly sequence and an Arg-Gly-Ser sequence.

As used herein, a "His6 tag" refers to a peptide comprising six His residues attached to a polypeptide. A His6 tag may be used to facilitate protein purification; for example, using chromatography specific for the His6 tag. Following purification, the His6 tag may be cleaved using an exopeptidase.

The term "substantially similar" or "substantially the same," as used herein, denotes a sufficiently high degree of similarity between two or more numeric values such that one of skill in the art would consider the difference between the two or more values to be of little or no biological and/or statistical significance within the context of the biological characteristic measured by said value. In some embodiments the two or more substantially similar values differ by no more than about any one of 5%, 10%, 15%, 20%, 25%, or 50%.

The phrase "substantially reduced," or "substantially different," as used herein, denotes a sufficiently high degree of difference between two numeric values such that one of skill in the art would consider the difference between the two values to be of statistical significance within the context of the biological characteristic measured by said values. In some embodiments, the two substantially different numeric values differ by greater than about any one of 10%, 20%, 25%, 30%, 35%, 40%, 45%, 50%, 60%, 70%, 80%, or 90%. In some embodiment, the two substantially different numeric values differ by about any one of 10%-20%, 20%-30%, 30%-40%, 40%-50%, 50%-60%, 60%-70%, 70%-80%, 80%-90%, 90%-95%, 95%-99% or 100%.

A "native sequence" polypeptide comprises a polypeptide having the same amino acid sequence as a polypeptide found in nature. Thus, a native sequence polypeptide can have the amino acid sequence of naturally occurring polypeptide from any organism. Such native sequence polypeptide can be isolated from nature or can be produced by recombinant or synthetic means. The term "native sequence" polypeptide specifically encompasses naturally occurring truncated or secreted forms of the polypeptide *(e.g.,* an extracellular domain sequence), naturally occurring variant forms (e.g., alternatively spliced forms) and naturally occurring allelic variants of the polypeptide.

A polypeptide "variant" means a biologically active polypeptide having at least about 80% amino acid sequence identity with the native sequence polypeptide after aligning the sequences and introducing gaps, if necessary, to achieve the maximum percent sequence identity, and not considering any conservative substitutions as part of the sequence identity. Such variants include, for instance, polypeptides wherein one or more amino acid residues are added, or deleted, at the N- or C-terminus of the polypeptide. In some embodiments, a variant will have at least about any one of 80%, 90% or 95% amino acid sequence identity with the native sequence polypeptide. In some embodiments, a variant will have about any one of 80%-90%, 90%-95% or 95% -99% amino acid sequence identity with the native sequence polypeptide.

As used herein, a "mutant protein" means a protein with one or more mutations compared to a protein occurring in nature. In one embodiment, the mutant protein has a sequence that differs from that of all proteins occurring in nature. In various embodiments, the amino acid sequence of the mutant protein is at least about any of 10, 15, 20, 25, 30, 40, 50, 60, 70, 80, 90, 95, 97, 98, 99, or 99.5% identical to that of the corresponding region of a protein occurring in nature. In some embodiments, the amino acid sequence of the mutant protein is at least about any of 10%-20%, 20%-30%, 30%-40%, 40%-50%, 50%-60%, 60%-70%, 70%-80%, 80%-90%, 90%-95%, 95%-99% or 100% identical to that of the corresponding region of a protein occurring in nature. In some embodiments, the mutant protein is a protein fragment that contains at least about any of 25, 50, 75, 100, 150, 200, 300, or 400 contiguous amino acids from a full-length protein. In some embodiments, the mutant protein is a protein fragment that contains about any of 25-50, 50-75, 75-100, 100-150, 150-200, 200-300, or 300-400 contiguous amino acids from a full-length protein. Sequence identity can be measured, for example, using sequence analysis software with the default parameters specified therein (e.g., Sequence Analysis Software Package of the Genetics Computer Group, University of Wisconsin Biotechnology Center, 1710 University Avenue, Madison, WI 53705). This software program matches similar sequences by assigning degrees of homology to various amino acids replacements, deletions, and other modifications.

As used herein, a "mutation" means an alteration in a reference nucleic acid or amino acid sequence occurring in nature. Exemplary nucleic acid mutations include an insertion, deletion, frameshift mutation, silent mutation, nonsense mutation, or missense mutation. In some embodiments, the nucleic acid mutation is not a silent mutation. Exemplary protein mutations include the insertion of one or more amino acids *(e.g.,* the insertion of 2, 3, 4, 5, 6, 7, 8,9, or 10 amino acids), the deletion of one or more amino acids *(e.g.,* a deletion of N-terminal, C-terminal, and/or internal residues, such as the deletion of at least about any of 5, 10, 15, 25, 50, 75, 100, 150, 200, 300, or more amino acids or a deletion of about any of 5-10, 10-15, 15-25, 25-50, 50-75, 75-100, 100-150, 150-200, 200-300, or 300-400 amino acids), the replacement of one or more amino acids *(e.g.,* the replacement of 1, 2, 3, 4, 5, 6, 7, 8, 9, or 10 amino acids), or combinations of two or more of the foregoing. The nomenclature used in referring to a particular amino acid mutation first identifies the wild-type amino acid, followed by the residue number and finally the substitute amino acid. For example, Y140L means that tyrosine has been replaced by a leucine at residue number 140. Likewise, a variant H-NOX protein may be referred to by the amino acid variations of the H-NOX protein. For example, a *T. tengcongensis* Y140L H-NOX protein refers to a *T. tengcongensis* H-NOX protein in which the tyrosine residue at position number 140 has been replaced by a leucine residue and a *T. tengcongensis* W9F/Y140L H-NOX protein refers to a *T. tengcongensis* H-NOX protein in which the tryptophan residue at position 9 has been replaced by a phenylalanine residue and the tyrosine residue at position number 140 has been replaced by a leucine residue.

An "evolutionary conserved mutation" is the replacement of an amino acid in one protein by an amino acid in the corresponding position of another protein in the same protein family.

As used herein, "derived from" refers to the source of the protein into which one or more mutations is introduced. For example, a protein that is "derived from a mammalian protein" refers to protein of interest that results from introducing one or more mutations into the sequence of a wild-type (*i.e.,* a sequence occurring in nature) mammalian protein.

As used herein, "Percent (%) amino acid sequence identity" and "homology" with respect to a peptide, polypeptide or antibody sequence are defined as the percentage of amino acid residues in a candidate sequence that are identical with the amino acid residues in the specific peptide or polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or MEGALIGNTM (DNASTAR) software. Those skilled in the art can determine appropriate parameters for measuring alignment, including any algorithms needed to achieve maximal alignment over the full length of the sequences being compared.

As used herein, a "k_{off}" refers to a dissociation rate, such as the rate of release of O₂ or NO from a protein. A lower numerical lower k_{off} indicates a slower rate of dissociation.

As used herein, "kₒₙ" refers to an association rate, such as the rate of binding of O₂ or NO to a protein. A lower numerical lower kₒₙ indicates a slower rate of association.

As used herein, "dissociation constant" refers to a "kinetic dissociation constant" or a "calculated dissociation constant." A "kinetic dissociation constant" or "K_{D}" is a ratio of kinetic off-rate (k_{off}) to kinetic on-rate (kₒₙ), such as a K_{D} value determined as an absolute value using standard methods (e.g., standard spectroscopic, stopped-flow, or flash-photolysis methods) including methods known to the skilled artisan and/or described herein. "Calculated dissociation constant" or "calculated K_{D}" refers to an approximation of the kinetic dissociation constant based on a measured k_{off}. A value for the kₒₙ is derived via the correlation between kinetic K_{D} and k_{off} as described herein.

As used herein, "oxygen affinity" is a qualitative term that refers to the strength of oxygen binding to the heme moiety of a protein. This affinity is affected by both the k_{off} and kₒₙ for oxygen. A numerically lower oxygen K_{D} value means a higher affinity.

As used herein, "NO affinity" is a qualitative term that refers to the strength of NO binding to a protein (such as binding to a heme group or to an oxygen bound to a heme group associated with a protein). This affinity is affected by both the k_{off} and kₒₙ for NO. A numerically lower NO K_{D} value means a higher affinity.

As used herein, "NO stability" refers to the stability or resistance of a protein to oxidation by NO in the presence of oxygen. For example, the ability of the protein to not be oxidized when bound to NO in the presence of oxygen is indicative of the protein's NO stability. In some embodiments, less than about any of 50, 40, 30, 10, or 5% of an H-NOX protein is oxidized after incubation for about any of 1, 2, 4, 6, 8, 10, 15, or 20 hours at 20°C.

As used herein, "NO reactivity" refers to the rate at which iron in the heme of a heme-binding protein is oxidized by NO in the presence of oxygen. A lower numerical value for NO reactivity in units of _{S}⁻¹ indicates a lower NO reactivity

As used herein, an "autoxidation rate" refers to the rate at which iron in the heme of a heme-binding protein is autoxidized. A lower numerical autoxidation rate in units of _{S}⁻¹ indicates a lower autoxidation rate.

The term "vector" is used to describe a polynucleotide that may be engineered to contain a cloned polynucleotide or polynucleotides that may be propagated in a host cell. A vector may include one or more of the following elements: an origin of replication, one or more regulatory sequences (such as, for example, promoters and/or enhancers) that regulate the expression of the polypeptide of interest, and/or one or more selectable marker genes (such as, for example, antibiotic resistance genes and genes that may be used in colorimetric assays, *e.g.*, β-galactosidase). The term "expression vector" refers to a vector that is used to express a polypeptide of interest in a host cell.

A "host cell" refers to a cell that may be or has been a recipient of a vector or isolated polynucleotide. Host cells may be prokaryotic cells or eukaryotic cells. Exemplary eukaryotic cells include mammalian cells, such as primate or non-primate animal cells; fungal cells, such as yeast; plant cells; and insect cells. Exemplary prokaryotic cells include bacterial cells; for example, *E. coli* cells.

The term "isolated" as used herein refers to a molecule that has been separated from at least some of the components with which it is typically found in nature or produced. For example, a polypeptide is referred to as "isolated" when it is separated from at least some of the components of the cell in which it was produced. Where a polypeptide is secreted by a cell after expression, physically separating the supernatant containing the polypeptide from the cell that produced it is considered to be "isolating" the polypeptide. Similarly, a polynucleotide is referred to as "isolated" when it is not part of the larger polynucleotide (such as, for example, genomic DNA or mitochondrial DNA, in the case of a DNA polynucleotide) in which it is typically found in nature, or is separated from at least some of the components of the cell in which it was produced, *e.g.,* in the case of an RNA polynucleotide. Thus, a DNA polynucleotide that is contained in a vector inside a host cell may be referred to as "isolated".

The terms "individual" or "subject" are used interchangeably herein to refer to an animal; for example a mammal. In some embodiments, methods of treating mammals, including, but not limited to, humans, rodents, simians, felines, canines, equines, bovines, porcines, ovines, caprines, mammalian laboratory animals, mammalian farm animals, mammalian sport animals, and mammalian pets, are provided. In some examples, an "individual" or "subject" refers to an individual or subject in need of treatment for a disease or disorder.

A "disease" or "disorder" as used herein refers to a condition where treatment is needed.

As used herein, the term "hypoxic penumbra" refers to the area surrounding an injury where blood flow, and therefore oxygen transport is reduced locally, leading to hypoxia of the cells near the location of the original insult. This lack of oxygen can lead to hypoxic cell death (infarction) and amplify the original damage from the injury.

As used herein, the term "hypoperfusion" refers to an inadequate supply of blood to an organ or extremity (e.g., the brain). If hypoperfusion persists, it can cause hypoxia and can deprive the tissue of needed nutrients, oxygen, and waste disposal. In some examples hypoperfusion can cause brain tissue death and long-term neurological dysfunction.

The term "cancer" refers to a malignant proliferative disorder associated with uncontrolled cell proliferation, unrestrained cell growth, and decreased cell death via apoptosis.

The term "tumor" is used herein to refer to a group of cells that exhibit abnormally high levels of proliferation and growth. A tumor may be benign, pre-malignant, or malignant; malignant tumor cells are cancerous. Tumor cells may be solid tumor cells or leukemic tumor cells. The term "tumor growth" is used herein to refer to proliferation or growth by a cell or cells that comprise a tumor that leads to a corresponding increase in the size of the tumor.

As used herein, "treatment" is an approach for obtaining beneficial or desired clinical results. "Treatment" as used herein, covers any administration or application of a therapeutic for disease in a mammal, including a human. For purposes of this invention, beneficial or desired clinical results include, but are not limited to, any one or more of: alleviation of one or more symptoms, diminishment of extent of disease, preventing or delaying spread (e.g., reducing infarct in a hypoxic penumbra associated with organ injury) of disease, preventing or delaying recurrence of disease, delay or slowing of disease progression, amelioration of the disease state, inhibiting the disease or progression of the disease, inhibiting or slowing the disease or its progression, arresting its development, and remission (whether partial or total). Also encompassed by "treatment" is a reduction of pathological consequence of a proliferative disease *(e.g.,* the pathological consequences of sustained hypoperfusion of a tissue). The medical uses of the invention contemplate any one or more of these aspects of treatment.

The terms "inhibition" or "inhibit" refer to a decrease or cessation of any phenotypic characteristic or to the decrease or cessation in the incidence, degree, or likelihood of that characteristic. To "reduce" or "inhibit" is to decrease, reduce or arrest an activity, function, and/or amount as compared to a reference. In certain embodiments, by "reduce" or "inhibit" is meant the ability to cause an overall decrease of 20% or greater. In another embodiment, by "reduce" or "inhibit" is meant the ability to cause an overall decrease of 50% or greater. In yet another embodiment, by "reduce" or "inhibit" is meant the ability to cause an overall decrease of 75%, 85%, 90%, 95%, or 99%.

As used herein, "delaying development of a disease" means to defer, hinder, slow, retard, stabilize, suppress and/or postpone development of the disease or disorder (such as tissue hypoxia related diseases and disorders). This delay can be of varying lengths of time, depending on the history of the disease and/or individual being treated. As is evident to one skilled in the art, a sufficient or significant delay can, in effect, encompass prevention, in that the individual does not develop the disease. For example, a late stage cancer, such as development of metastasis, may be delayed.

A "reference" as used herein, refers to any sample, standard, or level that is used for comparison purposes. A reference may be obtained from a healthy and/or non-diseased sample. In some examples, a reference may be obtained from an untreated sample. In some examples, a reference is obtained from a non-diseased on non-treated sample of a subject individual. In some examples, a reference is obtained from one or more healthy individuals who are not the subject or patient.

"Preventing," as used herein, includes providing prophylaxis with respect to the occurrence or recurrence of a disease in a subject that may be predisposed to the disease but has not yet been diagnosed with the disease.

An "effective amount" of an agent refers to an amount effective, at dosages and for periods of time necessary, to achieve the desired therapeutic or prophylactic result.

A "therapeutically effective amount" of a substance/molecule of the invention, agonist or antagonist may vary according to factors such as the disease state, age, sex, and weight of the individual, and the ability of the substance/molecule, agonist or antagonist to elicit a desired response in the individual. A therapeutically effective amount is also one in which any toxic or detrimental effects of the substance/molecule, agonist or antagonist are outweighed by the therapeutically beneficial effects. A therapeutically effective amount may be delivered in one or more administrations.

A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount.

The terms "pharmaceutical formulation" and "pharmaceutical composition" refer to a preparation which is in such form as to permit the biological activity of the active ingredient(s) to be effective, and which contains no additional components which are unacceptably toxic to a subject to which the formulation would be administered. Such formulations may be sterile and essentially free of endotoxins.

A "pharmaceutically acceptable carrier" refers to a non-toxic solid, semisolid, or liquid filler, diluent, encapsulating material, formulation auxiliary, or carrier conventional in the art for use with a therapeutic agent that together comprise a "pharmaceutical composition" for administration to a subject. A pharmaceutically acceptable carrier is non-toxic to recipients at the dosages and concentrations employed and is compatible with other ingredients of the formulation. The pharmaceutically acceptable carrier is appropriate for the formulation employed.

A "sterile" formulation is aseptic or essentially free from living microorganisms and their spores.

Administration "in combination with" one or more further therapeutic agents includes simultaneous (concurrent) and consecutive or sequential administration in any order.

The term "concurrently" or "simultaneously" is used herein to refer to administration of two or more therapeutic agents, where at least part of the administration overlaps in time or where the administration of one therapeutic agent falls within a short period of time relative to administration of the other therapeutic agent. For example, the two or more therapeutic agents are administered with a time separation of no more than about 1 day, such as no more than about any of 60, 30, 15, 10,5, or 1 minutes.

The term "sequentially" is used herein to refer to administration of two or more therapeutic agents where the administration of one or more agent(s) continues after discontinuing the administration of one or more other agent(s). For example, administration of the two or more therapeutic agents are administered with a time separation of more than about 15 minutes, such as about any of 20, 30, 40, 50, or 60 minutes, 1 day, 2 days, 3 days, 1 week, 2 weeks, or 1 month.

As used herein, "in conjunction with" refers to administration of one treatment modality in addition to another treatment modality. As such, "in conjunction with" refers to administration of one treatment modality before, during or after administration of the other treatment modality to the individual.

The term "package insert" is used to refer to instructions customarily included in commercial packages of therapeutic products, that contain information about the indications, usage, dosage, administration, combination therapy, contraindications and/or warnings concerning the use of such therapeutic products.

An "article of manufacture" is any manufacture (e.g., a package or container) or kit comprising at least one reagent, *e.g.,* a medicament for treatment of a disease or disorder *(e.g.,* a hypoxia related disease or disorder), or a probe for specifically detecting a biomarker described herein. In certain embodiments, the manufacture or kit is promoted, distributed, or sold as a unit for performing the methods described herein.

### H-NOX Proteins

### Overview of H-NOX Protein Family

Unless otherwise indicated, any wild-type or mutant H-NOX protein can be used in the compositions, kits, and methods as described herein. As used herein, an "H-NOX protein" means a protein that has an H-NOX domain (named for Heme-Nitric oxide and OXygen binding domain). An H-NOX protein may or may not contain one or more other domains in addition to the H-NOX domain. H-NOX proteins are members of a highly-conserved, well-characterized family of hemoproteins (Iyer, L. M. et al. (February 3, 2003). BMC Genomics 4(1):5; Karow, D. S. et al. (August 10, 2004). Biochemistry 43(31):10203-10211; Boon, E. M. et al. (2005). Nature Chem. Biol. 1:53-59; Boon, E. M. et al. (October 2005). Curr. Opin. Chem. Biol. 9(5):441-446; Boon, E. M. et al. (2005). J. Inorg. Biochem. 99(4):892-902). H-NOX proteins are also referred to as Pfam 07700 proteins or HNOB proteins (Pfam - A database of protein domain family alignments and Hidden Markov Models, Copyright (C) 1996-2006 The Pfam Consortium; GNU LGPL Free Software Foundation, Inc., 59 Temple Place - Suite 330, Boston, MA 02111-1307, USA). In some embodiments, an H-NOX protein has, or is predicted to have, a secondary structure that includes six alpha-helices, followed by two beta-strands, followed by one alpha-helix, followed by two beta-strands. An H-NOX protein can be an apoprotein that is capable of binding heme or a holoprotein with heme bound. An H-NOX protein can covalently or non-covalently bind a heme group. Some H-NOX proteins bind NO but not O₂, and others bind both NO and O₂. H-NOX domains from facultative aerobes that have been isolated bind NO but not O₂. H-NOX proteins from obligate aerobic prokaryotes, C. *elegans,* and *D. melanogaster* bind NO and O₂. Mammals have two H-NOX proteins: β1 and β2. An alignment of mouse, rat, cow, and human H-NOX sequences shows that these species share >99% identity. In some embodiments, of the disclosure the H-NOX domain of an H-NOX protein or the entire H-NOX protein is at least about any of 10, 15, 20, 25, 30, 40, 50, 60, 70, 80, 90, 95, 97, 98, 99, or 99.5% identical to that of the corresponding region of a naturally-occurring *Thermoanaerobacter tengcongensis* H-NOX protein *(e.g.* SEQ ID NO:2) or a naturally-occurring sGC protein *(e.g.,* a naturallyoccurring sGC β1 protein). In some embodiments of the disclosure, the H-NOX domain of an H-NOX protein or the entire H-NOX protein is at least about any of 10-20%, 20-30%, 30-40%, 40-50%, 50-60%, 60-70%, 70-80%, 80-90%, 90-95%, 95-99, or 99-99.9% identical to that of the corresponding region of a naturally-occurring *Thermoanaerobacter tengcongensis* H-NOX protein *(e.g.* SEQ ID NO:2) or a naturally-occurring sGC protein *(e.g.,* a naturally-occurring sGC β1 protein). As discussed further herein, an H-NOX protein may optionally contain one or more mutations relative to the corresponding naturally-occurring H-NOX protein. In some embodiments, the H-NOX protein includes one or more domains in addition to the H-NOX domain. In particular embodiments, the H-NOX protein includes one or more domains or the entire sequence from another protein. For example, the H-NOX protein may be a fusion protein that includes an H-NOX domain and part or all of another protein, such as albumin (e.g., human serum albumin). In some embodiments, only the H-NOX domain is present. In some embodiments, the H-NOX protein does not comprise a guanylyl cyclase domain. In some embodiments, the H-NOX protein comprises a tag; for example, a His₆ tag.

### Polymeric H-NOX proteins

Disclosed are polymeric H-NOX proteins comprising two or more H-NOX domains. The two or more H-NOX domains may be covalently linked or noncovalently linked. In some embodiments of the disclosure, the polymeric H-NOX protein is in the form of a dimer, a trimer, a tetramer, a pentamer, a hexamer, a heptamer, an octomer, a nanomer, or a decamer. In some embodiments of the disclosure, the polymeric H-NOX protein comprises homologous H-NOX domains. In some embodiments, the polymeric H-NOX protein comprises heterologous H-NOX domains; for example, the H-NOX domains may comprises amino acid variants of a particular species of H-NOX domain or may comprise H-NOX domains from different species. In some embodiments, at least one of the H-NOX domains of a polymeric H-NOX protein comprises a mutation corresponding to an L144F mutation of *T. tengcongensis* H-NOX. In some embodiments, the polymeric H-NOX proteins comprise one or more polymerization domains. In some embodiments, the polymeric H-NOX protein is a trimeric H-NOX protein. In some embodiments, the polymeric H-NOX protein comprises at least one trimerization domain. In some embodiments, the trimeric H-NOX protein comprises three *T. tengcongensis* H-NOX domains. In some embodiments the trimeric H-NOX domain comprises three *T. tengcongensis* L144F H-NOX domains.

In some aspects of the disclosure, the polymeric H-NOX protein comprises two or more associated monomers. The monomers may be covalently linked or noncovalently linked. In some embodiments, monomeric subunits of a polymeric H-NOX protein are produced where the monomeric subunits associate in vitro or in vivo to form the polymeric H-NOX protein. In some embodiments, the monomers comprise an H-NOX domain and a polymerization domain. In some embodiments, the polymerization domain is covalently linked to the H-NOX domain; for example, the C-terminus of the H-NOX domain is covalently linked to the N-terminus or the C-terminus of the polymerization domain. In other embodiments, the N-terminus of the H-NOX domain is covalently linked to the N-terminus or the C-terminus of the polymerization domain. In some embodiments, an amino acid spacer is covalently linked between the H-NOX domain and the polymerization domain. An "amino acid spacer" and an "amino acid linker" are used interchangeably herein. In some embodiments, at least one of the monomeric subunits of a polymeric H-NOX protein comprises a mutation corresponding to an L144F mutation of *T. tengcongensis* H-NOX. In some embodiments the polymeric H-NOX protein is a trimeric H-NOX protein. In some embodiments, the monomer of a trimeric H-NOX protein comprises an H-NOX domain and a foldon domain of T4 bacteriophage. In some embodiments, the monomer of a trimeric H-NOX protein comprises a *T. tengcongensis* H-NOX domain and a foldon domain. In some embodiments, the monomer of a trimeric H-NOX protein comprises a *T. tengcongensis* L144F H-NOX domain and a foldon domain. In some embodiments, the trimer H-NOX protein comprises three monomers, each monomer comprising a *T. tengcongensis* L144F H-NOX domain and a foldon domain. In some embodiments, the H-NOX domain is linked to the foldon domain with an amino acid linker; for example a Gly-Ser-Gly linker. In some embodiments, at least one H-NOX domain comprises a tag. In some embodiments, at least one H-NOX domain comprises a His₆ tag. In some embodiments, the His₆ tag is linked to the foldon domain with an amino acid linker; for example an Arg-Gly-Ser linker. In some embodiments, all of the H-NOX domains comprise a His₆ tag. In some embodiments, the trimeric H-NOX protein comprises the amino acid sequence set forth in SEQ ID NO:6 or SEQ ID NO:8.

The exemplary H-NOX domain from *T. tengcongensis* is approximately 26.7 kDal. In some embodiments, the polymeric H-NOX protein has an atomic mass greater than any of about 50 kDal, 75 kDal, 100 kDal, 125 kDal, to about 150 kDal.

Disclosed are polymeric H-NOX proteins that show greater accumulation in one or more tissues in an individual compared to a corresponding monomeric H-NOX protein comprising a single H-NOX domain following administration of the H-NOX protein to the individual. A corresponding H-NOX protein refers to a monomeric form of the H-NOX protein comprising at least one of the H-NOX domains of the polymeric H-NOX protein. Tissues of preferential polymeric H-NOX accumulation include, but are not limited to tissue with damaged vasculature. In some embodiments the polymeric H-NOX protein persists in a mammal for at least about 1, 2, 3, 4, 6, 12 or 24 hours or at least about 1, 2, 3, 4, 5, 6, 7, 8, 9 or 10 days following administration of the H-NOX protein to the individual. In some embodiments the polymeric H-NOX protein persists in a mammal for about 1-2, 2-3, 3-4, 4-6, 6-12 or 12-24 hours or 1-2 days, 2-4 days, 4-8 days, 8-10 days or greater than 10 days following administration of the H-NOX protein to the individual In some embodiments, less than about 10% of the polymeric H-NOX is cleared from mammal by the kidneys within less than any of about 1 hour, 2 hours or 3 hours following administration of the H-NOX protein to the individual.

### Sources of H-NOX Proteins and H-NOX domains

H-NOX proteins and H-NOX domains from any genus or species can be used in the compositions, kits, and methods described herein. In various embodiments, the H-NOX protein or the H-NOX domains of a polymeric H-NOX protein is a protein or domain from a mammal *(e.g.,* a primate *(e.g.,* human, monkey, gorilla, ape, lemur, *etc),* a bovine, an equine, a porcine, a canine, or a feline), an insect, a yeast, or a bacteria or is derived from such a protein. Exemplary mammalian H-NOX proteins include wild-type human and rat soluble guanylate cyclase (such as the β1 subunit). Examples of H-NOX proteins include wild-type mammalian H-NOX proteins, *e.g. H. sapiens, M. musculus, C. familiaris, B. Taurus, C. lupus* and *R. norvegicus;* and wild-type non-mammalian vertebrate H-NOX proteins, *e.g,. X. laevis, O. latipes, O. curivatus,* and F. *rubripes.* Examples of non-mammalian wild-type NO-binding H-NOX proteins include wild-type H-NOX proteins of *D. melanogaster, A. gambiae,* and *M. sexta*; examples of non-mammalian wild-type O₂-binding H-NOX proteins include wild-type H-NOX proteins of C. *elegans* gcy-31, gcy-32, gcy-33, gcy-34, gcy-35, gcy-36, and gcy-37; *D. melanogaster* CG14885, CG14886, and CG4154; and *M. sexta* beta-3; examples of prokaryotic wild-type H-NOX proteins include *T. tengcongensis, V. cholera, V. fischerii, N. punctiforme*, *D. desulfuricans, L. pneumophila 1, L. pneumophila* 2, and C. *acetobutylicum.*

NCBI Accession numbers for exemplary H-NOX proteins include the following: *Homo sapiens* β1 [gi:2746083], *Rattus norvegicus* β1 [gi:27127318], *Drosophila* melanogater β1 [gi:861203], *Drosophila* melanogater CG14885-PA [gi:23171476], *Caenorhabditis elegans* GCY-35 [gi:52782806], *Nostoc punctiforme* [gi:23129606], *Caulobacter crescentus* [gi:16127222], *Shewanella oneidensis* [gi:24373702], *Legionella pneumophila* (ORF 2) [CUCGC_272624], *Clostridium acetobutylicum* [gi:15896488], and *Thermoanaerobacter tengcongensis* [gi:20807169]. *Canis lupus* H-NOX is provided by GenBank accession DQ008576. Nucleic acid and amino acid sequences of exemplary H-NOX proteins and domains are provided in Figure 37.

Exemplary H-NOX protein also include the following H-NOX proteins that are listed by their gene name, followed by their species abbreviation and Genbank Identifiers (such as the following protein sequences available as of May 21, 2006; May 22, 2006; May 21, 2007; or May 22, 2007): Npun5905_Npu_23129606, alr2278_Ana_17229770, SO2144 Sone 24373702, Mdeg1343_Mde_23027521, VCA0720_Vch_15601476,CC2992_Ccr_16127222, Rsph2043_Rhsp_22958463 (gi:46192757), Mmc10739_Mcsp_22999020, Tar4_Tte_20807169,Ddes2822_Dde_23475919,CAC3243_Cac_15896488 ,gcy-31_Ce_17568389, CG14885_Dm_24647455,GUCYlB3_Hs_4504215, HpGCS-beta1_Hpul_14245738,_Gycbeta100B_Dm_24651577, CG4154_Dm_24646993 (gi:NP_650424.2, gi:62484298), gcy-32_Ce_13539160,gcy-36_Ce_17568391 (gi:32566352, gi:86564713), gcy-35_Ce-17507861 (gi:71990146), gcy-37_Ce_17540904 (gi:71985505), GCYla3_Hs_20535603, GCY1a2-Hs_899477, or GYCa-99B_Dm_729270 (gi:68067738) (Lakshminarayan et al. (2003). BMG Genomics 4:5-13). The species abbreviations used in these names include Ana - *Anabaena Sp;* Ccr - *Caulobacter crescentus;* Cac - *Clostridium acetobutylicum;* Dde - *Desulfovibrio desulfuricans;* Mcsp - *Magnetococcus sp.;* Mde - *Microbulbifer degradans;* Npu - *Nostoc punctiforme*; Rhsp - *Rhodobacter sphaeroides;* Sone - *Shewanella oneidensis;* Tte - *Thermoanaerobacter tengcongensis;* Vch - *Vibrio cholerae;* Ce - *Caenorhabditis elegans;* Dm - *Drosophila melanogaster;* Hpul - *Hemicentrotus pulcherrimus;* Hs - *Homo sapiens.*

Other exemplary H-NOX proteins include the following H-NOX proteins that are listed by their organism name and Pfam database accession number (such as the following protein sequences available as of May 21, 2006; May 22, 2006; May 17, 2007; May 21, 2007; or May 22, 2007, *Caenorhabditis briggsae* Q622M5_CAEBR, *Caenorhabditis briggsae* Q61P44_CAEBR, *Caenorhabditis briggsae* Q61R54_CAEBR, *Caenorhabditis briggsae* Q61V90_CAEBR, *Caenorhabditis briggsae* Q61A94_CAEBR, *Caenorhabditis briggsae* Q60TP4_CAEBR, *Caenorhabditis briggsae* Q60M10_CAEBR, *Caenorhabditis elegans* GCY37_CAEEL, *Caenorhabditis elegans* GCY31_CAEEL, *Caenorhabditis elegans* GCY36_CAEEL, *Caenorhabditis elegans* GCY32_CAEEL, *Caenorhabditis elegans* GCY35_CAEEL, *Caenorhabditis elegans* GCY34_CAEEL, *Caenorhabditis elegans* GCY33_CAEEL, *Oryzias curvinotus* Q7T040_ORYCU, *Oryzias curvinotus* Q75WF0_ORYCU, *Oryzias latipes* P79998_ORYLA, *Oryzias latipes* Q7ZSZ5_ORYLA, *Tetraodon nigroviridis* Q4SW38_TETNG, *Tetraodon nigroviridis* Q4RZ94_TETNG, *Tetraodon nigroviridis* Q4S6K5_TETNG, *Fugu rubripes* Q90VY5_FUGRU, *Xenopus laevis* Q6INK9_XENLA, *Homo sapiens* Q5T8J7_HUMAN, *Homo sapiens* GCYA2_HUMAN, *Homo sapiens* GCYB2_HUMAN, *Homo sapiens* GCYB 1_HUMAN, *Gorilla gorilla* Q9N193_9PRIM, *Pongo pygmaeus* Q5RAN8_PONPY, *Pan troglodytes* Q9N192_PANTR, *Macaca mulatta* Q9N194_MACMU, *Hylobates lar* Q9N191_HYLLA, *Mus musculus* Q8BXH3_MOUSE, *Mus musculus* GCYB 1-MOUSE, *Mus musculus* Q3UTI4_MOUSE, *Mus musculus* Q3UH83_MOUSE, *Mus musculus* Q6XE41_MOUSE, *Mus musculus* Q80YP4_MOUSE, *Rattus norvegicus* Q80WX7_RAT, *Rattus norvegicus* Q80WX8_RAT, *Rattus norvegicus* Q920Q1_RAT, *Rattus norvegicus* Q54A43_RAT, *Rattus norvegicus* Q80WY0_RAT, *Rattus norvegicus* Q80WY4_RAT, *Rattus norvegicus* Q8CH85_RAT, *Rattus norvegicus* Q80WY5_RAT, *Rattus norvegicus* GCYB1_RAT, *Rattus norvegicus* Q8CH90_RAT, *Rattus norvegicus* Q91XJ7_RAT, *Rattus norvegicus* Q80WX9_RAT, *Rattus norvegicus* GCYB2_RAT, *Rattus norvegicus* GCYA2_RAT, *Canis familiaris* Q4ZHR9_CANFA, *Bos taurus* GCYB1_BOVIN, *Sus scrofa* Q4ZHR7_PIG, *Gryllus bimaculatus* Q59HN5_GRYBI, *Manduca sexta* O77106_MANSE, *Manduca sexta* O76340 MANSE, *Apis mellifera* Q5UAF0_APIME, *Apis mellifera* Q5FAN0_APIME, *Apis mellifera* Q6L5L6_APIME, *Anopheles gambiae* str PEST Q7PYK9_ANOGA, *Anopheles gambiae* str PEST Q7Q9W6_ANOGA, *Anopheles gambiae* str PEST Q7QF3 1_ANOGA, *Anopheles gambiae* str PEST Q7PS01_ANOGA, *Anopheles gambiae* str PEST Q7PFY2_ANOGA, *Anopheles gambiae* Q7KQ93_ANOGA, *Drosophila melanogaster* Q24086_DROME, *Drosophila melanogaster* GCYH_DROME, *Drosophila melanogaster* GCY8E_DROME, *Drosophila melanogaster* GCYDA_DROME, *Drosophila melanogaster* GCYDB_DROME, *Drosophila melanogaster* Q9VA09_DROME, *Drosophila pseudoobscura* Q29CE1_DROPS, *Drosophila pseudoobscura* Q296C7_DROPS, *Drosophila pseudoobscura* Q296C8_DROPS, *Drosophila pseudoobscura* Q29BU7_DROPS, *Aplysia californica* Q7YWK7_APLCA, *Hemicentrotus pulcherrimus* Q95NK5_HEMPU, *Chlamydomonas reinhardtii,* Q5YLC2_CHLRE, *Anabaena sp* Q8YUQ7_ANASP, *Flavobacteria bacterium BBFL7* Q26GR8_9BACT, *Psychroflexus torquis* ATCC *700755* Q1VQE5_9FLAO, marine gamma proteobacterium HTCC2207 Q1YPJ5_9GAMM, marine gamma proteobacterium HTCC2207 Q1YTK4_9GAMM, *Caulobacter crescentus* Q9A451_CAUCR, *Acidiphilium cryptum* JF-5 Q2DG60_ACICY, *Rhodobacter sphaeroides* Q3JOU9_RHOS4, *Silicibacter pomeroyi* Q5LPV1 SILPO, *Paracoccus denitrificans* PD1222, Q3PC67_PARDE, *Silicibacter sp TM1040* Q3QNY2_9RHOB, *Jannaschia sp* Q28ML8_JANSC, *Magnetococcus sp* MC-1 Q3XT27_9PROT, *Legionella pneumophila* Q5WXP0_LEGPL, *Legionella pneumophila* Q5WTZ5_LEGPL, *Legionella pneumophila* Q5X268_LEGPA, *Legionella pneumophila* Q5X2R2_LEGPA, *Legionella pneumophila subsp pneumophila* Q5ZWM9_LEGPH, *Legionella pneumophila subsp pneumophila* Q5ZSQ8_LEGPH, *Colwellia psychrerythraea* Q47Y43_COLP3, *Pseudoalteromonas atlantica* T6c Q3CSZ5_ALTAT, *Shewanella oneidensis* Q8EF49_SHEON, *Saccharophagus degradans* Q21E20_SACD2, *Saccharophagus degradans* Q21ER7_SACD2, *Vibrio angustum* S14 Q1ZWE5_9VIBR, *Vibrio vulnificus* Q8DAE2_VIBVU, *Vibrio alginolyticus* 12G01 Q1VCP6_VIBAL, *Vibrio sp* DAT722 Q2FA22_9VIBR, *Vibrio parahaemolyticus* Q87NJ1_VIBPA, *Vibrio fischeri* Q5ElF5_VIBF1, *Vibrio vulnificus* Q7MJS8_VIBVY, *Photobacterium sp SKA34* Q2C6Z5_9GAMM, *Hahella chejuensis* Q2SFY7_HAHCH, *Oceanospirillum sp* MED92 Q2BKV0_9GAMM, *Oceanobacter sp* RED65 QlN035_9GAMM, *Desulfovibrio desulfuricans* Q310U7_DESDG, *Halothermothrix orenii* H 168 Q2AIW5_9FIRM, *Thermoanaerobacter tengcongensis* Q8RBX6_THETN, *Caldicellulosiruptor saccharolyticus* DSM 8903 Q2ZH17_CALSA, *Clostridium acetobutylicum* Q97E73_CLOAB, *Alkaliphilus metalliredigenes* QYMF Q3C763_9CLOT, *Clostridium tetani* Q899J9_CLOTE, and *Clostridium beijerincki NCIMB 8052* Q2WVN0_CLOBE. These sequences are predicted to encode H-NOX proteins based on the identification of these proteins as belonging to the H-NOX protein family using the Pfam database as described herein.

Additional H-NOX proteins, H-NOX domains of polymeric H-NOX proteins, and nucleic acids, which may be suitable for use in the pharmaceutical compositions and methods described herein, can be identified using standard methods. For example, standard sequence alignment and/or structure prediction programs can be used to identify additional H-NOX proteins and nucleic acids based on the similarity of their primary and/or predicted protein secondary structure with that of known H-NOX proteins and nucleic acids. For example, the Pfam database uses defined alignment algorithms and Hidden Markov Models (such as Pfam 21.0) to categorize proteins into families, such as the H-NOX protein family (Pfam - A database of protein domain family alignments and Hidden Markov Models, Copyright (C) 1996-2006 The Pfam Consortium; GNU LGPL Free Software Foundation, Inc., 59 Temple Place - Suite 330, Boston, MA 02111-1307, USA). Standard databases such as the swissprot-trembl database (world-wide web at "expasy.org", Swiss Institute of Bioinformatics Swiss-Prot group CMU - 1 rue Michel Servet CH-1211 Geneva 4, Switzerland) can also be used to identify members of the H-NOX protein family. The secondary and/or tertiary structure of an H-NOX protein can be predicted using the default settings of standard structure prediction programs, such as PredictProtein (630 West, 168 Street, BB217, New York, N.Y. 10032, USA). Alternatively, the actual secondary and/or tertiary structure of an H-NOX protein can be determined using standard methods.

In some embodiments, the H-NOX domain has the same amino acid in the corresponding position as any of following distal pocket residues in *T. tengcongensis* H-NOX: Thr4, Ile5, Thr8, Trp9, Trp67, Asn74, Ile75, Phe78, Phe82, Tyr140, Leu144, or any combination of two or more of the foregoing. In some embodiments, the H-NOX domain has a proline or an arginine in a position corresponding to that of Pro115 or Arg135 of *T. tengcongensis* H-NOX, respectively, based on sequence alignment of their amino acid sequences. In some embodiments, the H-NOX domain has a histidine that corresponds to His105 of *R. norvegicus* β1 H-NOX. In some embodiments, the H-NOX domain has or is predicted to have a secondary structure that includes six alpha-helices, followed by two beta-strands, followed by one alpha-helix, followed by two beta-strands. This secondary structure has been reported for H-NOX proteins.

If desired, a newly identified H-NOX protein or H-NOX domain can be tested to determine whether it binds heme using standard methods. The ability of an H-NOX domain to function as an O₂ carrier can be tested by determining whether the H-NOX domain binds O₂ using standard methods, such as those described herein. If desired, one or more of the mutations described herein can be introduced into the H-NOX domain to optimize its characteristics as an O₂ carrier. For example, one or more mutations can be introduced to alter its O₂ dissociation constant, k_{off} for oxygen, rate of heme autoxidation, NO reactivity, NO stability or any combination of two or more of the foregoing. Standard techniques such as those described herein can be used to measure these parameters.

### Mutant H-NOX Proteins

As discussed further herein, an H-NOX protein or an H-NOX domain of a polymeric H-NOX protein may contain one or more mutations, such as a mutation that alters the O₂ dissociation constant, the k_{off} for oxygen, the rate of heme autoxidation, the NO reactivity, the NO stability, or any combination of two or more of the foregoing compared to that of the corresponding wild-type protein. In some embodiments, disclosed is a polymeric H-NOX protein comprising one or more H-NOX domains that may contain one or more mutations, such as a mutation that alters the O₂ dissociation constant, the k_{off} for oxygen, the rate of heme autoxidation, the NO reactivity, the NO stability, or any combination of two or more of the foregoing compared to that of the corresponding wild-type protein. Panels of engineered H-NOX domains may be generated by random mutagenesis followed by empirical screening for requisite or desired dissociation constants, dissociation rates, NO-reactivity, stability, physio-compatibility, or any combination of two or more of the foregoing in view of the teaching provided herein using techniques as described herein and, additionally, as known by the skilled artisan. Alternatively, mutagenesis can be selectively targeted to particular regions or residues such as distal pocket residues apparent from the experimentally determined or predicted three-dimensional structure of an H-NOX protein (see, for example, Boon, E. M. et al. (2005). Nature Chemical Biology 1:53-59, which is hereby referred to in its entirety, particularly with respect to the sequences of wild-type and mutant H-NOX proteins) or evolutionarily conserved residues identified from sequence alignments (see, for example, Boon E.M. et al. (2005). Nature Chemical Biology 1:53-59, which is hereby referred to in its entirety, particularly with respect to the sequences of wild-type and mutant H-NOX proteins).

In some embodiments of the disclosure, the mutant H-NOX protein or mutant H-NOX domain of a polymeric H-NOX protein has a sequence that differs from that of all H-NOX proteins or domains occurring in nature. In various embodiments, the amino acid sequence of the mutant protein is at least about any of 10, 15, 20, 25, 30, 40, 50, 60, 70, 80, 90, 95, 97, 98, 99, or 99.5% identical to that of the corresponding region of an H-NOX protein occurring in nature. In various embodiments, the amino acid sequence of the mutant protein is about 10-20%, 20-30%, 30-40%, 40-50%, 50-60%, 60-70%, 70-80%, 80-90%, 90-95%, 95-99%, or 99.5% identical to that of the corresponding region of an H-NOX protein occurring in nature. In some embodiments, the mutant protein is a protein fragment that contains at least about any of 25, 50, 75, 100, 150, 200, 300, or 400 contiguous amino acids from a full-length protein. In some embodiments, the mutant protein is a protein fragment that contains 25-50, 50-75, 75-100, 100-150, 150-200, 200-300, or 300-400 contiguous amino acids from a full-length protein. Sequence identity can be measured, for example, using sequence analysis software with the default parameters specified therein (e.g., Sequence Analysis Software Package of the Genetics Computer Group, University of Wisconsin Biotechnology Center, 1710 University Avenue, Madison, WI 53705). This software program matches similar sequences by assigning degrees of homology to various amino acids replacements, deletions, and other modifications.

In some embodiments of the invention, the mutant H-NOX protein or mutant H-NOX domain of a polymeric H-NOX protein comprises the insertion of one or more amino acids *(e.g.,* the insertion of 2, 3, 4, 5, 6, 7, 8, 9, or 10 amino acids). In some embodiments of the invention, the mutant H-NOX protein or mutant H-NOX domain comprises the deletion of one or more amino acids *(e.g.,* a deletion of N-terminal, C-terminal, and/or internal residues, such as the deletion of at least about any of 5, 10, 15, 25, 50, 75, 100, 150, 200, 300, or more amino acids or a deletion of 5-10, 10-15, 15-25, 25-50, 50-75, 75-100, 100-150, 150-200, 200-300, or 300-400 amino acids). In some embodiments of the invention, the mutant H-NOX protein or mutant H-NOX domain comprises the replacement of one or more amino acids *(e.g.,* the replacement of 1, 2, 3, 4, 5, 6, 7, 8, 9, or 10 amino acids), or combinations of two or more of the foregoing. In some embodiments, a mutant protein has at least one amino acid alteration compared to a protein occurring in nature. In some embodiments, a mutant nucleic acid sequence encodes a protein that has at least one amino acid alteration compared to a protein occurring in nature. In some embodiments, the nucleic acid is not a degenerate version of a nucleic acid occurring in nature that encodes a protein with an amino acid sequence identical to a protein occurring in nature.

In some embodiments the mutation in the H-NOX protein or H-NOX domain of a polymeric H-NOX protein is an evolutionary conserved mutations (also denoted class I mutations). Examples of class I mutations are listed in Table 1A. In Table 1A, mutations are numbered/annotated according to the sequence of human β1 H-NOX, but are analogous for all H-NOX sequences. Thus, the corresponding position in any other H-NOX protein can be mutated to the indicated residue. For example, Phe4 of human β1 H-NOX can be mutated to a tyrosine since other H-NOX proteins have a tyrosine in this position. The corresponding phenylalanine residue can be mutated to a tyrosine in any other H-NOX protein. In particular embodiments, the one or more mutations are confined to evolutionarily conserved residues. In some embodiments, the one or more mutations may include at least one evolutionarily conserved mutation and at least one non-evolutionarily conserved mutation. If desired, these mutant H-NOX proteins are subjected to empirical screening for NO/O₂ dissociation constants, NO-reactivity, stability, and physio-compatibility in view of the teaching provided herein.

**Table 1A. Exemplary Class I H-NOX mutations targeting evolutionary conserved residues**

| | | |
|---|---|---|
| F4Y | Q30G | I145Y |
| F4L | E33P | I145H |
| H7G | N61G | K151E |
| A8E | C78H | I157F |
| L9W | A109F | E183F |

In some embodiments, the mutation is a distal pocket mutation, such as mutation of a residue in alpha-helix A, D, E, or G (Pellicena, P. et al. (August 31, 2004). Proc Natl. Acad Sci USA 101(35): 12854-12859). Exemplary distal pocket mutations (also denoted class II mutations) are listed in Table 1B. In Table 1B, mutations are numbered/annotated according to the sequence of human β1 H-NOX, but are analogous for all H-NOX sequences. Because several substitutions provide viable mutations at each recited residue, the residue at each indicated position can be changed to any other naturally or non-naturally-occurring amino acid (denoted "X"). Such mutations can produce H-NOX proteins with a variety of desired affinity, stability, and reactivity characteristics.

**Table 1B. Exemplary Class II H-NOX mutations targeting distal pocket residues**

| | | |
|---|---|---|
| V8X | M73X | I145X |
| L9X | F77X | I149X |
| F70X | C78X | |

In particular embodiments, the mutation is a heme distal pocket mutation. As described herein, a crucial molecular determinant that prevents O₂ binding in NO-binding members of the H-NOX family is the lack of an H-bond donor in the distal pocket of the heme. Accordingly, in some embodiments, the mutation alters H-bonding between the H-NOX domain and the ligand within the distal pocket. In some embodiments, the mutation disrupts an H-bond donor of the distal pocket and/or imparts reduced O₂ ligand-binding relative to the corresponding wild-type H-NOX domain. Exemplary distal pocket residues include Thr4, Ile5, Thr8, Trp9, Trp67, Asn74, I1e75, Phe78, Phe82, Tyr140, and Leu144 of *T. tengcongensis* H-NOX and the corresponding residues in any other H-NOX protein. In some embodiments, the H-NOX protein or H-NOX domain of a polymeric H-NOX protein comprises one or more distal pocket mutations. In some embodiments, the H-NOX protein or H-NOX domain of a polymeric H-NOX protein comprises one, two, three, four, five, six, seven, eight, nine, ten or more than ten distal pocket mutations. In some embodiments, the distal pocket mutation corresponds to a L144F mutation of *T. tengcongensis* H-NOX. In some embodiments, the distal pocket mutation is a L144F mutation of *T. tengcongensis* H-NOX. In some embodiments, H-NOX protein or the H-NOX domain of a polymeric H-NOX protein comprises two distal pocket mutations.

Residues that are not in the distal pocket can also affect the three-dimensional structure of the heme group; this structure in turn affects the binding of O₂ and NO to iron in the heme group. Accordingly, in some embodiments, the H-NOX protein or H-NOX domain of a polymeric H-NOX protein has one or more mutations outside of the distal pocket. Examples of residues that can be mutated but are not in the distal pocket include Pro115 and Arg135 of *T. tengcongensis* H-NOX. In some embodiments, the mutation is in the proximal pocket which includes His105 as a residue that ligates to the heme iron.

In some embodiments when two or more mutations are present; at least one mutation is in the distal pocket, and at least one mutation is outside of the distal pocket *(e.g.,* a mutation in the proximal pocket). In some embodiments, all the mutations are in the distal pocket.

To reduce the immunogenicity of H-NOX protein or H-NOX domains derived from sources other than humans, amino acids in an H-NOX protein or H-NOX domain can be mutated to the corresponding amino acids in a human H-NOX. For example, one or more amino acids on the surface of the tertiary structure of a non-human H-NOX protein or H-NOX domain can be mutated to the corresponding amino acid in a human H-NOX protein or H-NOX domain. In some variations, mutation of one or more surface amino acids may be combined with mutation of two or more distal pocket residues, mutation of one or more residues outside of the distal pocket *(e.g.,* a mutation in the proximal pocket), or combinations of two or more of the foregoing.

The invention also relates to any combination of mutation described herein, such as double, triple, or higher multiple mutations. For example, combinations of any of the mutations described herein can be made in the same H-NOX protein. Note that mutations in equivalent positions in other mammalian or non-mammalian H-NOX proteins are also encompassed by this invention. Exemplary mutant H-NOX proteins or mutant H-NOX domains comprise one or more mutations that impart altered O₂ or NO ligand-binding relative to the corresponding wild-type H-NOX domain and are operative as a physiologically compatible mammalian O₂ blood gas carrier.

The residue number for a mutation indicates the position in the sequence of the particular H-NOX protein being described. For example, *T. tengcongensis* I5A refers to the replacement of isoleucine by alanine at the fifth position in *T. tengcongensis* H-NOX. The same isoleucine to alanine mutation can be made in the corresponding residue in any other H-NOX protein or H-NOX domain (this residue may or may not be the fifth residue in the sequence of other H-NOX proteins). Since the amino acid sequences of mammalian β1 H-NOX domains differ by at most two amino acids, mutations that produce desirable mutant H-NOX proteins or H-NOX domains when introduced into wild-type rat β1 H-NOX proteins are also expected to produce desirable mutant H-NOX proteins or H-NOX domains when introduced into wild-type β1 H-NOX proteins or H-NOX domains from other mammals, such as humans.

In some embodiments, the H-NOX protein is a trimer comprising three *T. tengcongensis* L144F H-NOX domains and three foldon domains. In some embodiments, the H-NOX protein is a trimer comprising three *T. tengcongensis* wildtype H-NOX domains and three foldon domains.

### Modifications to H-NOX Proteins

Any of the wild-type or mutant H-NOX proteins, including polymeric H-NOX proteins, can be modified and/or formulated using standard methods to enhance therapeutic or industrial applications. For example, and particularly as applied to heterologous engineered H-NOX proteins, a variety of methods are known in the art for insulating such agents from immune surveillance, including crosslinking, PEGylation, carbohydrate decoration, *etc. (e.g.,* Rohlfs, R. J. et al. (May 15, 1998). J. Biol. Chem. 273(20): 12128-12134; Migita, R. et al. (June 1997). J. Appl. Physiol. 82(6):1995-2002; Vandegriff, K. D. et al. (August 15, 2004). Biochem J. 382(Pt 1): 183-189, which are each hereby referred to in their entireties, particularly with respect to the modification of proteins) as well as other techniques known to the skilled artisan. Fusing an H-NOX protein, including a polymeric H-NOX protein, with a human protein such as human serum albumin can increase the serum half-life, viscosity, and colloidal oncotic pressure. In some embodiments, an H-NOX protein is modified during or after its synthesis to decrease its immunogenicity and/or to increase its plasma retention time. H-NOX proteins can also be encapsulated (such as encapsulation within liposomes or nanoparticles).

In some embodiments, the H-NOX protein is covalently bound to polyethylene glycol (PEG). An H-NOX protein covalently bound to polyethylene glycol can be referred to as PEGylated (referred to herein as "H-NOXP"). An H-NOX protein that is not covalently bound to polyethylene glycol can be referred to as non-PEGylated. In some embodiments, the H-NOXP protein is a trimer comprising three *T. tengcongensis* L144F H-NOX domains and three foldon domains. In some embodiments, at least one monomer of a trimeric H-NOXP protein is PEGylated. In some embodiments, each monomer of a trimeric H-NOXP protein is PEGylated. In some embodiments, a monomeric H-NOX comprises three PEG molecules. In some embodiments, a trimeric H-NOX comprises nine PEG molecules (three for each monomer). In some embodiments the PEG has a molecular weight of 5000.

In some embodiments, both PEGylated and non-PEGylated H-NOX is administered to an individual to treat stroke, transient ischemic attacks and hypoxic brain injury. In some embodiments, the PEGylated and non-PEGylated H-NOX is administered at the same time. In some embodiments, the ratio of PEGylated to non-PEGylated H-NOX administered to the individual is any of about 99:1, 95:5, 90:10, 85:15, 80:20, 75:25, 70:30, 65:35, 60:40; 55:45, 50:50, 45:55, 40:60, 35:65, 30:70, 25:75, 20:80, 15:85; 10:90; 5:95; 1:99 or any ratio therebetween. In some embodiments, the PEGylated and non-PEGylated H-NOX is in a composition. In some embodiments, the PEGylated and non-PEGylated H-NOX is in the composition at a ratio of about any of 99:1, 95:5, 90:10, 85:15, 80:20, 75:25, 70:30, 65:35, 60:40; 55:45, 50:50, 45:55, 40:60, 35:65, 30:70, 25:75, 20:80, 15:85; 10:90; 5:95; 1:99 or any ratio therebetween. In some embodiments, the H-NOX proteins include a PEGylated trimeric H-NOX protein comprising three *T. tengcongensis* L144F H-NOX domains and three foldon domains. In some embodiments, the H-NOX proteins include a non-PEGylated trimeric H-NOX protein comprising three *T. tengcongensis* L144F H-NOX domains and three foldon domains. In some embodiments, the H-NOX proteins include a PEGylated trimeric H-NOX protein comprising three *T. tengcongensis* L144F H-NOX domains and three foldon domains and a non-PEGylated trimeric H-NOX protein comprising three *T. tengcongensis* L144F H-NOX domains and three foldon domains.

In some embodiments, the H-NOX protein comprises one of more tags; *e.g.* to assist in purification of the H-NOX protein. Examples of tags include, but are not limited to His₆, FLAG, GST, and MBP. In some embodiments, the H-NOX protein comprises one of more His₆ tags. The one or more His₆ tags may be removed prior to use of the polymeric H-NOX protein; *e.g.* by treatment with an exopeptidase. In some embodiments, the H-NOX protein is a trimer comprising three *T. tengcongensis* L144F H-NOX domains, three foldon domains, and three His₆ tags. In some embodiments, the H-NOX protein is a trimer comprising three *T. tengcongensis* wildtype H-NOX domains, three foldon domains, and three His₆ tags.

### Polymerization domains

Disclosed are furthermore polymeric H-NOX proteins comprising two or more H-NOX domains and one or more polymerization domains. Polymerization domains are used to link two or more H-NOX domains to form a polymeric H-NOX protein. One or more polymerization domains may be used to produce dimers, trimers, tetramers, pentamers, *etc.* of H-NOX proteins. Polymerization domains are known in the art, such as: the foldon of T4 bacteriophage fibritin, Arc, POZ, coiled coil domains (including GCN4, leucine zippers, Velcro), uteroglobin, collagen, 3-stranded coiled colis (matrilin-1), thrombosporins, TRPV1-C, P53, Mnt, avadin, streptavidin, Bcr-Abl, COMP, verotoxin subunit B, CamKII, RCK, and domains from N ethylmaleimide-sensitive fusion protein, STM3548, KaiC, TyrR, Hcp1, CcmK4, GP41, anthrax protective antigen, aerolysin, a-hemolysin, C4b-binding protein, Mi-CK, arylsurfatase A, and viral capsid proteins. The polymerization domains may be covalently or non-covalently linked to the H-NOX domains. In some embodiments, a polymerization domain is linked to an H-NOX domain to form a monomer subunit such that the polymerization domains from a plurality of monomer subunits associate to form a polymeric H-NOX domain. In some embodiments, the C-terminus of an H-NOX domain is linked to the N-terminus of a polymerization domain. In other embodiments, the N-terminus of an H-NOX domain is linked to the N-terminus of a polymerization domain. In yet other emodiments, the C-terminus of an H-NOX domain is linked to the C-terminus of a polymerization domain. In some emodiments, the N-terminus of an H-NOX domain is linked to the C-terminus of a polymerization domain.

Linkers may be used to join a polymerization domain to an H-NOX domain; for example, for example, amino acid linkers. In some embodiments, a linker comprising any one of one, two, three, four, five, six, seven, eight, nine, ten or more than ten amino acids may be placed betweent the polymerization domain and the H-NOX domain. Exemplary linkers include but are not limited to Gly-Ser-Gly and Arg-Gly-Ser linkers.

### Bacteriophage T4 fibritin trimerization domain

An exemplary polymerization domain is the foldon domain of bacteriophage T4. The *wac* gene from the bacteriophage T4 encodes the fibritin protein, a 486 amino acid protein with a C-terminal trimerization domain (residues 457-483) (Efimov, V. P. et al. (1994) J Mol Biol 242:470-486). The domain is able to trimerize fibritin both *in vitro* and *in vivo* (Boudko, S. P. et al. (2002) Eur J Biochem 269:833-841; Letarov, A. V., et al., (1999) Biochemistry (Mosc)64:817-823; Tao, Y., et al., (1997) Structure 5:789-798). The isolated 27 residue trimerization domain, often referred to as the *"foldon domain,"* has been used to construct chimeric trimers in a number of different proteins (including HIV envelope glycoproteins (Yang, X. et al., (2002) J Virol 76:4634-4642), adenoviral adhesins (Papanikolopoulou, K., et al., (2004) J Biol Chem 279:8991-8998; Papanikolopoulou, K. et al. (2004) J Mol Biol 342:219-227), collagen (Zhang, C., et al. (2009) Biotechnol Prog 25:1660-1668), phage P22 gp26 (Bhardwaj, A., et al. (2008) Protein Sci 17:1475-1485), and rabies virus glycoprotein (Sissoeff, L., et al. (2005) J Gen Virol 86:2543-2552). An exemplary sequence of the foldon domain is shown in Figure 1 and provided by SEQ ID NO:4.

The isolated foldon domain folds into a single 0-hairpin structure and trimerizes into a β-propeller structure involving three hairpins (Guthe, S. et al. (2004) J Mol Biol 337:905-915). The structure of the foldon domain alone has been determined by NMR (Guthe, S. et al. (2004) J Mol Biol 337:905-915) and the structures of several proteins trimerized with the foldon domain have been solved by X-ray crystallography (Papanikolopoulou, K., et al., (2004) J Biol Chem 279:8991-8998; Stetefeld, J. et al. (2003) Structure 11:339-346; Yokoi, N. et al. (2010) Small 6:1873-1879). The domain folds and trimerizes rapidly reducing the opportunity for misfolding intermediates or off-pathway oligomerization products (Guthe, S. et al. (2004) J Mol Biol 337:905-915). The foldon domain is very stable, able to maintain tertiary structure and oligomerization in >10% SDS, 6.0M guanidine hydrochloride, or 80° C (Bhardwaj, A., et al. (2008) Protein Sci 17:1475-1485; Bhardwaj, A., et al. (2007) J Mol Biol 371:374-387) and can improve the stability of sequences fused to the foldon domain (Du, C. et al. (2008) Appl Microbiol Biotechnol 79:195-202.

In some embodiments, the C-terminus of an H-NOX domain is linked to the N-terminus of a foldon domain. In other embodiments, the N-terminus of an H-NOX domain is linked to the N-terminus of a foldon domain. In yet other emodiments, the C-terminus of an H-NOX domain is linked to the C-terminus of a foldon domain. In some emodiments, the N-terminus of an H-NOX domain is linked to the C-terminus of a foldon domain.

In some embodiments, linkers are be used to join a foldon domain to an H-NOX domain. In some embodiments, a linker comprising any one of one, two, three, four, five, six, seven, eight, nine, ten or more than ten amino acids may be placed betweent the polymerization domain and the H-NOX domain. Exemplary linkers include but are not limited to Gly-Ser-Gly and Arg-Gly-Ser linkers. Disclosed is furthemore a trimeric H-NOX protein comprising from N-terminus to C-terminus: a *T. tengcongensis* H-NOX domain, a Gly-Ser-Gly amino acid linker, and a foldon domain. Disclosed is furthemore a trimeric H-NOX protein comprising from N-terminus to C-terminus: a *T. tengcongensis* H-NOX domain, a Gly-Ser-Gly amino acid linker, a foldon domain, an Arg-Gly-Ser amino acid linker, and a His₆ tag. In some embodiments, the *T. tengcongensis* H-NOX domain comprises an L144F mutation. In some embodiments, the *T. tengcongensis* H-NOX domain is a wild-type H-NOX domain.

### Monomeric H-NOX domain subunits

Disclosed are recombinant monomeric H-NOX proteins (*i.e.* monomeric H-NOX subunits of polymeric H-NOX proteins) that can associate to form polymeric H-NOX proteins. Disclosed are furthemore recombinant H-NOX proteins comprising an H-NOX domain as described herein and a polymerization domain. The H-NOX domain and the polymerization domain may be covalently linked or noncovalently linked. In some embodiments, the C-terminus of an H-NOX domain of the recombinant monomeric H-NOX protein is linked to the N-terminus of a polymerization domain. In other embodiments, the N-terminus of an H-NOX domain of the recombinant monomeric H-NOX protein is linked to the N-terminus of a polymerization domain. In yet other emodiments, the C-terminus of an H-NOX domain of the recombinant monomeric H-NOX protein is linked to the C-terminus of a polymerization domain. In some emodiments, the N-terminus of an H-NOX domain of the recombinant monomeric H-NOX protein is linked to the C-terminus of a polymerization domain.In some embodiments, the recombinant monomeric H-NOX protein does not comprise a guanylyl cyclase domain.

In some embodiments, of the disclosure the monomeric H-NOX protein comprises a wild-type H-NOX domain. In some embodiments of the invention, the monomeric H-NOX protein comprises one of more mutations in the H-NOX domain. In some embodiments, the one or more mutations alter the O₂ dissociation constant, the k_{off} for oxygen, the rate of heme autooxidation, the NO reactivity, the NO stabilty or any combination of two or more of the foregoing compared to that of the corresponding wild-type H-NOX domain. In some embodiments, the mutation is a distal pocket mutation. In some embodiments, the mutation comprises a mutation that is not in the distal pocket. In some embodiments, the distal pocket mutation corresponds to a L144 mutation of *T. tengcongensis (e.g.* a L144F mutation).

Disclosed are furthemore recombinant monomeric H-NOX proteins that associate to form trimeric H-NOX proteins. In some embodiments, the recombinant H-NOX protein comprises an H-NOX domain and a trimerization domain. In some embodiments, the trimerization domain is a foldon domain as discussed herein. In some embodiments, the H-NOX domain is a *T. tengcongensis* H-NOX domain. In some embodiments the C-terminus of the *T. tengcongensis* H-NOX domain is covalently linked to the N-terminus of the foldon domain. In some embodiments the C-terminus of the *T. tengcongensis* H-NOX domain is covalently linked to the C-terminus of the foldon domain. In some embodiments, the *T. tengcongensis* domain is an L144F H-NOX domain. In some embodiments, of the disclosure the *T. tengcongensis* domain is a wild-type H-NOX domain.

In some embodiments, the H-NOX domain is covalently linked to the polymerization domain using an amino acid linker sequence. In some embodiments, the amino acid linker sequence is one, two, three, four, five, six, seven, eight, nine, ten or more than ten amino acids in length. Exemplary amino acid linker sequences include but are not limited to a Gly-Ser-Gly sequence and an Arg-Gly-Ser sequence. In some embodiments, the polymeric H-NOX protein is a trimeric H-NOX protein comprising three H-NOX domains and three trimerization sequences wherein the H-NOX domain is covalently linked to the trimerization domain via an amino acid linker sequence. In some embodiments, the monomeric H-NOX protein comprises the following from the N-terminus to the C-terminus: a *T. tengcongensis* L144F H-NOX domain, a Gly-Ser-Gly amino acid linker sequence, and a foldon domain. In some embodiments, the monomeric H-NOX protein comprises the following from the N-terminus to the C-terminus: a wild-type *T*. *tengcongensis* H-NOX domain, a Gly-Ser-Gly amino acid linker sequence, and a foldon domain.

In some embodiments, the recombinant monomeric H-NOX protein comprises a tag; *e.g.,* A His₆, a FLAG, a GST, or an MBP tag. In some embodiments, the recombinant monomeric H-NOX protein comprises a His₆ tag. In some embodiments, the recombinant monomeric H-NOX protein does not comprise a tag. In some embodiments, the tag *(e.g.* a His₆ tag) is covalently linked to the polymerization domain using an amino acid spacer sequence. In some embodiments, the amino acid linker sequence is one, two, three, four, five, six, seven, eight, nine, ten or more than ten amino acids in length. Exemplary amino acid linker sequences include but are not limited to a Gly-Ser-Gly sequence and an Arg-Gly-Ser sequence. In some embodiments, the polymeric H-NOX protein is a trimeric H-NOX protein comprising three H-NOX domains, three trimerization sequences, and three His₆ tags, wherein the H-NOX domain is covalently linked to the trimerization domain via an amino acid linker sequence and the trimerization domain is covalently linked to the His₆ tag via an amino acid linker sequence. In some embodiments, the monomeric H-NOX protein comprises the following from the N-terminus to the C-terminus: an L144F *T. tengcongensis* H-NOX domain, a Gly-Ser-Gly amino acid linker sequence, a foldon domain, an Arg-Gly-Ser linker sequence, and a His₆ tag. In some embodiments, the monomeric H-NOX protein comprises the following from the N-terminus to the C-terminus: a wild-type *T. tengcongensis* H-NOX domain, a Gly-Ser-Gly amino acid linker sequence, a foldon domain, an Arg-Gly-Ser linker sequence, and a His₆ tag.

In some embodiments the recombinant monomeric H-NOX protein comprises the amino acid sequence of SEQ ID NO:6 or SEQ ID NO:8.

### Characteristics of Wild-type and Mutant H-NOX Proteins

As described herein, a large number of diverse H-NOX mutant proteins, including polymeric H-NOX proteins, providing ranges of NO and O₂ dissociation constants, O₂ k_{off}, NO reactivity, and stability have been generated. To provide operative blood gas carriers, the H-NOX proteins may be used to functionally replace or supplement endogenous O₂ carriers, such as hemoglobin. In some embodiments, H-NOX proteins such as polymeric H-NOX proteins are used to deliver O₂ to hypoxic tissue (*e.g.* a hypoxic penumbra). Accordingly, in some embodiments, an H-NOX protein has a similar or improved O₂ association rate, O₂ dissociation rate, dissociation constant for O₂ binding, NO stability, NO reactivity, autoxidation rate, plasma retention time, or any combination of two or more of the foregoing compared to an endogenous O₂ carrier, such as hemoglobin. In some embodiments, the H-NOX protein is a polymeric H-NOX protein. In some embodiments, the polymeric H-NOX protein is a trimeric H-NOX protein comprising three monomers, each monomer comprising a *T. tengcongensis* L144F H-NOX domain and a foldon domain. In some embodiments, the polymeric H-NOX protein is a trimeric H-NOX protein comprising three monomers, each monomer comprising a *T*. *tengcongensis* L144F H-NOX domain and a foldon domain.

In various embodiments, the k_{off} for O₂ for an H-NOX protein, including a polymeric H-NOX protein, is between about 0.01 to about 200 s⁻¹ at 20 °C, such as about 0.1 to about 200 s⁻¹, about 0.1 to 100 s⁻¹, about 1.0 to about 16.0 s⁻¹, about 1.35 to about 23.4 s⁻¹, about 1.34 to about 18 s⁻¹, about 1.35 to about 14.5 s⁻¹, about 0.21 to about 23. 4 s⁻¹, about1.35 to about 2.9 s⁻¹, about 2 to about 3 s⁻¹, about 5 to about 15 s⁻¹, or about 0.1 to about 1 s⁻¹. In some embodiments, the H-NOX protein has a k_{off} for oxygen that is less than or equal to about 0.65 s⁻¹ at 20 °C (such as between about 0.21 s⁻¹ to about 0.65 s⁻¹ at 20 °C).

In various embodiments, the kₒₙ for O₂ for an H-NOX protein, including a polymeric H-NOX protein, is between about 0.14 to about 60 µM⁻¹s⁻¹ at 20 °C, such as about 6 to about 60 µM⁻¹s⁻¹, about 6 to 12 µM⁻¹s⁻¹, about 15 to about 60 µM⁻¹s⁻¹, about 5 to about 18µM⁻ s⁻¹, or about 6 to about 15 µM⁻¹s⁻¹.

In various embodiments, the kinetic or calculated K_{D} for O₂ binding by an H-NOX protein, including a polymeric H-NOX protein, is between about 1 nM to 1 mM, about 1 µM to about 10 µM, or about 10 µM to about 50 µM. In some embodiments the calculated K_{D} for O₂ binding is any one of about 2 nM to about 2 µM, about 2µ M to about 1 mM, about 100 nM to about 1 µM, about 9 µM to about 50 µM, about 100 µM to about 1 mM, about 50 nM to about 10 µM, about 2 nM to about 50 µM, about 100 nM to about 1.9 µM, about 150 nM to about 1 µM, or about 100 nM to about 255 nM, about 20 nM to about 2 µM, 20 nM to about 75 nM, about 1 µM to about 2 µM, about 2 µM to about 10 µM, about 2 µM to about 9 µM, or about 100 nM to 500 nM at 20 °C. In some embodiments, the kinetic or calculated K_{D} for O₂ binding is less than about any of 100 nM, 80 nM, 50 nM, 30 nM, 25 nM, 20 nM, or 10 nM at 20 °C.

In various embodiments, the kinetic or calculated K_{D} for O₂ binding by an H-NOX protein, including a polymeric H-NOX protein, is within about 0.01 to about 100-fold of that of hemoglobin under the same conditions (such as at 20 °C), such as between about 0.1 to about 10-fold or between about 0.5 to about 2-fold of that of hemoglobin under the same conditions (such as at 20 °C). In various embodiments, the kinetic or calculated K_{D} for NO binding by an H-NOX protein is within about 0.01 to about 100-fold of that of hemoglobin under the same conditions (such as at 20 °C), such as between about 0.1 to about 10-fold or between about 0.5 to about 2-fold of that of hemoglobin under the same conditions (such as at 20 °C).

In some embodiments, less than about any of 50, 40, 30, 10, or 5% of an H-NOX protein, including a polymeric H-NOX protein, is oxidized after incubation for about any of 1, 2, 4, 6, 8, 10, 15, or 20 hours at 20 °C.

In various embodiments, the NO reactivity of an H-NOX protein, including a polymeric H-NOX protein, is less than about 700 s⁻¹ at 20 °C, such as less than about 600 s⁻¹, 500 s⁻¹, 400 s⁻¹, 300 s⁻¹, 200 s⁻¹, 100 s⁻¹, 75 s⁻¹, 50 s⁻¹, 25 s⁻¹, 20 s⁻¹, 10 s⁻¹, 50 s⁻¹, 3 s⁻¹, 2 s⁻¹, 1.8 s⁻¹, 1.5 s⁻¹, 1.2 s⁻¹, 1.0 s⁻¹, 0.8 s⁻¹, 0.7 s⁻¹, or 0.6 s⁻¹ at 20 °C. In various embodiments, the NO reactivity of an H-NOX protein is between about 0.1 to about 600 s⁻¹ at 20 °C, such as between about 0.5 to about 400 s⁻¹, about 0.5 to about 100 s⁻¹, about 0.5 to about 50 s⁻¹, about 0.5 to about 10 s⁻¹, about 1 to about 5 s⁻¹, or about 0.5 to about 2.1 s⁻¹ at 20 °C. In various embodiments, the reactivity of an H-NOX protein is at least about 10, 100, 1,000, or 10,000 fold lower than that of hemoglobin under the same conditions, such as at 20 °C.

In various embodiments, the rate of heme autoxidation of an H-NOX protein, including a polymeric H-NOX protein, is less than about 1.0 h⁻¹ at 37 °C, such as less than about any of 0.9 h⁻¹, 0.8 h⁻¹, 0.7 h⁻¹, 0.6 h⁻¹, 0.5 h⁻¹, 0.4 h⁻¹, 0.3 h⁻¹, 0.2 h⁻¹, 0.1 h⁻¹, or 0.05 h⁻¹ at 37 C. In various embodiments, the rate of heme autoxidation of an H-NOX protein is between about 0.006 to about 5.0 h⁻¹ at 37 °C, such as about 0.006 to about 1.0 h⁻¹, 0.006 to about 0.9 h⁻¹, or about 0.06 to about 0.5 h⁻¹ at 37 °C.

In various embodiments, a mutant H-NOX protein, including a polymeric H-NOX protein, has (a) an O₂ or NO dissociation constant, association rate (kₒₙ for O₂ or NO), or dissociation rate (k_{off} for O₂ or NO) within 2 orders of magnitude of that of hemoglobin, (b) has an NO affinity weaker *(e.g.,* at least about 10-fold, 100-fold, or 1000-fold weaker) than that of sGC β1, respectively, (c) an NO reactivity with bound O₂ at least 1000-fold less than hemoglobin, (d) an *in vivo* plasma retention time at least 2, 10, 100, or 1000-fold higher than that of hemoglobin, or (e) any combination of two or more of the foregoing.

Exemplary suitable O₂ carriers provide dissociation constants within two orders of magnitude of that of hemoglobin, *i.e.* between about 0.01 and 100-fold, such as between about 0.1 and 10-fold, or between about 0.5 and 2-fold of that of hemoglobin. A variety of established techniques may be used to quantify dissociation constants, such as the techniques described herein (Boon, E. M. et al. (2005). Nature Chem. Biol. 1:53-59; Boon, E. M. et al. (October 2005). Curr. Opin. Chem. Biol. 9(5):441-446; Boon, E. M. et al. (2005). J. Inorg. Biochem. 99(4):892-902), Vandegriff, K. D. et al. (August 15, 2004). Biochem J. 382(Pt 1):183-189, referred to which are each hereby in their entireties, particularly with respect to the measurement of dissociation constants), as well as those known to the skilled artisan. Exemplary O₂ carriers provide low or minimized NO reactivity of the H-NOX protein with bound O₂, such as an NO reactivity lower than that of hemoglobin. In some embodiments, the NO reactivity is much lower, such as at least about 10, 100, 1,000, or 10,000-fold lower than that of hemoglobin. A variety of established techniques may be used to quantify NO reactivity (Boon, E. M. et al. (2005). Nature Chem. Biol. 1:53-59; Boon, E. M. et al. (October 2005). Curr. Opin. Chem. Biol. 9(5):441-446; Boon, E. M. et al. (2005). J. Inorg. Biochem. 99(4):892-902), Vandegriff, K. D. et al. (August 15, 2004). Biochem J. 382(Pt 1):183-189, which are each hereby referred to in their entireties, particularly with respect to the measurement of NO reactivity) as well as those known to the skilled artisan. Because wild-type *T*. *tengcongensis* H-NOX has such a low NO reactivity, other wild-type H-NOX proteins and mutant H-NOX proteins may have a similar low NO reactivity. For example, *T. tengcongensis* H-NOX Y140H has an NO reactivity similar to that of wild-type *T. tengcongensis* H-NOX.

In addition, suitable O₂ carriers provide high or maximized stability, particularly *in vivo* stability. A variety of stability metrics may be used, such as oxidative stability *(e.g.,* stability to autoxidation or oxidation by NO), temperature stability, and *in vivo* stability. A variety of established techniques may be used to quantify stability, such as the techniques described herein (Boon, E. M. et al. (2005). Nature Chem. Biol. 1:53-59; Boon, E. M. et al. (October 2005). Curr. Opin. Chem. Biol. 9(5):441-446; Boon, E. M. et al. (2005). J. Inorg. Biochem. 99(4):892-902), as well as those known to the skilled artisan. For *in vivo* stability in plasma, blood, or tissue, exemplary metrics of stability include retention time, rate of clearance, and half-life. H-NOX proteins from thermophilic organisms are expected to be stable at high temperatures. In various embodiments, the plasma retention times are at least about 2-, 10-, 100-, or 1000-fold greater than that of hemoglobin *(e.g.* Bobofchak, K. M. et al. (August 2003). Am. J. Physiol. Heart Circ. Physiol. 285(2):H549-H561). As will be appreciated by the skilled artisan, hemoglobin-based blood substitutes are limited by the rapid clearance of cell-free hemoglobin from plasma due the presence of receptors for hemoglobin that remove cell-free hemoglobin from plasma. Since there are no receptors for H-NOX proteins in plasma, wild-type and mutant H-NOX proteins are expected to have a longer plasma retention time than that of hemoglobin. If desired, the plasma retention time can be increased by PEGylating or crosslinking an H-NOX protein or fusing an H-NOX protein with another protein using standard methods (such as those described herein and those known to the skilled artisan).

In various embodiments, the H-NOX protein, including a polymeric H-NOX protein, has an O₂ dissociation constant between about 1 nM to about 1 mM at 20 °C and a NO reactivity at least about 10-fold lower than that of hemoglobin under the same conditions, such as at 20 °C. In some embodiments, the H-NOX protein has an O₂ dissociation constant between about 1 nM to about 1 mM at 20 °C and a NO reactivity less than about 700 s⁻¹ at 20 °C *(e.g.,* less than about 600 s⁻¹, 500 s⁻¹, 100 s⁻¹, 20 s⁻¹, or 1.8 s⁻¹ at 20 °C). In some embodiments, the H-NOX protein has an O₂ dissociation constant within 2 orders of magnitude of that of hemoglobin and a NO reactivity at least about 10-fold lower than that of hemoglobin under the same conditions, such as at 20 °C. In some embodiments, the H-NOX protein has a k_{off} for oxygen between about 0.01 to about 200 s⁻¹ at 20 °C and an NO reactivity at least about 10-fold lower than that of hemoglobin under the same conditions, such as at 20 °C. In some embodiments, the H-NOX protein has a k_{off} for oxygen that is less than about 0.65 s⁻¹ at 20 °C (such as between about 0.21 s⁻¹ to about 0.64 s⁻¹ at 20 °C) and a NO reactivity at least about 10-fold lower than that of hemoglobin under the same conditions, such as at 20 °C. In some embodiments of the invention, the O₂ dissociation constant of the H-NOX protein is between about 1 nM to about 1 µM (1000 nM), about 1 µM to about 10 µM, or about 10 µM to about 50 µM. In particular embodiments, the O₂ dissociation constant of the H-NOX protein is between about 2 nM to about 50 µM, about 50 nM to about 10 µM, about 100 nM to about 1.9 µM, about 150 nM to about 1 µM, or about 100 nM to about 255 nM at 20 °C. In various embodiments, the O₂ dissociation constant of the H-NOX protein is less than about 80 nM at 20 °C, such as between about 20 nM to about 75 nM at 20 °C. In some embodiments, the NO reactivity of the H-NOX protein is at least about 100-fold lower or about 1,000 fold lower than that of hemoglobin, under the same conditions, such as at 20 °C. In some embodiments, the NO reactivity of the H-NOX protein is less than about 700 s⁻¹ at 20 °C, such as less than about 600 s⁻¹, 500 s⁻¹, 400 s⁻¹, 300 s⁻¹, 200 s⁻¹, 100 s⁻¹, 75 s⁻¹, 50 s⁻¹, 25 s⁻¹, 20 s⁻¹, 10 s⁻¹, 50 s⁻¹, 3 s⁻¹, 2 s⁻¹, 1.8 s⁻¹, 1.5 s⁻¹, 1.2 s⁻¹, 1.0 s⁻¹, 0.8 s⁻¹, 0.7 s⁻¹, or 0.6 s⁻¹ at 20 °C. In some embodiments, the k_{off} for oxygen of the H-NOX protein is between 0.01 to 200 s⁻¹ at 20 °C, such as about 0.1 to about 200 s⁻¹, about 0.1 to 100 s⁻¹, about 1.35 to about 23.4 s⁻¹, about 1.34 to about 18 s⁻¹, about 1.35 to about 14.5 s⁻¹, about 0.21 to about 23. 4 s⁻¹, about 2 to about 3 s⁻¹, about 5 to about 15 s⁻¹, or about 0.1 to about 1 s⁻¹. In some embodiments, the O₂ dissociation constant of the H-NOX protein is between about 100 nM to about 1.9 µM at 20 °C, and the k_{off} for oxygen of the H-NOX protein is between about 1.35 s⁻¹ to about 14.5 s⁻¹ at 20 °C. In some embodiments, the rate of heme autoxidation of the H-NOX protein is less than about 1 h⁻¹ at 37 °C, such as less than about any of 0.9 h⁻¹, 0.8 h⁻¹, 0.7 h⁻¹, 0.6 h⁻¹, 0.5 h⁻¹, 0.4 h⁻¹, 0.3 h⁻¹, 0.2 h⁻¹, or 0.1 h⁻¹. In some embodiments, the k_{off} for oxygen of the H-NOX protein is between about 1.35 s⁻¹ to about 14.5 s⁻¹ at 20 °C, and the rate of heme autoxidation of the H-NOX protein is less than about 1 h⁻¹ at 37 °C. In some embodiments, the k_{off} for oxygen of the H-NOX protein is between about 1.35 s⁻¹ to about 14.5 s⁻¹ at 20 °C, and the NO reactivity of the H-NOX protein is less than about 700 s⁻¹ at 20 °C *(e.g.,* less than about 600 s⁻¹, 500 s¹, 100 s⁻¹, 20 s⁻¹, or 1.8 s⁻¹ at 20 °C). In some embodiments, the rate of heme autoxidation of the H-NOX protein is less than about 1 h⁻¹ at 37 °C, and the NO reactivity of the H-NOX protein is less than about 700 s⁻¹ at 20 °C *(e.g.,* less than about 600 s⁻¹, 500 s⁻¹, 100 s⁻¹, 20 s⁻¹, or 1.8 s⁻¹ at 20 °C).

In some embodiments, the viscosity of the H-NOX protein solution, including a polymeric H-NOX protein solution, is between 1 and 4 centipoise (cP). In some embodiments, the colloid oncotic pressure of the H-NOX protein solution is between 20 and 50 mm Hg.

### Measurement of O2 and/or NO binding

One skilled in the art can readily determine the oxygen and nitric oxide binding characteristics of any H-NOX protein including a polymeric H-NOX protein such as a trimeric H-NOX protein by methods known in the art and by the non-limiting exemplary methods described below.

### Kinetic KD: Ratio of k_{off} to kₒₙ

The kinetic K_{D} value is determined for wild-type and mutant H-NOX proteins, including polymeric H-NOS proteins, essentially as described by Boon, E.M. et al. (2005). Nature Chemical Biology 1:53-59, particularly with respect to the measurement of O₂ association rates, O₂ dissociation rates, dissociation constants for O₂ binding, autoxidation rates, and NO dissociation rates.

### kₒₙ (O₂ Association Rate)

O₂ association to the heme is measured using flash photolysis at 20 °C. It is not possible to flash off the Fe^{II}-O₂ complex as a result of the very fast geminate recombination kinetics; thus, the Fe^{II}-CO complex is subjected to flash photolysis with laser light at 560 nm (Hewlett-Packard, Palo Alto, CA), producing the 5-coordinate Fe^{II} intermediate, to which the binding of molecular O₂ is followed at various wavelengths. Protein samples are made by anaerobic reduction with 10 mM dithionite, followed by desalting on a PD-10 column (Millipore, Inc., Billerica, MA). The samples are then diluted to 20 µM heme in 50 mM TEA, 50 mM NaCl, pH 7.5 buffer in a controlled-atmosphere quartz cuvette, with a size of 100 µL to 1 mL and a path-length of 1-cm. CO gas is flowed over the headspace of this cuvette for 10 minutes to form the Fe^{II}-CO complex, the formation of which is verified by UV-visible spectroscopy (Soret maximum 423 nm). This sample is then either used to measure CO-rebinding kinetics after flash photolysis while still under 1 atmosphere of CO gas, or it is opened and stirred in air for 30 minutes to fully oxygenate the buffer before flash photolysis to watch O₂-rebinding events. O₂ association to the heme is monitored at multiple wavelengths versus time. These traces are fit with a single exponential using Igor Pro software (Wavemetrics, Inc., Oswego, OR; latest 2005 version). This rate is independent of observation wavelength but dependent on O₂ concentration. UV-visible spectroscopy is used throughout to confirm all the complexes and intermediates (Cary 3K, Varian, Inc. Palo Alto, CA). Transient absorption data are collected using instruments described in Dmochowski, I. J. et al. (August 31, 2000). J Inorg Biochem. 81(3):221-228, which is hereby referred to in its entirety, particularly with respect to instrumentation. The instrument has a response time of 20 ns, and the data are digitized at 200 megasamples s⁻¹.

### k_{off} (O₂ dissociation rate)

To measure the k_{off}, Fe^{II}-O₂ complexes of protein (5 µM heme), are diluted in anaerobic 50 mM TEA, 50 mM NaCl, pH 7.5 buffer, and are rapidly mixed with an equal volume of the same buffer (anaerobic) containing various concentrations of dithionite and/or saturating CO gas. Data are acquired on a HI-TECH Scientific SF-61 stopped-flow spectrophotometer equipped with a Neslab RTE-100 constant-temperature bath set to 20 °C (TGK Scientific LTD., Bradford On Avon, United Kingdom). The dissociation of O₂ from the heme is monitored as an increase in the absorbance at 437 nm, a maximum in the Fe^{II} - Fe^{II}-O₂ difference spectrum, or 425 nm, a maximum in the Fe^{II} - Fe^{II}-CO difference spectrum. The final traces are fit to a single exponential using the software that is part of the instrument. Each experiment is done a minimum of six times, and the resulting rates are averaged. The dissociation rates measured are independent of dithionite concentration and independent of saturating CO as a trap for the reduced species, both with and without 10 mM dithionite present.

### Kinetic K_{D}

The kinetic K_{D} is determined by calculating the ratio of k_{off} to kₒₙ using the measurements of k_{off} and kₒₙ described above.

### Calculated K_{D}

To measure the calculated K_{D}, the values for the k_{off} and kinetic K_{D} that are obtained as described above are graphed. A linear relationship between k_{off} and kinetic K_{D} is defined by the equation (y=mx+b). k_{off} values were then interpolated along the line to derive the calculated K_{D} using Excel: MAC 2004 (Microsoft, Redmond, WA). In the absence of a measured kₒₙ, this interpolation provides a way to relate k_{off} to K_{D}.

### Rate of Autoxidation

To measure the rate of autoxidation, the protein samples are anaerobically reduced, then diluted to 5 µM heme in aerobic 50 mM TEA, 50 mM NaCl, pH 7.5 buffer. These samples are then incubated in a Cary 3E spectrophotometer equipped with a Neslab RTE-100 constant-temperature bath set to 37 °C and scanned periodically (Cary 3E, Varian, Inc., Palo Alto, CA). The rate of autoxidation is determined from the difference between the maximum and minimum in the Fe^{III} - Fe^{II} difference spectrum plotted versus time and fit with a single exponential using Excel: MAC 2004 (Microsoft, Redmond, WA).

### Rate of reaction with NO

NO reactivity is measured using purified proteins (H-NOX, polymeric H-NOX, *Homo sapiens* hemoglobin (Hs Hb) *etc.)* prepared at 2 µM in buffer A and NO prepared at 200 µM in Buffer A (Buffer A: 50 mM Hepes, pH 7.5, 50 mM NaCl). Data are acquired on a HI-TECH Scientific SF-61 stopped-flow spectrophotometer equipped with a Neslab RTE-100 constant-temperature bath set to 20 °C (TGK Scientific LTD., Bradford On Avon, United Kingdom). The protein is rapidly mixed with NO in a 1:1 ratio with an integration time of 0.00125 sec. The wavelengths of maximum change are fit to a single exponential using the software that is part of the spectrometer, essentially measuring the rate-limiting step of oxidation by NO. The end products of the reaction are ferric-NO for the HNOX proteins and ferric-aquo for Hs Hb.

### p50 measurements

If desired, the p50 value for mutant or wild-type H-NOX proteins can be measured as described by Guarnone, R. et al. (September/October 1995). Haematologica 80(5):426-430, which is hereby referred to in its entirety, particularly with respect to the measurement of p50 values. The p50 value is determined using a HemOx analyzer. The measurement chamber starts at 0% oxygen and slowly is raised, incrementally, towards 100% oxygen. An oxygen probe in the chamber measures the oxygen saturation %. A second probe (UV-Vis light) measures two wavelengths of absorption, tuned to the alpha and beta peaks of the hemoprotein's (*e.g*., a protein such as H-NOX complexed with heme) UV-Vis spectra. These absorption peaks increase linearly as hemoprotein binds oxygen. The percent change from unbound to 100% bound is then plotted against the % oxygen values to generate a curve. The p50 is the point on the curve where 50% of the hemoprotein is bound to oxygen.

Specifically, the Hemox-Analyzer (TCS Scientific Corporation, New Hope, PA) determines the oxyhemoprotein dissociation curve (ODC) by exposing 50 µL of blood or hemoprotein to an increasing partial pressure of oxygen and deoxygenating it with nitrogen gas. A Clark oxygen electrode detects the change in oxygen tension, which is recorded on the x-axis of an x-y recorder. The resulting increase in oxyhemoprotein fraction is simultaneously monitored by dual-wavelength spectrophotometry at 560 nm and 576 nm and displayed on the y-axis. Blood samples are taken from the antemedial vein, anticoagulated with heparin, and kept at 4°C on wet ice until the assay. Fifty µL of whole blood are diluted in 5 µL of Hemox-solution, a manufacturer-provided buffer that keeps the pH of the solution at a value of 7.4±0.01. The sample-buffer is drawn into a cuvette that is part of the Hemox-Analyzer and the temperature of the mixture is equilibrated and brought to 37°C; the sample is then oxygenated to 100% with air. After adjustment of the pO₂ value the sample is deoxygenated with nitrogen; during the deoxygenation process the curve is recorded on graph paper. The P50 value is extrapolated on the x-axis as the point at which O₂ saturation is 50% using the software that is part of the Hemox-Analyzer. The time required for a complete recording is approximately 30 minutes.

### H-NOX Nucleic Acids

The invention also features nucleic acids encoding any of the mutant H-NOX proteins, polymeric H-NOX, or recombinant monomer H-NOX protein subunits as described herein.

In particular embodiments, the nucleic acid includes a segment of or the entire nucleic acid sequence of any of nucleic acids encoding an H-NOX protein or an H-NOX domain. In some embodiments, the nucleic acid includes at least about 50, 100, 150, 200, 300, 400, 500, 600, 700, 800, or more contiguous nucleotides from a H-NOX nucleic acid and contains one or more mutations (*e.g*., 1, 2, 3, 4, 5, 6, 7, 8, 9, or 10 mutations) compared to the H-NOX nucleic acid from which it was derived. In various embodiments, a mutant H-NOX nucleic acid contains less than about 20, 15, 12, 10, 9, 8, 7, 6, 5, 4, 3, or 2 mutations compared to the H-NOX nucleic acid from which it was derived. The invention also features degenerate variants of any nucleic acid encoding a mutant H-NOX protein.

In some embodiments, the nucleic acid includes nucleic acids encoding two or more H-NOX domains. In some embodiments, the nucleic acids including two or more H-NOX domains are linked such that a polymeric H-NOX protein is expressed from the nucleic acid. In further embodiments, the nucleic acid includes nucleic acids encoding one or more polymerization domains. In some embodiments, the nucleic acids including the two or more H-NOX domains and the one or more polymerization domains are linked such that a polymeric H-NOX protein is expressed from the nucleic acid.

In some embodiments, the nucleic acid includes a segment or the entire nucleic acid sequence of any nucleic acid encoding a polymerization domain. In some embodiments the nucleic acid comprises a nucleic acid encoding an H-NOX domain and a polymerization domain. In some embodiments, the nucleic acid encoding an H-NOX domain and the nucleic acid encoding a polymerization domain a linked such that the produced polypeptide is a fusion protein comprising an H-NOX domain and a polymerization domain.

In some embodiments, the nucleic acid comprises nucleic acid encoding one or more His₆ tags. In some embodiments the nucleic acid further comprised nucleic acids encoding linker sequences positioned between nucleic acids encoding the H-NOX domain, the polymerization domain and/or a His₆ tag.

In some embodiments, the invention provides a nucleic acid encoding an H-NOX domain and a foldon domain. In some embodiments, the H-NOX domain is a *T. thermoanaerobacter* H-NOX domain. In some embodiments, the H-NOX domain is a wild-type *T. thermoanaerobacter* H-NOX domain. In some embodiments, the H-NOX domain is a *T. thermoanaerobacter* L144F H-NOX domain.

In some embodiments, the invention provides nucleic acids encoding the following 5' to 3' : a L144F *T. tengcongensis* H-NOX domain, a Gly-Ser-Gly amino acid linker sequence, and a foldon domain. In some embodiments, the invention provides nucleic acids encoding the following 5' to 3': a wild-type *T. tengcongensis* H-NOX domain, a Gly-Ser-Gly amino acid linker sequence, and a foldon domain.

In some embodiments, the invention provides nucleic acids encoding the following 5' to 3': a L144F *T. tengcongensis* H-NOX domain, a Gly-Ser-Gly amino acid linker sequence, a foldon domain, an Arg-Gly-Ser linker sequence, and a His₆ tag. In some embodiments, the invention provides nucleic acids encoding the following 5' to 3': a wild-type *T. tengcongensis* H-NOX domain, a Gly-Ser-Gly amino acid linker sequence, a foldon domain, an Arg-Gly-Ser linker sequence, and a His₆ tag.

In some embodiments, the nucleic acid comprises the nucleic acid sequence set forth in SEQ ID NO:1, SEQ ID NO:5, or SEQ ID NO:7.

The invention also includes a cell or population of cells containing at least one nucleic acid encoding a mutant H-NOX protein described herein. Exemplary cells include insect, plant, yeast, bacterial, and mammalian cells. These cells are useful for the production of mutant H-NOX proteins using standard methods, such as those described herein.

Disclosed herein is furthermore a cell comprising a nucleic acid encoding an H-NOX domain and a foldon domain. In some embodiments, the H-NOX domain is a *T. thermoanaerobacter* H-NOX domain. In some embodiments, the H-NOX domain is a wild-type *T. thermoanaerobacter* H-NOX domain. In some embodiments, the H-NOX domain is a *T. thermoanaerobacter* L144F H-NOX domain. Disclosed herein is furthermore a cell comprising a nucleic acid comprising the nucleic acid sequence set forth in SEQ ID NO:1, SEQ ID NO:5, or SEQ ID NO:7.

### Formulations of H-NOX Proteins

Any wild-type or mutant H-NOX protein, including polymeric H-NOX proteins, described herein may be used for the formulation of pharmaceutical or non-pharmaceutical compositions. In some embodiments of the disclosure, the formulations comprise a monomeric H-NOX protein comprising an H-NOX domain and a polymerization domain such that the monomeric H-NOX proteins associate in vitro or in vivo to produce a polymeric H-NOX protein. As discussed further below, these formulations are useful in a variety of therapeutic and industrial applications.

In some embodiments of the disclosure, the pharmaceutical composition includes one or more wild-type or mutant H-NOX proteins described herein including polymeric H-NOX proteins and a pharmaceutically acceptable carrier or excipient. Examples of pharmaceutically acceptable carriers or excipients include, but are not limited to, any of the standard pharmaceutical carriers or excipients such as phosphate buffered saline solutions, water, emulsions such as oil/water emulsion, and various types of wetting agents. Exemplary diluents for aerosol or parenteral administration are phosphate buffered saline or normal (0.9%) saline. Compositions comprising such carriers are formulated by well-known conventional methods (see, for example, Remington's Pharmaceutical Sciences, 18th edition, A. Gennaro, ed., Mack Publishing Co., Easton, PA, 1990; and Remington, The Science and Practice of Pharmacy 20th Ed. Mack Publishing, 2000, which are each hereby referred to in their entireties, particularly with respect to formulations). In some embodiments, the formulations are sterile. In some embodiments, the formulations are essentially free of endotoxin.

While any suitable carrier known to those of ordinary skill in the art may be employed in the pharmaceutical compositions of this invention, the type of carrier will vary depending on the mode of administration. Compositions can be formulated for any appropriate manner of administration, including, for example, intravenous, intra-arterial, intravesicular, inhalation, intraperitoneal, intrapulmonary, intramuscular, subcutaneous, intra-tracheal, transmucosal, intraocular, intrathecal, intracranial, administration to CSF or transdermal administration. In some embodiments, delivery may be directly to a site of vascular occlusion or directly to hypoxic tissue. For parenteral administration, such as subcutaneous injection, the carrier may include, *e.g.*, water, saline, alcohol, a fat, a wax, or a buffer. For oral administration, any of the above carriers or a solid carrier, such as mannitol, lactose, starch, magnesium stearate, sodium saccharine, talcum, cellulose, glucose, sucrose, or magnesium carbonate, may be employed. Biodegradable microspheres (*e.g.*, polylactate polyglycolate) may also be used as carriers.

In some embodiments, the pharmaceutical or non-pharmaceutical compositions include a buffer *(e.g.,* neutral buffered saline, phosphate buffered saline, *etc*)*,* a carbohydrate *(e.g.,* glucose, mannose, sucrose, dextran, *etc.),* an antioxidant, a chelating agent *(e.g.,* EDTA, glutathione, *etc.),* a preservative, another compound useful for binding and/or transporting oxygen, an inactive ingredient *(e.g.,* a stabilizer, filler, *etc.),* or combinations of two or more of the foregoing. In some embodiments, the composition is formulated as a lyophilizate. H-NOX proteins may also be encapsulated within liposomes or nanoparticles using well known technology. Other exemplary formulations that can be used for H-NOX proteins are described by, *e.g.,* U.S. Pat. Nos. 6,974,795, and 6,432,918, which are each hereby referred to in their entireties, particularly with respect to formulations of proteins.

The compositions described herein may be administered as part of a sustained release formulation (*e.g*., a formulation such as a capsule or sponge that produces a slow release of compound following administration). Such formulations may generally be prepared using well known technology and administered by, for example, oral, rectal or subcutaneous implantation, or by implantation at the desired target site. Sustained-release formulations may contain an H-NOX protein dispersed in a carrier matrix and/or contained within a reservoir surrounded by a rate controlling membrane. Carriers for use within such formulations are biocompatible, and may also be biodegradable. In some embodiments, the formulation provides a relatively constant level of H-NOX protein release. The amount of H-NOX protein contained within a sustained release formulation depends upon the site of implantation, the rate and expected duration of release, and the nature of the condition to be treated or prevented.

In some embodiments of the disclosure, the pharmaceutical composition contains an effective amount of a wild-type or mutant H-NOX protein. In some embodiments of the disclosure, the pharmaceutical composition contains an effective amount of a polymeric H-NOX protein comprising two or more wild-type or mutant H-NOX domains. In some embodiments of the disclosure, the pharmaceutical composition contains an effective amount of a recombinant monomeric H-NOX protein comprising a wild-type or mutant H-NOX domain and a polymerization domain as described herein. In some embodiments, the formulation comprises a trimeric H-NOX protein comprising three monomers, each monomer comprising a *T. tengcongensis* L144F H-NOX domain and a foldon domain. In some embodiments, the formulation comprises a trimeric H-NOX protein comprising three monomers, each monomer comprising a *T. tengcongensis* L144F H-NOX domain and a foldon domain.

An exemplary dose of hemoglobin as a blood substitute is from about 10 mg to about 5 grams or more of extracellular hemoglobin per kilogram of patient body weight. Thus, in some embodiments, an effective amount of an H-NOX protein for administration to a human is between a few grams to over about 350 grams. Other exemplary doses of an H-NOX protein include about any of 4.4., 5, 10, or 13 G/DL (where G/DL is the concentration of the H-NOX protein solution prior to infusion into the circulation) at an appropriate infusion rate, such as about 0.5 ml/min (see, for example, Winslow, R. Chapter 12 *In* Blood Substitutes). It will be appreciated that the unit content of active ingredients contained in an individual dose of each dosage form need not in itself constitute an effective amount since the necessary effective amount could be reached by the combined effect of a plurality of administrations. The selection of the amount of an H-NOX protein to include in a pharmaceutical composition depends upon the dosage form utilized, the condition being treated, and the particular purpose to be achieved according to the determination of the ordinarily skilled artisan in the field.

Exemplary compositions include genetically engineered, recombinant H-NOX proteins, which may be isolated or purified, comprising one or more mutations that collectively impart altered O₂ or NO ligand-binding relative to the corresponding wild-type H-NOX protein, and operative as a physiologically compatible mammalian blood gas carrier. For example, mutant H-NOX proteins as described herein. In some embodiments, the H-NOX protein is a polymeric H-NOX protein. In some embodiments, the H-NOX protein is a recombinant monomeric H-NOX protein comprising a wild-type or mutant H-NOX domain and a polymerization domain as described herein. In some embodiments, the composition comprises a trimeric H-NOX protein comprising three monomers, each monomer comprising a *T. tengcongensis* L144F H-NOX domain and a foldon domain. In some embodiments, the composition comprises a trimeric H-NOX protein comprising three monomers, each monomer comprising a *T. tengcongensis* L144F H-NOX domain and a foldon domain.

To reduce or prevent an immune response in human subjects who are administered a pharmaceutical composition, human H-NOX proteins or domains (either wild-type human proteins or human proteins into which one or more mutations have been introduced) or other non-antigenic H-NOX proteins or domains (*e.g*., mammalian H-NOX proteins) can be used. To reduce or eliminate the immunogenicity of H-NOX proteins derived from sources other than humans, amino acids in an H-NOX protein or H-NOX domain can be mutated to the corresponding amino acids in a human H-NOX. For example, one or more amino acids on the surface of the tertiary structure of a non-human H-NOX protein can be mutated to the corresponding amino acid in a human H-NOX protein.

In some embodiments, formulations of H-NOX comprise both PEGylated and non-PEGylated H-NOX. In some embodiments, the ratio of PEGylated to non-PEGylated H-NOX in the formulation is any of about 99:1, 95:5, 90:10, 85:15, 80:20, 75:25, 70:30, 65:35, 60:40; 55:45, 50:50, 45:55, 40:60, 35:65, 30:70, 25:75, 20:80, 15:85; 10:90; 5:95; 1:99 or any ratio therebetween. In some embodiments, formulations of H-NOX comprise both PEGylated and non-PEGylated trimeric *T. tengcongensis* L144F H-NOX. In some embodiments, the ratio of PEGylated to non-PEGylated trimeric *T. tengcongensis* L144F H-NOX in the formulation is any of about 99:1, 95:5, 90:10, 85:15, 80:20, 75:25, 70:30, 65:35, 60:40; 55:45, 50:50, 45:55, 40:60, 35:65, 30:70, 25:75, 20:80, 15:85; 10:90; 5:95; 1:99 or any ratio therebetween. The invention inter alia provides a pharmaceutical composition comprising a mixture of:
(i) an H-NOX protein covalently bound to polyethylene glycol (a PEGylated H-NOX protein), and
(ii) an H-NOX protein not covalently bound to polyethylene glycol (a non-PEGylated H-NOX protein),
wherein both the PEGylated H-NOX protein and the non-PEGylated H-NOX protein are each a trimeric H-NOX protein comprising three H-NOX monomers, wherein each H-NOX monomer comprises a *T. tengcongensis* H-NOX domain and a trimerization domain, and wherein the H-NOX domain in each H-NOX monomer comprises a L144F substitution.

### Therapeutic Applications of H-NOX Proteins

Any of the wild-type or mutant H-NOX proteins, including polymeric H-NOX proteins, or pharmaceutical compositions described herein may be used in therapeutic applications. Particular H-NOX proteins, including polymeric H-NOX proteins, can be selected for such applications based on the desired O₂ association rate, O₂ dissociation rate, dissociation constant for O₂ binding, NO stability, NO reactivity, autoxidation rate, plasma retention time, or any combination of two or more of the foregoing for the particular indication being treated.

Because the distribution in the vasculature of extracellular H-NOX proteins is not limited by the size of the red blood cells, polymeric H-NOX proteins according to the present invention can be used to deliver O₂ to areas that red blood cells cannot penetrate. These areas can include any tissue areas that are located downstream of obstructions to red blood cell flow, such as areas downstream of one or more thrombi, arterial occlusions, peripheral vascular occlusions, angioplasty balloons, surgical instruments, tissues that are suffering from oxygen starvation or are hypoxic, and the like. Additionally, all types of tissue ischemia can be treated using H-NOX proteins. Such tissue ischemias include, for example, perioperative ischemia, stroke (*e.g.* ischemic stroke and hemorrhagic stroke), emerging stroke, transient ischemic attacks, myocardial stunning and hibernation, acute or unstable angina, emerging angina, and myocardial infarction (*e.g.*, ST-segment elevation myocardial infarction). In other embodiments, H-NOX is used to treat hypoxic brain injury including but not limited to hypoxic brain injury, vessel occlusion, and brain hemorrhage. In other embodiments, H-NOX is used to treat cerebral vassospasm or for aneurysm repair. Other exemplary cardiovascular indications that can be treated using H-NOX proteins include cardioplegia and sickle cell anemia. Exemplary target indications include conditions of functional hemoglobin deficiency, such as where a blood substitute or O₂ carrier is indicated, including blood loss, hypoxia, *etc.*

Disclosed are furthermore methods to reduce hypoxia in the brain of in an individual. Disclosed are furthermore methods to treat stroke in an individual by administering H-NOX to the individual. Disclosed is furthermore a method to deliver oxygen to an individual following a stroke by administering H-NOX to the individual. In some embodiments, the H-NOX comprises H-NOX covalently bound to polyethylene glycol (PEGylated) and H-NOX that is not covalently bound to polyethylene glycol (non-PEGylated). In some embodiments, the ratio of PEGylated H-NOX to non-PEGylated H-NOX administered to the individual is any of about 99:1, 95:5, 90:10, 85:15, 80:20, 75:25, 70:30, 65:35, 60:40; 55:45, 50:50, 45:55, 40:60, 35:65, 30:70, 25:75, 20:80, 15:85; 10:90; 5:95; 1:99 or any ratio therebetween. In some embodiments, the PEGylated and non-PEGylated H-NOX is in a composition. In some embodiments, the H-NOX comprises PEGylated *T. tengcongensis* L144F trimeric H-NOX and non-PEGylated *T. tengcongensis* L144F trimeric H-NOX wherein the ratio of PEGylated to non-PEGylated H-NOX is any of about 99:1, 95:5, 90:10, 85:15, 80:20, 75:25, 70:30, 65:35, 60:40; 55:45, 50:50, 45:55, 40:60, 35:65, 30:70, 25:75, 20:80, 15:85; 10:90; 5:95; 1:99 or any ratio therebetween.

Disclosed are furthermore methods to transient ischemic attack in an individual by administering H-NOX to the individual. Disclosed is furthermore a method to deliver oxygen to an individual following a transient ischemic attack by administering H-NOX to the individual. In some embodiments, the H-NOX comprises H-NOX covalently bound to polyethylene glycol (PEGylated) and H-NOX that is not covalently bound to polyethylene glycol (non-PEGylated). In some embodiments, the ratio of PEGylated H-NOX to non-PEGylated H-NOX administered to the individual is any of about 99:1, 95:5, 90:10, 85:15, 80:20, 75:25, 70:30, 65:35, 60:40; 55:45, 50:50, 45:55, 40:60, 35:65, 30:70, 25:75, 20:80, 15:85; 10:90; 5:95; 1:99 or any ratio therebetween. In some embodiments, the PEGylated and non-PEGylated H-NOX is in a composition.

Disclosed are furthermore methods to treat hypoxic brain injury in an individual by administering H-NOX to the individual. Disclosed is furthermore a method to deliver oxygen to an individual following hypoxic brain injury by administering H-NOX to the individual. In some embodiments, the H-NOX comprises H-NOX covalently bound to polyethylene glycol (PEGylated) and H-NOX that is not covalently bound to polyethylene glycol (non-PEGylated). In some embodiments, the ratio of PEGylated H-NOX to non-PEGylated H-NOX administered to the individual is any of about 99:1, 95:5, 90:10, 85:15, 80:20, 75:25, 70:30, 65:35, 60:40; 55:45, 50:50, 45:55, 40:60, 35:65, 30:70, 25:75, 20:80, 15:85; 10:90; 5:95; 1:99 or any ratio therebetween. In some embodiments, the PEGylated and non-PEGylated H-NOX is in a composition.

In some embodiments, the PEGylated H-NOX and the non-PEGylated H-NOX are delivered simultaneously or sequentially to treat stroke, transient ischemic attack or hypoxic brain injury in an individual. In some embodiments, the PEGylated H-NOX is administered before the non-PEGylated H-NOX. In some embodiments, the PEGylated H-NOX is delivered after the non-PEGylated H-NOX. In some embodiments, the PEGylated H-NOX is delivered any of about 5 minutes, 10 minutes, 15 minutes, 30 minutes, 45 minutes, about 1 hour, about 2 hours, about 4 hours, about 6 hours, about 8 hours, about 10 hours, about 12 hours, about 16 hours, about 24 hours, about 2 days, about 3 days, about 4 days, about 5 days, about 6 days, about 7 days, about two weeks, about 3 weeks, about 4 weeks or more that about 1 month after administration of the non-PEGylated H-NOX.

In some embodiments, the PEGylated H-NOX and/or the non-PEGylate H-NOX is administered to the individual multiple times. In some embodiments, the PEGylated H-NOX and/or the non-PEGylate H-NOX is administered any of two times, three times, four times, five times, six times, seven times, ten times or more than ten times. In some embodiments, the H-NOX is administered multiple times until hypoxic areas of the brain following stroke, transient ischemic attack or ischemic brain injury have essentially been removed. In some embodiments, non-PEGylated H-NOX is administered to an individual suffering from stroke, transient ischemic attack or hypoxic brain injury followed by multiple administrations of PEGylated H-NOX. In some embodiments, PEGylated H-NOX is administered one or more of one hour, one day, two days, three days, four days, five days, six days, seven days, eight days, nine days or ten days after administration of non-PEGylated H-NOX.

In some embodiments, the therapeutically effective amount of an H-NOX protein is administered to the individual in conjunction with another therapy. In some embodiments, the therapeutically effective amount of PEGylated H-NOX and/or non-PEGylated H-NOX is administered to the individual in conjunction with another therapy. In some embodiments, the H-NOX protein is administered in combination with mechanical or chemical recanalization of an occluded vessel. Examples of mechanical recanalization include but are not limited to angioplasty such as balloon angioplasty. Examples of chemical recanalization include but are not limited to tissue plasminogen activator (tPA). In some embodiments, the H-NOX protein is administered in combination with anti-coagulents such as heparin or warfarin (Coumadin). In some embodiments, the H-NOX is administered in combination with a neuroprotectant. In some embodiments, the H-NOX is administered before, at the same time, or after treatment with the other therapy.

Disclosed are furthermore methods to hypoxia in the brain of an individual comprising administering a bolus of an H-NOX protein to the individual followed by infusion of H-NOX to the individual. Disclosed are furthermore methods to treat stroke in an individual comprising administering a bolus of an H-NOX protein to the individual followed by infusion of H-NOX to the individual. Disclosed are furthermore methods to treat stroke in an individual comprising administering a bolus of an H-NOX protein to the individual by subcutaneous injection followed by infusion of H-NOX to the individual. Disclosed are furthermore methods to treat stroke in an individual comprising administering a bolus of an H-NOX protein to the individual by subcutaneous injection followed by infusion of H-NOX to the individual, wherein the H-NOX includes PEGylated H-NOX and/or non-PEGylated H-NOX. For example, the bolus of H-NOX may be administered in the field followed by an infusion of H-NOX in the clinic. In some embodiments, non-PEGylated H-NOX is delivered as a bolus followed by infusion of PEGylated H-NOX. In some embodiments, the H-NOX protein is administered to the individual by infusion over more than about any of 1 hour, 2 hours, 3 hours, 4 hours, 5 hours, 6 hours, 7 hours, 8 hours, 9 hours, 10 hours, 11 hours, 12 hours, 16 hours, 18 hours, 1 day, 2 days, 3 days, 4 days, 5 days, 6 days, or 7 days. In some embodiments, the H-NOX protein is administered to the individual as a bolus followed by infusion over more than about any of 1 hour, 2 hours, 3 hours, 4 hours, 5 hours, 6 hours, 7 hours, 8 hours, 9 hours, 10 hours, 11 hours, 12 hours, 16 hours, 18 hours, 1 day, 2 days, 3 days, 4 days, 5 days, 6 days, or 7 days. In some embodiments, the H-NOX protein is administered by infusion immediately after or more than about any of 1 hour, 2 hours, 3 hours, 4 hours, 5 hours, 6 hours, 7 hours, 8 hours, 9 hours, 10 hours, 11 hours, 12 hours, 16 hours, 18 hours, 1 day, 2 days, 3 days, 4 days, 5 days, 6 days, or 7 days following administration of the H-NOX protein by bolus.

In some embodiments, the H-NOX is delivered to the individual by bolus systemically followed by infusion systemically. In some embodiments, the H-NOX is delivered to the individual by bolus to the site of occlusion in the brain followed by infusion systemically. In some embodiments, the H-NOX is delivered to the individual by bolus to the site of occlusion in the brain followed by infusion to the site of occlusion in the brain or to the hypoxic penumbra associated with the occlusion in the brain. In some embodiments, the H-NOX is delivered to the individual by bolus systemically followed by infusion to the site of occlusion in the brain or to the hypoxic penumbra associated with the site of occlusion in the brain. In some embodiments, the H-NOX is delivered by bolus and/or by infusion directly into the brain. In some embodiments, the H-NOX is delivered by bolus and/or by infusion to the cerebral spinal fluid (CSF). In some embodiments, the H-NOX is delivered intramuscularly or subcutaneously.

Disclosed are furthermore methods for delivering O₂ to the penumbra following a stroke in an individual comprising administering a therapeutically effective amount of H-NOX to the individual as a bolus and/or by infusion. Delivery of O₂ to a hypoxic penumbra associated with a stroke reduces the level of infarct in the penumbra.

Disclosed are furthermore methods for treating a hypoxic brain injury in an individual comprising administering a therapeutically effective amount of an H-NOX protein to the individual as a bolus and/or by infusion. Disclosed are furthermore methods for treating hypoxia associated with a hypoxic brain injury in an individual comprising administering a therapeutically effective amount of an H-NOX protein to the individual as a bolus and/or by infusion. Disclosed are furthermore methods for delivering O₂ to hypoxic tissue associated with a hypoxic brain injury in an individual comprising administering a therapeutically effective amount of an H-NOX protein to the individual as a bolus and/or by infusion. In some embodiments, the hypoxic brain injury is a focal cerebral ischemia; for example, as a result of a thrombosis or embolism. In other aspects, the hypoxic brain injury is a global cerebral ischemia; for example, as a result of cardiac arrest. In some embodiments, the hypoxic brain injury is a result of a congenital disorder; for example, sickle cell anemia or improper artery formation. In some embodiments, the hypoxic brain injury is a result of plaque formation or compression of blood vessels.

Disclosed are furthermore methods for treating a transient ischemic attack in an individual, the method comprising administering a therapeutically effective amount of an H-NOX protein to the individual as a bolus and/or by infusion. Disclosed are furthermore methods for hypoxia associated with a transient ischemic attack in an individual, the method comprising administering a therapeutically effective amount of an H-NOX protein to the individual as a bolus and/or by infusion. Disclosed are furthermore Disclosed are furthermore methods for delivering O₂ to hypoxic tissue associated with a transient ischemic attack in an individual, the method comprising administering a therapeutically effective amount of an H-NOX protein to the individual as a bolus and/or by infusion. In many cases, a transient ischemic attack is followed by a stroke. Treatment of a transient ischemic attack with H-NOX may reduce the likelihood of a stroke in an individual.

In some embodiments, the individual to be treated with H-NOX displays sustained hypoperfusion of the brain as a result of the stroke, hypoxic brain injury or transient ischemic attack. Hypoperfusion of the brain may result in a hypoxic penumbra which may in turn result in infarct of the tissue associated with the penumbra. Delivery of O₂ to sites hypoperfusion may reduce the harmful consequences that result from hypoperfusion.

Disclosed are furthermore methods for reducing inflammation at the hypoxic penumbra associated with a vascular occlusion in the brain of an individual (*e.g*., following a stroke, a transient ischemic attack, a hypoxic brain injury, *etc.),* the method comprising administering a therapeutically effective amount of an H-NOX protein to the individual. In some embodiments, delivery of H-NOX to the site of occlusion or the hypoxic penumbra associated with a site of occlusion reduces the activation of infiltrating inflammatory cells, including but not limited to including but not limited to astrocytes, macrophages, T cells, neutrophils, monocytes, eosinophils, basophils and/or microglia at the site of occlusion or the penumbra associated with a site of occlusion.

In some embodiments, delivery of H-NOX to the site of occlusion or the hypoxic penumbra associated with a site of occlusion reduces sensorimotor deficits associated with stroke, hypoxic brain injury or transient ischemic attack. In some embodiments, delivery of H-NOX to the site of occlusion or the hypoxic penumbra associated with a stroke, hypoxic brain injury or transient ischemic attack reduces consequences of hypoxic conditions in the brain including, but are not limited to, aphasia, dysarthria (motor speech disorder resulting from neurological injury), apraxia (altered voluntary movements), memory, altered gait or altered coordination.

Disclosed are furthermore methods for reducing the risk of stroke in an individual, the method comprising administering a therapeutically effective amount of an H-NOX protein as a bolus and/or by infusion to the individual at risk for having a stroke. Disclosed are furthermore methods for reducing the adverse consequences of stroke in an individual, the method comprising administering a therapeutically effective amount of an H-NOX protein as a bolus and/or by infusion to the individual at risk for having a stroke. Disclosed are furthermore methods for delivering oxygen to an individual at risk of having a stroke, the method comprising administering a therapeutically effective amount of an H-NOX protein to the individual as a bolus and/or by infusion. In some embodiments, the individual at risk of having a stroke has had a transient ischemic attack. In some embodiments, an individual at risk of stroke displays one or more of the following conditions, face drooping; arm weakness; speech difficulties; sudden numbness or weakness of face, arm, or leg, especially on one side of the body; sudden confusion; sudden trouble understanding speech; sudden trouble seeing in one or both eyes; sudden trouble; sudden trouble walking, dizziness, loss of balance or coordination; and sudden severe headache with no known cause. Adverse consequences of a stroke include, but are not limited to, sensorimotor deficits, cognitive deficits, aphasia, dysarthria, apraxia, memory, altered gait or altered coordination.

In some embodiments of the methods of treatment described above, the H-NOX protein of the disclosed methods is a polymeric H-NOX protein *(e.g.* a trimeric H-NOX protein). In some embodiments, the polymeric H-NOX protein comprises one or more H-NOX domains comprising a mutation at a position corresponding to L144 of *T. tengcongensis* H-NOX. In some embodiments, the polymeric H-NOX protein comprises one or more H-NOX domains comprising a mutation corresponding to a L144F mutation of *T. tengcongensis* H-NOX. In some embodiments, the H-NOX domain is a human H-NOX domain. In some embodiments, the polymeric H-NOX protein comprises a *T. tengcongensis* L144F H-NOX domain.

Disclosed are furthermore methods for treating hypoxic tissue associated with injury to an organ or limb in an individual comprising administering a therapeutically effective amount of an H-NOX protein to the individual. The hypoxia may be associated directly with the injury to the organ or may be associated indirectly with the injury. In some embodiments, the hypoxia may be a result of the primary treatment of the injury. Disclosed are furthermore methods for treating hypoxic tissue associated with injury to an organ in an individual comprising administering a therapeutically effective amount of an H-NOX protein to the individual. Disclosed are furthermore methods for treating a hypoxic penumbra associated with injury to an organ in an individual comprising administering a therapeutically effective amount of an H-NOX protein to the individual. Disclosed are furthermore methods for reducing the extent of a hypoxic penumbra associated with injury to an organ in an individual comprising administering a therapeutically effective amount of an H-NOX protein to the individual. Reducing the extent of a hypoxic penumbra may be a spatial reduction and/or a temporal reduction. In some embodiments reducing the level of hypoxia in the tissue reduces the loss of cellular function and/or cell death which can lead to organ and/or body dysfunction. In some embodiments, the organ or tissue is part of the musculoskeletal system, the respiratory system, the digestive system, the urinary system, the circulatory system, the reproductive system, the nervous system, or is skin or an endocrine gland. In some embodiments, the organ is a brain, a heart, a liver, a kidney, a lung, a spleen, intestines, or a pancreas. In some embodiments, the tissue is muscle. Disclosed are furthermore methods treating hypoxia associated with injury to a limb in an individual comprising administering a therapeutically effective amount of an H-NOX protein to the individual.

Disclosed are furthermore methods for delivering O₂ to hypoxic tissue associated with injury to an organ in an individual comprising administering a therapeutically effective amount of an H-NOX protein to the individual. Disclosed are furthermore methods for delivering O₂ to hypoxic tissue associated with injury to an organ in an individual comprising administering a therapeutically effective amount of an H-NOX protein to the individual. Disclosed are furthermore methods for delivering O₂ to hypoxic tissue associated with injury to an organ to reduce the extent of a hypoxic penumbra associated with injury to an organ in an individual comprising administering a therapeutically effective amount of an H-NOX protein to the individual. Reducing the extent of a hypoxic penumbra may be a spatial reduction and/or a temporal reduction. In some embodiments, the organ is a brain, a heart, a liver, a kidney, a lung. Disclosed are furthermore methods treating hypoxia associated with injury to a limb in an individual comprising administering a therapeutically effective amount of an H-NOX protein to the individual.

Disclosed are furthermore methods for treating hypoxia associated with injury to a limb in an individual comprising administering a therapeutically effective amount of an H-NOX protein to the individual. Disclosed are furthermore methods for treating hypoxia associated with injury to a limb in an individual comprising administering a therapeutically effective amount of an H-NOX protein to the individual. Disclosed are furthermore methods for treating a hypoxia penumbra associated with injury to a limb in an individual comprising administering a therapeutically effective amount of an H-NOX protein to the individual. Disclosed are furthermore methods for reducing the extent of a hypoxic penumbra associated with injury to a limb in an individual comprising administering a therapeutically effective amount of an H-NOX protein to the individual. Reducing the extent of a hypoxic penumbra may be a spatial reduction and/or a temporal reduction. In some embodiments, the limb is an arm, a hand, a finger, a leg, a knee, a foot, or a toe. Disclosed are furthermore methods treating hypoxia associated with injury to a limb in an individual comprising administering a therapeutically effective amount of an H-NOX protein to the individual.

Disclosed are furthermore methods for delivering O₂ to hypoxic tissue associated with injury to a limb in an individual comprising administering a therapeutically effective amount of an H-NOX protein to the individual. Disclosed are furthermore methods for delivering O₂ to hypoxic tissue associated with injury to a limb in an individual comprising administering a therapeutically effective amount of an H-NOX protein to the individual. Disclosed are furthermore methods for delivering O₂ to hypoxic tissue associated with injury to a limb to reduce the extent of a hypoxic penumbra associated with injury to a limb in an individual comprising administering a therapeutically effective amount of an H-NOX protein to the individual. Reducing the extent of a hypoxic penumbra may be a spatial reduction and/or a temporal reduction. In some embodiments, the limb is an arm, a hand, a finger, a leg, a knee, a foot, or a toe. Disclosed are furthermore methods treating hypoxia associated with injury to a limb in an individual comprising administering a therapeutically effective amount of an H-NOX protein to the individual.

In some embodiments, the injury to the organ is a result of a vascular occlusion. For example, the injury may be due to occlusion of a coronary vessel, occlusion of a vessel of the brain *(e.g.,* a cerebral vessel or a cerebellar vessel, *etc.),* a vessel feeding an organ such as the liver *(e.g.,* a portal vessel), the kidneys *(e.g.,* a renal vessel), or the lungs *(e.g.,* a pulmonary vessel). In some embodiments, the organ injury is a result of ischemia. In some embodiments, the organ injury is a result of trauma to the organ. In some embodiments, the injury to the organ results in hypoperfusion of tissue of the injured organ or associated with the injured organ. In some embodiments, the injury to the organ results in sustained hypoperfusion of tissue of the injured organ or associated with the injured organ. In some embodiments, the injury to the organ results in hypoperfusion of tissue of the injured organ or associated with the injured organ, either spatially of functionally. In some embodiments, the injury to the organ results in the development of a hypoxic penumbra at or near the injured organ.

In some embodiments, the injury to the limb is a result of a vascular occlusion. For example, the injury may be a peripheral vascular occlusion. In some embodiments, the vascular occlusion results of ischemia. In some embodiments, the limb injury is a result of trauma to the limb. In some embodiments, the injury to the limb results in hypoperfusion of tissue of the limb or associated with the limb. In some embodiments, the injury to the limb results in sustained hypoperfusion of tissue of the limb or associated with the limb. In some embodiments, the injury to the limb results in hypoperfusion of tissue of the limb or associated with the limb, either spatially of functionally. In some embodiments, the injury to the limb results in the development of a hypoxic penumbra at or near the injured limb.

Disclosed are furthermore methods for reducing consequences of hypoxia associated with injury to an organ in an individual comprising administering a therapeutically effective amount of an H-NOX protein to the individual. Examples of consequences of hypoxia associated with injury to an organ include, but are not limited to sensorimotor deficits, locomotion deficits, cognition deficits, speech deficits, emotional issues (anxiety, apathy, psychosis, depression) *etc.*

In some embodiments, administration of the H-NOX protein reduces inflammation of the injured organ and/or reduces inflammation of a hypoxic penumbra associated with the injured organ. In some embodiments, administration of the H-NOX protein reduces activation of macrophages in the injured organ and/or hypoxic penumbra associated with the injured organ. In some embodiments, administration of the H-NOX protein reduces apoptosis of cells of the injured organ and/or hypoxic penumbra associated with the injured organ.

In some embodiments, the hypoxic penumbra is a result of hypoperfusion connected with the organ injury. Hypoperfusion refers to an inadequate supply of blood to an organ or extremity like the brain or hand. If hypoperfusion persists, it can cause oxygen deprivation, and can deprive the tissue of needed nutrients and waste disposal. Often hypoperfusion leads to tissue death. Symptoms of hypoperfusion vary depending on the location. Hypoperfusion of an organ like the heart can create functional problems, while hypoperfusion in the brain may lead to cognitive deficits, slurred speech, and/or confusion.

In some embodiments, an H-NOX protein is administered to an individual at risk of developing hypoxia associated with an injury or trauma to an organ or limb. The H-NOX protein may be administered to an individual undergoing a medical intervention in which developing hypoxia is a risk. For example, the H-NOX protein may be administered to an individual before, during or after a surgery to reduce the likelihood of surgery-related hypoxia.

Disclosed is furthermore a a method of delivering O₂ to an individual *(e.g.,* a mammal, such as a primate *(e.g.,* a human, a monkey, a gorilla, an ape, a lemur, *etc.),* a bovine, an equine, a porcine, a canine, or a feline) by administering to an individual in need thereof a wild-type or mutant H-NOX protein, including a polymeric H-NOX protein in an amount sufficient to deliver O₂ to the individual. Disclosed are furthermore methods of carrying or delivering blood gas to an individual such as a mammal, comprising the step of delivering *(e.g.,* transfusing, *etc.)* to the blood of the individual *(e.g.,* a mammal) one or more of H-NOX compositions. Methods for delivering O₂ carriers to blood or tissues (*e.g*., mammalian blood or tissues) are known in the art. In various embodiments, the H-NOX protein is an apoprotein that is capable of binding heme or is a holoprotein with heme bound. The H-NOX protein may or may not have heme bound prior to the administration of the H-NOX protein to the individual. In some embodiments, O₂ is bound to the H-NOX protein before it is delivered to the individual. In other embodiments, O₂ is not bound to the H-NOX protein prior to the administration of the protein to the individual, and the H-NOX protein transports O₂ from one location in the individual to another location in the individual.

Wild-type and mutant H-NOX proteins, including polymeric H-NOX proteins, with a relatively low K_{D} for O₂ (such as less than about 80 nM or less than about 50 nM) are expected to be particularly useful to treat tissues with low oxygen tension (such as tumors, some wounds, or other areas where the oxygen tension is very low, such as a p50 below 1 mm Hg). The high affinity of such H-NOX proteins for O₂ may increase the length of time the O₂ remains bound to the H-NOX protein, thereby reducing the amount of O₂ that is released before the H-NOX protein reaches the tissue to be treated.

In some embodiments for the direct delivery of an H-NOX protein with bound O₂ to a particular site in the body (such as a site of organ injury), the k_{off} for O₂ is more important than the K_{D} value because O₂ is already bound to the protein (making the kₒₙ less important) and oxygen needs to be released at or near a particular site in the body (at a rate influenced by the k_{off}). In some embodiments, the k_{off} may also be important when H-NOX proteins are in the presence of red cells in the circulation, where they facilitate diffusion of O₂ from red cells, and perhaps prolonging the ability of diluted red cells to transport O₂ to further points in the vasculature.

In some embodiments for the delivery of an H-NOX protein that circulates in the bloodstream of an individual, the H-NOX protein binds O₂ in the lungs and releases O₂ at one or more other sites in the body. For some of these applications, the K_{D} value is more important than the k_{off} since O₂ binding is at or near equilibrium. In some embodiments for extreme hemodilution, the K_{D} more important than the k_{off} when the H-NOX protein is the primary O₂ carrier because the H-NOX protein will bind and release O₂ continually as it travels through the circulation. Since hemoglobin has a p50 of 14 mm Hg, red cells (which act like capacitors) have a p50 of -30 mm Hg, and HBOCs have been developed with ranges between 5 mm Hg and 90 mm Hg, the optimal K_{D} range for H-NOX proteins may therefore be between ~2 mm Hg to -100 mm Hg for some applications.

Polymeric H-NOX proteins can also be used for imaging. In particular, light imaging *(e.g.,* optical coherence tomography; *see,* for example, Villard, J. W. (2002). Circulation 105:1843-1849, which is referred to in its entirety particularly with respect to optical coherence tomography) is obfuscated by erythrocytes. Perfusion with an H-NOX solution allows for clearer images of the circulation and vessel walls because the H-NOX protein is much smaller than erythrocytes.

H-NOX proteins, including polymeric H-NOX proteins, and pharmaceutical compositions of the invention can be administered to an individual by any conventional means such as by oral, topical, intraocular, intrathecal, intrapulmonary, intra-tracheal, or aerosol administration; by transdermal or mucus membrane adsorption; or by injection (*e.g*., subcutaneous, intravenous, intra-arterial, intravesicular, or intramuscular injection). H-NOX proteins may also be included in large volume parenteral solutions for use as blood substitutes. In exemplary embodiments, the H-NOX protein is administered to the blood *(e.g.,* administration to a blood vessel such as a vein, artery, or capillary), a wound, a tumor, a hypoxic tissue, or a hypoxic organ of the individual.

In some embodiments, the H-NOX protein is delivered as a bolus. In some embodiments, the H-NOX protein is delivered by infusion. In some embodiments, the H-NOX is PEGylated. In some embodiments, the H-NOX is not PEGylated. In some embodiments, the H-NOX comprises PEGylated and non-PEGylated H-NOX. In some embodiments, the H-NOX protein is administered to the individual by infusion over more than about any of 1 hour, 2 hours, 3 hours, 4 hours, 5 hours, 6 hours, 7 hours, 8 hours, 9 hours, 10 hours, 11 hours, 12 hours, 16 hours, 18 hours, 1 day, 2 days, 3 days, 4 days, 5 days, 6 days, or 7 days. In some embodiments the H-NOX protein is administered to the individual by infusion to the injured organ or by systemic infusion. In some embodiments, the H-NOX protein is administered to the individual by a bolus followed by administration to the individual by infusion. In some embodiments, the H-NOX protein is administered to the individual as a bolus followed by infusion over more than about any of 1 hour, 2 hours, 3 hours, 4 hours, 5 hours, 6 hours, 7 hours, 8 hours, 9 hours, 10 hours, 11 hours, 12 hours, 16 hours, 18 hours, 1 day, 2 days, 3 days, 4 days, 5 days, 6 days, or 7 days. In some embodiments, the H-NOX protein is administered by infusion more than about any of 1 hour, 2 hours, 3 hours, 4 hours, 5 hours, 6 hours, 7 hours, 8 hours, 9 hours, 10 hours, 11 hours, 12 hours, 16 hours, 18 hours, 1 day, 2 days, 3 days, 4 days, 5 days, 6 days, or 7 days following administration of the H-NOX protein by bolus. In some embodiments, the H-NOX is delivered to the individual by bolus systemically followed by infusion systemically. In some embodiments, the H-NOX is delivered to the individual by bolus to the injured organ followed by infusion systemically. In some embodiments, the H-NOX is delivered to the individual by bolus to the injured organ followed by infusion to the injured organ or to the hypoxic penumbra associated with the injured organ. In some embodiments, the H-NOX is delivered to the individual by bolus systemically followed by infusion to the injured organ or to the hypoxic penumbra associated with the injured organ.

In some embodiments, the H-NOX is administered at a dose of about 10 mg/kg to about 300 mg/kg. In some embodiments, the H-NOX is administered at a dose ranging from any of about 10 mg/kg to about 50 mg/kg, about 50 mg/kg to about 100 mg/kg, about 100 mg/kg to about 150 mg/kg, about 150 mg/kg to about 200 mg/kg, about 200 mg/kg to about 250 mg/kg, or about 250 mg/kg to about 300 mg/kg. In some embodiments, the H-NOX is delivered in a volume of about 10 ml to about 1 L. In some embodiments, the H-NOX is delivered in a volume of about 10 ml to about 25 ml, about 25 ml to about 50 ml, about 50 ml to about 100 ml, about 100 ml to about 200 ml, about 200 ml to about 300 ml, about 300 ml to about 400 ml, about 400 ml to about 500 ml, about 500 ml to about 600 ml, about 600 ml to about 700 ml, about 700 ml to about 800 ml, about 800 ml to about 900 ml, or about 900 ml to about 1 L. In some embodiments, the H-NOX is delivered as a bolus over a period of less than any of 1 minute, 2 minutes, 3 minutes, 4 minutes, 5 minutes, 10 minutes, 15 minutes, 20 minutes, 25 minutes, or about 30 minutes. In some embodiments, the H-NOX is delivered by infusion over a period of about 30 minutes to about 7 days. In some embodiment, the H-NOX is delivered by infusion over more than about any of 30 minutes, 1 hour, 2 hours, 3 hours, 4 hours, 5 hours, 6 hours, 7 hours, 8 hours, 9 hours, 10 hours, 11 hours, 12 hours, 16 hours, 18 hours, 1 day, 2 days, 3 days, 4 days, 5 days, 6 days, or 7 days. In some embodiments, the H-NOX protein is administered by infusion more than about any of 30 minutes to 1 hour, about 1 hour to about 2 hours, about 2 hours to about 3 hours, about 3 hours to about 4 hours, about 4 hours to about 5 hours, about 5 hours to about 6 hours, about 6 hours to about 7 hours, about 7 hours to about 8 hours, about 8 hours to about 9 hours, about 9 hours to about 10 hours, about 10 hours to about 11 hours, about 11 hours to about 12 hours, about 12 hours to about 16 hours, about 16 hours to about 18 hours, about 18 hours to about 1 day, about 1 day to about 2 days, about 2 days to about 3 days, about 3 days to about 4 days, about 4 days to about 5 days, about 5 days to about 6 days, or about 6 days to about 7 days. In some embodiments, the H-NOX is PEGylated. In some embodiments, the H-NOX is not PEGylated. In some embodiments, the H-NOX comprises PEGylated and non-PEGylated H-NOX.

In some embodiments, an effective amount of an H-NOX protein for administration to a human is between a few grams to over about 350 grams. Other exemplary doses of an H-NOX protein include about any of 4.4., 5, 10, or 13 G/DL (where G/DL is the concentration of the H-NOX protein solution prior to infusion into the circulation) at an appropriate infusion rate, such as about 0.5 ml/min (see, for example, Winslow, R. Chapter 12 In Blood Substitutes). It will be appreciated that the unit content of active ingredients contained in an individual dose of each dosage form need not in itself constitute an effective amount since the necessary effective amount could be reached by the combined effect of a plurality of administrations. The selection of the amount of an H-NOX protein to include in a pharmaceutical composition depends upon the dosage form utilized, the condition being treated, and the particular purpose to be achieved according to the determination of the ordinarily skilled artisan in the field. In some embodiments, the H-NOX is PEGylated. In some embodiments, the H-NOX is not PEGylated. In some embodiments, the H-NOX comprises PEGylated and non-PEGylated H-NOX.

In some embodiments, a sustained continuous release formulation of the composition is used. Administration of an H-NOX protein can occur, *e.g.,* for a period of seconds to hours depending on the purpose of the administration. For example, as a blood delivery vehicle, an exemplary time course of administration is as rapid as possible. Other exemplary time courses include about any of 10, 20, 30, 40, 60, 90, or 120 minutes. Exemplary infusion rates for H-NOX solutions as blood replacements are from about 30 mL/hour to about 13,260 mL/hour, such as about 100 mL/hour to about 3,000 mL/hour. An exemplary total dose of H-NOX protein is about 900 mg/kg administered over 20 minutes at 13,260 mL/hour. An exemplary total dose of H-NOX protein for a swine is about 18.9 grams.

Exemplary dosing frequencies include, but are not limited to, at least 1, 2, 3, 4, 5, 6, or 7 times (*i.e.,* daily) a week. In some embodiments, an H-NOX protein is administered at least 2, 3, 4, or 6 times a day. The H-NOX protein can be administered, *e.g.*, over a period of a few days or weeks. In some embodiments, the H-NOX protein is administrated for a longer period, such as a few months or years. The dosing frequency of the composition may be adjusted over the course of the treatment based on the judgment of the administering physician.

In some embodiments of the invention, the H-NOX protein according to the invention (*e.g.* a polymeric H-NOX protein) is used in combination or in conjunction with another therapy. For example, for the treatment of stroke or hypoxia related to stroke. In some embodiments, the H-NOX is administered to the individual any of at least about 1, 2, 3, 4, 5, 6, 12 or 24 hours before administration of the other therapy. In some embodiments, the H-NOX is administered to the individual at the same time as administration of the other therapy. In some embodiments, the H-NOX is administered to the individual any of at least about 1, 2, 3, 4, 5, 6, 12 or 24 hours after administration of the other therapy. In some embodiments, PEGylated H-NOX is administered in combination with another therapy. In some embodiments, non-PEGylated H-NOX is administered in combination with another therapy. In some embodiments, PEGylated and non-PEGylated H-NOX is administered to an individual in combination with another therapy.

As noted above, the selection of dosage amounts for H-NOX proteins depends upon the dosage form utilized, the frequency and number of administrations, the condition being treated, and the particular purpose to be achieved according to the determination of the ordinarily skilled artisan in the field. In some embodiments, an effective amount of an H-NOX protein for administration to human is between a few grams to over 350 grams.

In some embodiments, two or more different H-NOX proteins are administered simultaneously, sequentially, or concurrently. In some embodiments, another compound or therapy useful for the delivery of O₂ is administered simultaneously, sequentially, or concurrently with the administration of one or more H-NOX proteins.

Other exemplary therapeutic applications for which H-NOX proteins can be used are described by, *e.g.,* U.S. Pat. Nos. 6,974,795, and 6,432,918, which are each hereby referred to in their entireties, particularly with respect to therapeutic applications for O₂ carriers.

### Kits with H-NOX proteins

Also provided are articles of manufacture and kits that include any of the H-NOX proteins described herein including polymeric H-NOX proteins, and suitable packaging. Disclosed is a kit with (i) a H-NOX protein (such as a wild-type or mutant H-NOX protein described herein or formulations thereof as described herein) and (ii) instructions for using the kit to deliver O₂ to an individual.

In some embodiments of the disclosure, kits are provided for use in treatment of stroke, transient ischemic attack or hypoxic brain injury in an individual. In some embodiments of the disclosure, kits are provided for use in reducing the consequences of stroke, transient ischemic attack or hypoxic brain injury in an individual (*e.g*., reducing sensorimotor deficits associated with stroke). In some embodiments, of the disclosure, kits are provided for use in the delivery of O₂ to hypoxic tissue (*e.g*., a hypoxic penumbra) following stroke, transient ischemic attack or hypoxic brain injury in an individual. In some embodiments of the disclosure, kits are provided for use in the treatment of hypoxia associated with injury to an organ in an individual. In some embodiments of the disclosure, kits are provided for use in the delivery of O₂ to hypoxic tissue (*e.g*., a hypoxic penumbra) following injury to an organ in an individual. In some embodimentsof the disclosure, kits are provided for use in reducing the consequences of hypoxia associated with injury to an organ in an individual.

In some embodiments of the disclosure, the kits comprise both PEGylated and non-PEGylated H-NOX. In some embodiments, the PEGylated H-NOX and non-PEGylated H-NOX in the kit are in a composition. In some embodiments of the disclosure, the ratio of PEGylated to non-PEGylated H-NOX in the composition is any of about 99:1, 95:5, 90:10, 85:15, 80:20, 75:25, 70:30, 65:35, 60:40; 55:45, 50:50, 45:55, 40:60, 35:65, 30:70, 25:75, 20:80, 15:85; 10:90; 5:95; 1:99 or any ratio therebetween.

In some embodiments of the disclosure, the kit comprises a polymeric H-NOX protein *(e.g.,* a PEGylated polymeric H-NOX protein and/or a non-PEGylated H-NOX protein). In some embodiments of the disclosure, the kit comprises an effective amount of a polymeric H-NOX protein comprising two or more wild-type or mutant H-NOX domains. In some embodiments of the disclosure, the kit comprises an effective amount of a recombinant monomeric H-NOX protein comprising a wild-type or mutant H-NOX domain and a polymerization domain as described herein. In some embodiments of the disclosure, the kit comprises a trimeric H-NOX protein comprising three monomers, each monomer comprising a mutation corresponding to a *T. tengcongensis* L144F H-NOX mutation and a trimerization domain. In some embodiments, the trimeric H-NOX protein comprises human H-NOX domains. In some embodiments, the trimeric H-NOX protein comprises canine H-NOX domains. In some embodiments of the disclosure, the kit comprises a trimeric H-NOX protein comprising three monomers, each monomer comprising a *T. tengcongensis* L144F H-NOX domain and a foldon domain. In some embodiments of the disclosure, the kit comprises a trimeric H-NOX protein comprising three monomers, each monomer comprising a *T. tengcongensis* L144F H-NOX domain and a foldon domain.

Suitable packaging for compositions described herein are known in the art, and include, for example, vials (*e.g*., sealed vials), vessels, ampules, bottles, jars, flexible packaging *(e.g.,* sealed Mylar or plastic bags), and the like. These articles of manufacture may further be sterilized and/or sealed. Also provided are unit dosage forms comprising the compositions described herein. These unit dosage forms can be stored in a suitable packaging in single or multiple unit dosages and may also be further sterilized and sealed. Instructions supplied in the kits disclosed herein are typically written instructions on a label or package insert *(e.g.,* a paper sheet included in the kit), but machine-readable instructions (*e.g*., instructions carried on a magnetic or optical storage disk) are also acceptable. The instructions relating to the use of H-NOX proteins generally include information as to dosage, dosing schedule, and route of administration for the intended treatment or industrial use. The kit may further comprise a description of selecting an individual suitable or treatment. In some embodiments of the disclosure, the kit comprises instructions for treatment of stroke, transient ischemic attack and/or hypoxic brain injury.

The containers may be unit doses, bulk packages (*e.g*., multi-dose packages) or subunit doses. For example, kits may also be provided that contain sufficient dosages of H-NOX proteins disclosed herein to provide effective treatment for an individual for an extended period, such as about any of a week, 2 weeks, 3 weeks, 4 weeks, 6 weeks, 8 weeks, 3 months, 4 months, 5 months, 6 months, 7 months, 8 months, 9 months, or more. Kits may also include multiple unit doses of H-NOX proteins and instructions for use and packaged in quantities sufficient for storage and use in pharmacies, for example, hospital pharmacies and compounding pharmacies. In some embodiments, the kit includes a dry *(e.g.,* lyophilized) composition that can be reconstituted, resuspended, or rehydrated to form generally a stable aqueous suspension of H-NOX protein.

Disclosed is furthermore an article of manufacture containing a PEGylated and/or a non-PEGylated H-NOX. Disclosed is furthermore an article of manufacture containing a mixture of PEGylated and non-PEGylated H-NOX. In some embodiments of the disclosure, the article of manufacture is a vessel, a vial, a jar, an ampule, a capsule, a syringe, or a bag. In some embodiments, the ratio of PEGylated H-NOX to non-PEGylated H-NOX is any of about 99:1, about 95:5, about 90:10, about 80:20, about 75:25, about 70:30, about 60:40, about 50:50, about 40:60, about 30:70, about 25:75, about 20:80, about 10:90, about 5:95, or about 1:99. In some embodiments, the PEGylated H-NOX is sequestered from the non-PEGlyated H-NOX by a barrier. In some embodiments the barrier may be removed prior to use to allow the PEGylated H-NOX and the non-PEGylated H-NOX to mix. Disclosed is furthermore a syringe containing a mixture of PEGylated and non-PEGylated H-NOX.

### Exemplary Methods for Production of H-NOX Proteins

Disclosed are furthermore methods for the production of compositions comprising PEGylated and non-PEGylated H-NOX proteins, the method comprising mixing PEGylated H-NOX with non-PEGylated H-NOX. In some embodiments, the ratio of PEGylated to non-PEGylated H-NOX in the composition is any of about 99:1, 95:5, 90:10, 85:15, 80:20, 75:25, 70:30, 65:35, 60:40; 55:45, 50:50, 45:55, 40:60, 35:65, 30:70, 25:75, 20:80, 15:85; 10:90; 5:95; 1:99 or any ratio therebetween.

Disclosed are furthermore methods for the production of any of the H-NOX proteins (*e.g*., polymeric H-NOX proteins) described herein. In some embodiments, the method involves culturing a cell that has a nucleic acid encoding a polymeric H-NOX protein under conditions suitable for production of the polymeric H-NOX protein. In various embodiments, the polymeric H-NOX is also purified (such as purification of the H-NOX protein from the cells or the culture medium). In some embodiments, the method involves culturing a cell that has a nucleic acid encoding a monomer H-NOX protein comprising an H-NOX domain and a polymerization domain. The monomers then associate *in vivo* or *in vitro* to form a polymeric H-NOX protein. A polymeric H-NOX protein comprising heterologous H-NOX domains may be generated by co-introducing two or more nucleic acids encoding monomeric H-NOX proteins with the desired H-NOX domains and where in the two or more monomeric H-NOX proteins comprise the same polymerization domain.

In some embodiments, a polymeric H-NOX protein comprising heterologous H-NOX domains is prepared by separately preparing polymeric H-NOX proteins comprising homologous monomeric H-NOX subunits comprising the desired H-NOX domains and a common polymerization domain. The different homologous H-NOX proteins are mixed at a desired ratio of heterologous H-NOX subunits, the homologous polymeric H-NOX proteins are dissociated *(e.g.* by heat, denaturant, high salt, *etc.),* then allowed to associate to form heterologous polymeric H-NOX proteins. The mixture of heterologous polymeric H-NOX proteins may be further purified by selecting for the presence of the desired subunits at the desired ratio. For example, each different H-NOX monomer may have a distinct tag to assist in purifying heterologous polymeric H-NOX proteins and identifying and quantifying the heterologous subunits.

As noted above, the sequences of several wild-type H-NOX proteins and nucleic acids are known and can be used to generate mutant H-NOX domains and nucleic acids of the present invention. Techniques for the mutation, expression, and purification of recombinant H-NOX proteins have been described by, *e.g.,* Boon, E.M. et al. (2005). Nature Chemical Biology 1:53-59 and Karow, D. S. et al. (August 10, 2004). Biochemistry 43(31):10203-10211, which is hereby referred to in its entirety, particularly with respect to the mutation, expression, and purification of recombinant H-NOX proteins. These techniques or other standard techniques can be used to generate any mutant H-NOX protein.

In particular, mutant H-NOX proteins described herein can be generated a number of methods that are known in the art. Mutation can occur at either the amino acid level by chemical modification of an amino acid or at the codon level by alteration of the nucleotide sequence that codes for a given amino acid. Substitution of an amino acid at any given position in a protein can be achieved by altering the codon that codes for that amino acid. This can be accomplished by site-directed mutagenesis using, for example: (i) the Amersham technique (Amersham mutagenesis kit, Amersham, Inc., Cleveland, Ohio) based on the methods of Taylor, J.W. et al. (December 20, 1985). Nucleic Acids Res. 13(24):8749-8764; Taylor, J.W. et al. (December 20, 1985). Nucleic Acids Res. 13(24):8765-8785; Nakamaye, K. L. et al. (December 22, 1986). Nucleic Acids Res. 14(24):9679-9698; and Dente et al. (1985). in DNA Cloning, Glover, Ed., IRL Press, pages 791-802, (ii) the Promega kit (Promega Inc., Madison, Wis.), or (iii) the Biorad kit (Biorad Inc., Richmond, Calif.), based on the methods of Kunkel, T. A. (January 1985). Proc. Natl. Acad. Sci. USA 82(2):488-492; Kunkel, T. A. (1987). Methods Enzymol. 154:367-382; Kunkel, U.S. Pat. No. 4,873,192, which are each hereby referred to in their entireties, particularly with respect to the mutagenesis of proteins. Mutagenesis can also be accomplished by other commercially available or non-commercial means, such as those that utilize site-directed mutagenesis with mutant oligonucleotides.

Site-directed mutagenesis can also be accomplished using PCR-based mutagenesis such as that described in Zhengbin et al. (1992). pages 205-207 in PCR Methods and Applications, Cold Spring Harbor Laboratory Press, New York; Jones, D. H. et al. (February 1990). Biotechniques 8(2):178-183; Jones, D. H. et al. (January 1991). Biotechniques 10(1):62-66, which are each hereby referred to in their entireties, particularly with respect to the mutagenesis of proteins. Site-directed mutagenesis can also be accomplished using cassette mutagenesis with techniques that are known to those of skill in the art.

A mutant H-NOX nucleic acid and/or polymerization domain can be incorporated into a vector, such as an expression vector, using standard techniques. For example, restriction enzymes can be used to cleave the mutant H-NOX nucleic acid and the vector. Then, the compatible ends of the cleaved mutant H-NOX nucleic acid and the cleaved vector can be ligated. The resulting vector can be inserted into a cell *(e.g.,* an insect cell, a plant cell, a yeast cell, or a bacterial cell) using standard techniques (*e.g*., electroporation) for expression of the encoded H-NOX protein.

In particular, heterologous proteins have been expressed in a number of biological expression systems, such as insect cells, plant cells, yeast cells, and bacterial cells. Thus, any suitable biological protein expression system can be utilized to produce large quantities of recombinant H-NOX protein. In some embodiments, the H-NOX protein *(e.g.,* a mutant or wild-type H-NOX protein) is an isolated protein.

If desired, H-NOX proteins can be purified using standard techniques. In some embodiments, the protein is at least about 60%, by weight, free from other components that are present when the protein is produced. In various embodiments, the protein is at least about 75%, 90%, or 99%, by weight, pure. A purified protein can be obtained, for example, by purification (*e.g*., extraction) from a natural source, a recombinant expression system, or a reaction mixture for chemical synthesis. Exemplary methods of purification include immunoprecipitation, column chromatography such as immunoaffinity chromatography, magnetic bead immunoaffinity purification, and panning with a plate-bound antibody, as well as other techniques known to the skilled artisan. Purity can be assayed by any appropriate method, *e.g.,* by column chromatography, polyacrylamide gel electrophoresis, or HPLC analysis. In some embodiments, the purified protein is incorporated into a pharmaceutical composition of the invention or used in a method disclosed herein. The pharmaceutical composition of the invention may have additives, carriers, or other components in addition to the purified protein.

In some embodiments, the polymeric H-NOX protein comprises one or more His₆ tags. An H-NOX protein comprising at least one His₆ tag may be purified using chromatography; for example, using Ni²⁺-affinity chromatography. Following purification, the His₆ tag may be removed; for example, by using an exopeptidase. Disclosed is furthermore a purified polymeric H-NOX protein, wherein the polymeric H-NOX protein was purified through the use of a His₆ tag. In some embodiments, the purified H-NOX protein is treated with an exopeptidase to remove the His₆ tags.

### EXAMPLES

The examples, which are intended to be purely exemplary of the invention and should therefore not be considered to limit the invention in any way, also describe and detail aspects and embodiments of the invention discussed above. The examples are not intended to represent that the experiments below are all or the only experiments performed. Unless indicated otherwise, temperature is in degrees Centigrade and pressure is at or near atmospheric.

### Example 1. Animal models of hypoxia

### Myocardial hypoxia in lambs

Neonatal sheep were anesthetized with ketamine hydrochloride (~1 mg/kg), intubated with a 4.5-mm OD endotracheal tube, and mechanically ventilated with a Healthdyne pediatric time-cycled, pressure-limited ventilator (Healthdyne, Marietta, GA). Succinylcholine chloride (2 mg/kg) was given intermittently for muscle relaxation. Ventilation with 21% oxygen was adjusted to maintain a systemic arterial PCO₂ between 35 and 45 Torr. Via a mid-sternotomy incision, polyvinyl catheters were placed to measure systemic, pulmonary, right and left atrial pressures. Admittance catheters were placed in the right and left ventricles for pressure-volume loop analysis, and a flow probe was placed on the left pulmonary artery to measure blood flow. The sternum was then closed with towel clamps. After 45 min of recovery with stable ventilation, the hemodynamic variables, systemic arterial blood gases, and pH were measured (baseline normoxia). Normocarbic hypoxia (10% O₂) was then induced by adding nitrogen to the gas mixture to produce systemic arterial blood gases with a PO2, between 25 and 35 Torr. After 15-30 min of steady-state pulmonary hypertension, all variables were measured (hypoxia).

### Permanent Middle Cerebral Artery Occlusion (MCAO) model

Permanent focal cerebral ischemia was produced by occlusion of the MCA in 8-weeks-old male Sprague Dawley rats (290-310 g) (Charles River) using the silicone-tipped intraluminal thread occlusion method, with minor modifications. Anesthesia was induced by inhalation of 3% isoflurane with spontaneous ventilation and maintained at 2-2.5% during surgery. The ventral neck region was disinfected with Chlorexhidrine and alcohol and a surgical mid-line incision was made to expose the left common, internal and external carotid arteries. The external carotid artery (ECA) was ligated, and the common carotid artery (CCA) and internal carotid artery (ICA) were closed temporarily with a micro vascular clip. A small incision was made into the ECA and a silicon-coated monofilament suture (Doccol Corporation) was inserted in the ICA through the ECA. The filament was advanced 25 mm further to occlude the origin of the MCA. The permanent occlusion was achieved by leaving the occluding thread in the ICA. Micro vascular clips were removed carefully and the wound was then closed and disinfected. For sham surgery, all the arteries were exposed for the surgical period but the filament was not inserted.

### Endothelin-1 (ET1) Middle Cerebral Artery Occlusion (MCAO) Stroke Model

Transient MCAO (tMCAO) was induced in adult male Sprague-Dawley (SD) rats (3-4 months old: 280-320 g; 6-10 months old: 500-800 g) purchased from Charles River. Rats were administered an analgesic agent (buprenorphine; 0.05 mg kg⁻¹ s.c), anaesthetized with a mixture of O₂ and 4% isoflurane then placed in a Kopf stereotaxic frame. Anesthesia was maintained for the duration of the surgery using a mixture of O₂ and 2% isoflurane delivered through a nose cone attached to the frame. The skull was exposed, and a small hole was drilled in the cranium dorsal to the right hemisphere. With a 26 gauge needle, 240 µM of the vasoactive peptide ET1 was infused adjacent to the right MCA at a rate of 1 µl/min at the following stereotaxic coordinates: 1.6 mm anterior, 5.2 mm lateral and 8.7 mm ventral to bregma. The needle was left in place 3 min after the injection then withdrawn. Body temperature was monitored using a rectal probe, pre- and post-occlusion and maintain at 37.0 ± 0.2°C with a thermoregulatory heating pad. During MCAO surgery, vital signs were continuously monitored using a capnograph and pulse oximeter. Regional cerebral blood flow (rCBF) was measured with a Laser Doppler flow probe (VP5 probe, Moor Instruments) attached to the right side of the dorsal surface of the skull before ischemia, during MCAO, and during reperfusion (FIGS. 1A and 1B). Following surgery rats were given softened food in order to minimize weight loss. The flux signal from the Laser-Doppler flowmetry (LDF) allowed a rapid and continuous recording of regional cerebral blood flow and detection of transient changes in blood flow. Animals that did not show a CBF reduction of at least 60% were excluded from the experimental group, as well as animals that died after ischemia induction.

The *hypoxic at-risk penumbra* in the ET1-induced tMCAO model was visualized histologically with the hypoxia marker pimonidazole (pimo, administered at 60 mg/kg 1-2 hours before sacrifice), which labels viable tissue under acute hypoxia but is excluded from dead tissue (FIG. 2A). Animals sacrificed between day 1 and day 7 after tMCAO were processed histologically for hypoxia volume by pimo staining and infarct volume by cresyl violet staining (FIG. 2B; n = 5 per group), for neuronal cell death by NeuN/FluoroJade (FJ) staining (FIGS. 2C and 2D; n = 5 per group), for microglia/macrophage infiltration by ED1 staining (FIG. 2E; n = 5 per group), and for kinetics of astrogliosis by GFAP staining (FIG. 2F; n = 5 per group). Coronal brain sections (15 µm thickness) were obtained over 9 coordinate levels every 1 mm from Bregma +4 to -4 and processed for immunohistochemistry. Sections were fixed with 100% methanol for 20 minutes at -20°C, then blocked and permeabilized with 5% BSA, 5% goat serum, and 0.1% Tween 20 for 1-2 h at room temperature. Sections were then incubated with anti-pimonidazole (Hypoxyprobe, 1:100), anti-NeuN (Abcam), anti-GFAP (Life Technologies), or anti-EDl (AbD Serotec) antibodies overnight at 4°C, followed by anti-rabbit or anti-rat secondary antibodies (1:1000, Jackson Immunoresearch Laboratories, West Grove, PA, USA) for 2 h at room temperature. To visualize neuronal injury, brain sections were then stained with Fuoro-Jade C (FJC, Histo-Chem Inc., AR, USA) as described (Nguemeni et al. 2015 J Neurosci Methods. 2015 Mar 15;242:72-6). The sections were mounted in SlowFade DAPI (Invitrogen) and imaged at the UCSF Laboratory for Cell Analysis Core with an HD AxioImager Zeiss microscope equipped with a CCD digital camera. Neurons were labeled with NeuN or Fluoro-Jade (FJ; neurodegeneration) and inflammatory cells were detected with ED1 (activated microglia/macrophages) or GFAP (astrocytes). The numbers of NeuN⁺, FJ⁺ and ED1⁺ cells were counted and the percent of area covered by GFAP signal was measured in 8 sections per coordinate level in 2 non-overlapping regions of the cortex located in the peri-infarct area.

Animals sacrificed between 1 hour to 1 day (n = 10 per group) after tMCAO were processed by ELISA for pimonidazole to follow evolution of hypoxic tissue into infarcted tissue. Rats were injected with the exogenous hypoxia marker pimonidazole intravenously (60 mg/kg, Hypoxyprobe, Burlington Massachusetts) 2 h before sacrifice. Brain proteins were then extracted and processed for competitive pimonidazole ELISA as previously described (Kleither et al 2006 Int. J. Radiation Oncology Biol. Phys*).* Briefly, samples were harvested and homogenized in an extraction buffer (Abcam kit # ab117996) supplemented with anti-proteases. Protein concentration was quantified in each sample using a Bradford assay. Samples were assayed for pimonidazole (Hypoxyprobe-1) amount using a competitive ELISA assay developed by Omniox. The decrease in pimonidazole signal indicates evolution of the penumbra into infarcted tissue (FIG. 2G). Quantification of the IC50 values was performed with a 5-parameter fit of the standard curve for the pimonidazole ELISA. Values were normalized relative to the protein concentration in each sample then normalized relative to the vehicle control.

### Example 2. Pharmacokinetics and Distribution of H-NOX

Trimeric H-NOX L144F and PEGylated trimeric H-NOX L144F were expressed in E. coli and purified via a 3-step chromatography and buffer exchange protocol to yield >99% pure product with endotoxin levels <0.01 EU/mg. Rats were injected intravenously with 100 mg/kg of trimeric H-NOX L144F or 50 mg/kg of PEGylated trimeric H-NOX L144F and plasma samples were collected 5 min to 96 hours post injection as indicated (FIG. 3). The plasma levels of trimeric H-NOX L144F were quantified using an ELISA assay specific for the H-NOX protein, and steady state volume of distribution (Vss) was determined (Table 2). Circulation half-life was calculated by fitting data points using a 5 parameter non-linear fit model, yielding a circulation half-life of 1 hour for trimeric H-NOX L144F and a significantly longer half-life of 20 hours for PEGylated trimeric H-NOX L144F. The restricted distribution of PEGylated trimeric H-NOX L144F into normal tissues was demonstrated by the small volume of distribution at steady-state for rats (-41 ml/kg), which is only slightly greater than that of the plasma volume in rats (31.2 mL/kg; Davis, B. & Morris, T, 1993, Pharmaceutical Res 10, 1093). No adverse effects were noted in the treated rats and trimeric H-NOX L144F did not induce significant changes in blood chemistry or hematology parameters, indicating that it is well tolerated (data not shown).

**Table 2. H-NOX Pharmacokinetics**

| | Dose (mk/kg) | Circulation t_{1/2} (hours) | Vss (mL/kg) |
|---|---|---|---|
| Trimeric H-NOX L144F | 100 | 1.03 ± 0.02 | 86 ± 51 |
| PEGylated trimeric H-NOX L144F | 50 | 18.8 ± 0.7 | 41 ± 18 |

Trimeric H-NOX L144F or PEGylated trimeric H-NOX L144F (400 mg/kg) were administered i.v. in rats 1h after ET1-induced tMCAO. Animals were sacrificed 2 hours to 24 hours post-occlusion. Brains were sectioned and stained with anti-H-NOX and anti-CD31 antibodies. CD31 is a marker for endothelial cells used to visualize the vasculature. Both proteins diffused out of leaky vasculature and penetrated deep into brain interstitial tissue (FIGS. 4A and 4B). Trimeric H-NOX L144F was detected outside the vasculature in ischemic lesions 1h after administration, demonstrating an early distribution of trimeric H-NOX L144F within the brain parenchyma (FIG. 4A). Due to its long circulation half-life, PEGylated trimeric H-NOX L144F was retained for at least 24h in the brain parenchyma in infarct and peri-infarct regions, yet remained in the vessel lumen on the non-ischemic contralateral side (FIG. 4B). Based on the infarcted brain retention time, PEGylated trimeric H-NOX L144F was selected for further testing.

### Example 3. Functional H-NOX-mediated delivery of oxygen to hypoxic tissue

### Real-time detection of oxygen in hypoxic tissues (OxyLite Probe).

Seven to eight week old Nu/Nu female mice were subcutaneously implanted with 3 × 10⁶ of H460 human lung cancer cells and monitored until the tumors reached average size of -600 mm³ (9-18 days post-implantation of tumor cells). Mice bearing 500-700 mm³ xenograft H460 tumors were anesthetized with isofluorane mixed in 20% of oxygen and the OxyLite^{™} oxygen-sensing nanofiber device (Oxford Optronix, UK) was implanted into H460 subcutaneous xenograft tumors using a micromanipulator. The OxyLite^{™} consists of the ruthenium chloride dye held in a polymer matrix of 230 µm in diameter at the tip. After equilibration for -20-30 minutes, pO₂ was measured using optical fluorescence sensors and a four-channel unit. A low starting pO₂ confirmed entry into hypoxic tissue away from neighbouring blood vessels (-0.2-1 mmHg). After probe implantation, probe was left for -30 min in order for pO₂ measurements to stabilize. Mice were injected intravenously or intratumorally with 10 mg of trimeric H-NOX L144F or PEGylated trimeric H-NOX L144F in 50-100ul of formulation buffer (50 mM succinate, 50 mM NaCl, 3.4 mM EDTA, and 10 mM reduced glutathione at pH 7) and fluorescent quenching was recorded in real time (FIG. 5). Trimeric H-NOX L144F resulted in peak oxygen delivery at about 15 min post-injection, with tissue reverting to normoxic levels until about 50 min post-injection, while PEGylated trimeric H-NOX L144F resulted in a more gradual and prolonged release of oxygen, with reversion of the hypoxic tissue to normoxic levels extending from about 200 min post-injection to at least 450 min post-injection.

### Example 4. H-NOX ameliorates ischemic lesions

The *hypoxic at-risk penumbra* was visualized histologically with the hypoxia marker pimonidazole (pimo), which labels viable tissue under acute hypoxia but is excluded from dead tissue. Pimonidazole was administered at 60 mg/kg (rats) or 100 mg/kg (lambs) 1-2h before sacrifice. Animals were sacrificed 6h post-H-NOX/vehicle administration. Organs were frozen and processed for competitive pimonidazole ELISA (FIG. 6). The level of hypoxia, as measured by pimonidazole staining, was significantly lower after trimeric H-NOX L144F administration versus vehicle in both the ET1 MCAO rodent stroke model and the myocardial hypoxia lamb model. Alternatively, samples were collected in lambs for sectioning and staining with anti-pimoniadazole antibody (Hydroxyprobe) and representative pictures are shown in lower panels.

To probe the effect of trimeric H-NOX L144F on infarct size, PEGylated trimeric H-NOX L144F (240 mg/kg) or vehicle was systemically administered 1 hour after tMCAO, with repeated administration every 24 hours for 3 days. Animas were sacrificed between day 1 and day 7, and infarct volume was quantified by histology using cresyl violet staining (FIG. 7A). In another experiment, animals were sacrificed on day 1 or day 7, and infarct and hypoxic tissue volume was quantified by histology using cresyl violet and pimonidazole staining, respectively, as previously described (FIG. 7B). The oxygen-deprived hypoxic tissue seen in vehicle-treated rats at 1d post-tMCAO (FIG. 7B, white bar) slowly died by 7d, resulting in infarct expansion by -25%. In contrast, PEGylated trimeric H-NOX L144F prevented -90% of the oxygen-deprived hypoxic tissue from progressing into infarcted tissue, indicating that PEGylated trimeric H-NOX L144F has salvaged a majority of the hypoxic tissue (FIG. 7B).

Following vessel occlusion, many neuronal cells localized in the oxygen-deprived penumbra undergo apoptosis for several hours to days after onset. Therefore, we investigated if trimeric H-NOX L144F and a 1:1 mixture of trimeric H-NOX L144F + PEGylated trimeric H-NOX L144F reduces hypoxia and cell death by delivering oxygen once it reaches tissue areas with extremely low O₂ concentration. Hypoxia levels were measured with an ELISA assay for pimonidazole, which labels viable tissue under severe hypoxia (FIG. 8A), and caspase 9 activity was measured using a bioluminescent assay (FIG. 8B). Activity of caspase-9 was measured with Caspase-Glo^{®} 9 assay system (Promega) following the manufacturer's instructions. Briefly, brain proteins were extracted with the native lysis buffer (Abcam) and 25 µL of protein extract were incubated with 25 µL of Caspase-Glo^{®} 9 Reagent in the presence or absence of the Caspase 9 inhibitor Ac-LEHD-CHO (Enzo Lifesciences). The luminescence of each sample was measured every 90 seconds at 37°C for 1 hour with the Victor 3 luminometer (Perkin Elmer). Areas under the curve of the luminescent signal were calculated for each sample and plotted. A single dose (120 mg/kg or 240 mg/kg) of a 1:1 mixture of trimeric H-NOX L144F + PEGylated trimeric H-NOX L144F administered 30 min post-tMCAO significantly decreased the level of hypoxia and caspase 9 activity by -50% compared to vehicle at 6 hours after onset in a dose-dependent manner. No difference in level of hypoxia at 6 hours after onset was seen when PEGylated trimeric H-NOX L144F alone (240 mg/kg) or vehicle was administered 1 hour post-tMCAO.

Severe oxygen depletion due to ET1-mediated vessel occlusion triggers inflammatory processes leading to microglia, macrophage, and astrocyte activation that contribute to neurovascular injury. To probe whether reduction of severe hypoxia by trimeric H-NOX L144F reduced gliosis, rats with ET1-induced tMCAO were administered PEGylated trimeric H-NOX L144F (240 mg/kg) 1 hour after occlusion and administration was repeated every 24 hours for 3 days. Rats were sacrificed at day 7 after tMCAO, and brains were sectioned and processed by immunohistochemistry for GFAP (astrocytes) and ED1 (activated microglia and macrophages). PEGylated trimeric H-NOX L144F-treated animals showed a ~50-80% significant reduction in the presence of activated microglia and macrophages at 7 days following tMCAO (FIG. 9).

As non-human primates and humans also develop hypoxic penumbra following stroke, the present rodent data provides a good model for stroke in primates and the present results are applicable to primates including humans.

### Example 5. H-NOX reduces ischemic functional deficits in aged rats

Age is a major risk factor for stroke, highlighting the importance of assessing long-term histological and functional outcomes in aged rats. When administered 1 hour post-tMCAO, aged PEGylated trimeric H-NOX L144F-treated rats showed a significant increase in the number of peri-infarct neurons 29 days post-tMCAO (FIG. 10). The brains of the rats were prepared as described above and stained with NeuN and GFAP. The number of NeuN⁺ cells were counted and the percent of area covered by GFAP signal was measured in sections over 8 coordinates per level (every 1 mm) in 2 non-overlapping regions of the cortex located in the peri-infarct area. Rats treated with PEGylated trimeric H-NOX L144F had significantly more neurons in the peri-infarct area than the vehicle-treated controls.

Consistent with reduced selective neuronal loss, PEGylated trimeric H-NOX L144F improved sensory function by -80% and motor function by -180% (Fig. 11) at 13 days post-tMCAO when administered 1 hour post-tMCAO as compared to controls in an adhesive tape removal task. When administered 2 hours post-tMCAO, aged rats treated with PEGylated trimeric H-NOX L144F showed a significant amelioration of motor functions by -80% compared to controls at 20 days after tMCAO (FIG. 12). The task consisted of applying a strip of adhesive tape (8x8 mm) to each forelimb paw and measuring the rat's performance. Four measurements were recorded: *1 and 2 (detection):* the time it takes each forelimb to react to the presence of the strip when the rat either shakes its forelimb and/or directly brings its forelimb to its mouth, and *3 and 4 (removal):* the time it takes the animal to orally remove the strip of tape from the ipsilateral (unimpaired side) or contralateral (lesion side) paws. If the rat was unsuccessful after 120 sec, the task was stopped. The time-to-contact and time-to-remove separate out sensory from motor deficits. Rats with unilateral brain damage develop a bias for both contacting and removing the adhesive from the ipsilateral paw first. To decrease inter-individual differences, animals were trained for 5 days before surgery (4 trials per day). All rats reached optimal performances at the end of the training, displaying similar performance and very short latency in time-to-contact and time-to-remove the adhesive tape between right and left paws without asymmetry between right and left sides. The adhesive removal test was performed ~2-3 times per week for one month following stroke on aged rats (8-9 month old) treated with vehicle or PEGylated and unPEGylated trimeric H-NOX L144F. The results indicate improved sensory and motor functioning as a result of trimeric H-NOX L144F treatment.

As non-human primates and humans also develop hypoxic penumbra following stroke, the present rodent data provides a good model for stroke in primates and the present results are applicable to primates including humans.

### Example 6. H-NOX safety in combination with tPA treatment

### Free radical assay

Since reperfusion can generate an overproduction of free radicals, one safety concern of oxygen carriers is their potential to increase free radical production that leads to secondary oxidative damage on protein, lipids and DNA. A 1:1 mixture of trimeric H-NOX L144F + PEGylated trimeric H-NOX L144F (400 mg/kg) was administered 1 hour post-tMCAO and brains were assayed 6 hours post-tMCAO for protein carbonyl contents (FIG. 13A) by ELISA (OxiSelect^{™}) and lipid peroxydation (FIG. 13B) with the TBARs assay (Cayman), two markers widely accepted as indicators of oxidative stress. Ischemia/reperfusion injury induced an increase in protein carbonylation in the ipsilateral side of tMCAO rats. However, trimeric H-NOX L144F administration neither increased accumulation of malondialdehyde, a marker for lipid peroxidation, nor protein carbonyls, a marker of protein oxidation.

### In vivo tPA assay

The standard procedure for middle cerebral artery occlusion (MCAO) model was performed in 7-8-weeks-old Sprague Dawley rats. Rats were anesthetized with 4% isoflurane mixed in oxygen and nitrous oxide (30:70) by facemask. A midline neck incision was made exposing the left common carotid artery. The external carotid and pterygopalatine arteries were ligated with 4-0 silk. An incision was made in the wall of the external carotid artery close to the bifurcation point of the external and internal carotid arteries. A 4-0 heat-blunted nylon suture (Ethicon) was then inserted and advanced approximately 17.5 mm from the bifurcation point into the internal carotid arteries, thereby blocking the ostium of the middle cerebral artery for 60 minutes. After the pre-determined occlusion duration, the nylon suture was removed from carotid artery to allow the reperfusion of blood flow into the MCA. tPA was injected at 10 mg/ml after reperfusion and PEGylated trimeric H-NOX L144F was injected at 100 mg/ml 30 min after tPA. Twenty four hours later, rats were perfused with heparinized saline, the brain was cut into 2mm sections and visually inspected for parenchymal hemorrhage (PH), hemorrhagic infarction (HI) and punctate or petechial hemorrhage (HPT). Once processed and digitally photographed, all sections were fixed in 4% PFA at 4 degrees. The studies were conducted randomized and blinded per STAIR and RIGOR criteria. For all experiments, rats were randomly allocated into treatment groups before the surgical/stroke procedure, with concealment of the randomization guaranteed by using an independent party. The randomization sequence was not revealed until all postmortem analyses were complete. As shown in Table 3, there was no difference between control and H-NOX-treated animals for mortality, morbidity, or hemorrhage incidence.

**Table 3**

| Arms | % mortality | % morbidity | Hemorrhages |
|---|---|---|---|
| tPA + vehicle | 0% | 10% | 10% |
| tPA + trimeric H-NOX L144F | 0% | 10% | 10% |

### In vitro tPA assay

The assay for tissue plasminogen activator (tPA, Genentech Activase) activity utilizing a chromogenic substrate was conducted in 96 well plates. Briefly, 1 µl of tPA solution (5 ng/well) and 177 µl of Reagent 1 (30 mM Tris-HCl, 30 mM imidazole and 130 mM NaCl) were pre-incubated at 39 °C. 10 µl of chromogenic substrate solution (4 mM; final concentration 0.212 mM) was added to initiate the reaction. Reactions were measured using a heated SpectraMax M2 spectrophotometer maintained at 39 °C (Molecular Devices, Sunnyvale California). Change in absorbance using and OD of 405 nm was measured (ΔA/min or ΔA/5 min). Using the assays established using the procedures described above, we used 2µl of PEGylated trimeric H-NOX L144F (final concentration: 0.01-1000X the amount of tPA in the assay well), which was pre-incubated with 1µl of tPA and 175 µl of Reagent 1 as described above. ΔA/min was measured over 5 minutes at 1 minute intervals. To control the effectiveness of the inhibitor, we used 2 µl of PAI-1 (final concentration: 0.247 µM), which was pre-incubated with 1µl of tPA and 175 µl of Reagent 1 as described above. ΔA/min was measured over 5 minutes at 1 minute intervals (not shown). As shown in FIG. 14, when using a concentration of PEGylated trimeric H-NOX L144F 100X the amount of tPA, there was no significant difference in tPA activity.

### Example 7. H-NOX in a rat permanent MCAO (pMCAO) model

Trimeric H-NOX L144F was evaluated in a proximal pMCAO model that generates a consistent infarct in cortical and subcortical structures (FIG. 16A), larger than the one observed in the ET1 model. Infarct volume was measured using 2,3,5-triphenyltetrazolium chloride (TTC) staining. For each TTC-stained section (6 sections every 2 mm), the total area of each hemisphere and the infarct area were measured using ImageJ. Infarct volume corrected for edema was calculated (FIG. 16A). However, in the pMCAO model the penumbra volume at 6 hours post-occlusion is smaller and/or disappears faster compared to the ET1 model as measured by pimonidazole labeling (FIG. 16B). Pimonidazole (60 mg/kg) was injected i.v. 2 hours before sacrifice and sections were stained with an anti-pimonidazole antibody or tissue was analyzed by ELISA as described above.

To evaluate trimeric H-NOX L144F localization, a single dose of trimeric H-NOX L144F (240 mg/kg) was administered 1 hour after pMCAO, animals were euthanized 2 hours post-pMCAO, and brains were sectioned and stained with anti-H-NOX and anti-CD31 antibodies.When administered 1 hour post-pMCAO, trimeric H-NOX L144F was localized in the peri-infarct brain parenchyma as early as 2 hours post-pMCAO (FIG. 17A). In normal brain tissue (contralateral), trimeric H-NOX L144F was restricted to vessels. The effect of trimeric H-NOX L144F on ischemic lesions was then tested. Animals were administered a single dose of trimeric H-NOX L144F (240 mg/kg) 1 hour after occlusion and brains were harvested 6 hours post-pMCAO and assayed for hypoxia by ELISA for pimonidazole as previously described and caspase 9 activity by luminometry. As shown in FIG. 17B, trimeric H-NOX L144F administration reduced hypoxia and caspase 9 activity 6 hours post-pMCAO, indicating that trimeric H-NOX L144F is a promising agent for treating stroke.

## Claims

1. A pharmaceutical composition comprising a mixture of:
(i) an H-NOX protein covalently bound to polyethylene glycol (a PEGylated H-NOX protein), and
(ii) an H-NOX protein not covalently bound to polyethylene glycol (a non-PEGylated H-NOX protein),
wherein both the PEGylated H-NOX protein and the non-PEGylated H-NOX protein are each a trimeric H-NOX protein comprising three H-NOX monomers, wherein each H-NOX monomer comprises a *T*. *tengcongensis* H-NOX domain and a trimerization domain, and
wherein the H-NOX domain in each H-NOX monomer comprises a L144F substitution.

2. The pharmaceutical composition of claim 1, wherein the *T*. *tengcongensis* H-NOX domain in each H-NOX monomer comprises the amino acid sequence SEQ ID NO: 6.

3. The pharmaceutical composition of any one of claims 1-2, wherein each H-NOX monomer comprises the *T*. *tengcongensis* H-NOX domain covalently linked to the trimerization domain.

4. The pharmaceutical composition of claim 3, wherein in each monomer the C-terminus of the *T*. *tengcongensis* H-NOX domain is covalently linked to the trimerization domain.

5. The pharmaceutical composition of any one of claims 1-4, wherein the trimerization domain in each H-NOX monomer is a foldon domain of bacteriophage T4 fibritin.

6. The pharmaceutical composition of claim 5, wherein the foldon domain of bacteriophage T4 fibritin comprises the amino acid sequence of SEQ ID NO:4.

7. The pharmaceutical composition of any one of claims 1-6, wherein the H-NOX domain is linked to the trimerization domain via an amino acid linker in each H-NOX monomer.

8. The pharmaceutical composition of claim 7, wherein the amino acid linker is three, four, five, six, seven, eight, nine, or ten amino acids in length.

9. The pharmaceutical composition of claim 1, wherein each H-NOX monomer comprises the amino acid sequence of SEQ ID NO:8.

10. The pharmaceutical composition of any one of claims 1-9, wherein the PEGylated H-NOX protein comprises three polyethylene glycol (PEG) molecules per monomer.

11. The pharmaceutical composition of claim 10, wherein each PEG molecule has a molecular weight of 5 KDa.

12. The pharmaceutical composition of any one of claims 1-11, wherein the pharmaceutical composition further comprises a pharmaceutically acceptable carrier or excipient.

13. A pharmaceutical composition as defined in any one of claims 1-12 for use in a method for treating a stroke, a transient ischemic attack, or a hypoxic tissue associated with injury to an organ or limb, in a mammal.

14. A pharmaceutical composition as defined in any one of claims 1-12 for use in a method of delivering oxygen to a hypoxic tissue associated with injury to an organ or limb in a mammal, or in a method of delivering oxygen following a stroke or a transient ischemic attack, in a mammal.

15. The pharmaceutical composition for use of claim 13 or 14, wherein the pharmaceutical composition is for treating or delivering oxygen to a hypoxic tissue associated with injury to an organ or limb, and wherein the organ or limb is a brain, a heart, a liver, a kidney, a lung, a spleen, intestines, or a pancreas.

16. The pharmaceutical composition for use of claim 13 or 14, wherein the pharmaceutical composition is for treating a stroke or delivering oxygen following a stroke, wherein the stroke is an ischemic stroke or a hemorrhagic stroke.

17. The pharmaceutical composition of any one of claims 1-12 for use in a method of treating a hypoxic brain injury in a mammal, or in a method of delivering oxygen following a hypoxic brain injury in a mammal.

18. The pharmaceutical composition for use of any one of claims 13-17, wherein the method comprises the administration of the pharmaceutical composition to the mammal in combination with an additional therapy, wherein the additional therapy is a tissue plasminogen activator, a neuroprotectant, or recanalization.

19. The pharmaceutical composition for use of any one of claims 13-18, wherein the mammal is a human.

20. The pharmaceutical composition for use of any one of claims 13-19, wherein the method comprises administering the pharmaceutical composition by oral, topical, intraocular, intrathecal, intrapulmonary, intra-tracheal, or aerosol administration; or by transdermal or mucus membrane adsorption; or by subcutaneous, intravenous, intraarterial, intravesicular, or intramuscular injection.

## Patentansprüche

1. Pharmazeutische Zusammensetzung, umfassend eine Mischung aus:
(i) ein kovalent an Polyethylenglykol gebundenes H-NOX-Protein (ein PEGyliertes H-NOX-Protein), und
(ii) ein H-NOX-Protein, das nicht kovalent an Polyethylenglykol gebunden ist (ein nicht-PEGyliertes H-NOX-Protein),
wobei sowohl das PEGylierte H-NOX-Protein als auch das nicht-PEGylierte H-NOX-Protein jeweils ein trimeres H-NOX-Protein sind, das drei H-NOX-Monomere umfasst, wobei jedes H-NOX-Monomer eine *T. tengcongensis* H-NOX-Domäne und eine Trimerisierungsdomäne umfasst, und
wobei die H-NOX-Domäne in jedem H-NOX-Monomer eine L144F-Substitution umfasst.

2. Pharmazeutische Zusammensetzung nach Anspruch 1, wobei die *T. tengcongensis* H-NOX-Domäne in jedem H-NOX-Monomer die Aminosäuresequenz SEQ ID NO: 6 umfasst.

3. Pharmazeutische Zusammensetzung nach irgendeinem der Ansprüche 1-2, wobei jedes H-NOX-Monomer die *T*. *tengcongensis* H-NOX-Domäne umfasst, die kovalent an die Trimerisierungsdomäne gebunden ist.

4. Pharmazeutische Zusammensetzung nach Anspruch 3, wobei in jedem Monomer der C-Terminus der *T. tengcongensis* H-NOX-Domäne kovalent mit der Trimerisierungsdomäne verbunden ist.

5. Pharmazeutische Zusammensetzung nach irgendeinem der Ansprüche 1-4, wobei die Trimerisierungsdomäne in jedem H-NOX-Monomer eine Foldon-Domäne ("foldon domain") von Bakteriophagen-T4-Fibritin ist.

6. Pharmazeutische Zusammensetzung nach Anspruch 5, wobei die Foldon-Domäne ("foldon domain") des Bakteriophagen T4-Fibritins die Aminosäuresequenz von SEQ ID NO:4 umfasst.

7. Pharmazeutische Zusammensetzung nach irgendeinem der Ansprüche 1-6, wobei die H-NOX-Domäne über einen Aminosäure-Linker in jedem H-NOX-Monomer mit der Trimerisierungsdomäne verbunden ist.

8. Pharmazeutische Zusammensetzung nach Anspruch 7, wobei der Aminosäurelinker drei, vier, fünf, sechs, sieben, acht, neun oder zehn Aminosäuren lang ist.

9. Pharmazeutische Zusammensetzung nach Anspruch 1, wobei jedes H-NOX-Monomer die Aminosäuresequenz von SEQ ID NO:8 umfasst.

10. Pharmazeutische Zusammensetzung nach irgendeinem der Ansprüche 1-9, wobei das PEGylierte H-NOX-Protein drei Polyethylenglykol (PEG)-Moleküle pro Monomer umfasst.

11. Pharmazeutische Zusammensetzung nach Anspruch 10, wobei jedes PEG-Molekül ein Molekulargewicht von 5 KDa aufweist.

12. Pharmazeutische Zusammensetzung nach irgendeinem der Ansprüche 1-11, wobei die pharmazeutische Zusammensetzung ferner einen pharmazeutisch akzeptablen Träger oder Hilfsstoff umfasst.

13. Pharmazeutische Zusammensetzung, wie in irgendeinem der Ansprüche 1-12 definiert, zur Verwendung in einem Verfahren zur Behandlung eines Schlaganfalls, einer transitorischen ischämischen Attacke oder eines hypoxischen Gewebes, das mit einer Verletzung eines Organs oder einer Gliedmaße verbunden ist, bei einem Säugetier.

14. Pharmazeutische Zusammensetzung, wie in irgendeinem der Ansprüche 1-12 definiert, zur Verwendung in einem Verfahren zur Abgabe von Sauerstoff an ein hypoxisches Gewebe, das mit einer Verletzung eines Organs oder einer Gliedmaße bei einem Säugetier verbunden ist, oder in einem Verfahren zur Abgabe von Sauerstoff nach einem Schlaganfall oder einer transitorischen ischämischen Attacke bei einem Säugetier.

15. Pharmazeutische Zusammensetzung zur Verwendung nach Anspruch 13 oder 14, wobei die pharmazeutische Zusammensetzung zur Behandlung oder Abgabe von Sauerstoff an ein hypoxisches Gewebe dient, das mit einer Verletzung eines Organs oder einer Gliedmaße verbunden ist, und wobei das Organ oder die Gliedmaße ein Gehirn, ein Herz, eine Leber, eine Niere, eine Lunge, eine Milz, Darm oder eine Bauchspeicheldrüse ist.

16. Pharmazeutische Zusammensetzung zur Verwendung nach Anspruch 13 oder 14, wobei die pharmazeutische Zusammensetzung zur Behandlung eines Schlaganfalls oder zur Abgabe von Sauerstoff nach einem Schlaganfall dient, wobei der Schlaganfall ein ischämischer Schlaganfall oder ein hämorrhagischer Schlaganfall ist.

17. Pharmazeutische Zusammensetzung nach irgendeinem der Ansprüche 1-12 zur Verwendung in einem Verfahren zur Behandlung einer hypoxischen Hirnverletzung bei einem Säugetier oder in einem Verfahren zur Abgabe von Sauerstoff nach einer hypoxischen Hirnverletzung bei einem Säugetier.

18. Pharmazeutische Zusammensetzung zur Verwendung nach irgendeinem der Ansprüche 13-17, wobei das Verfahren die Verabreichung der pharmazeutischen Zusammensetzung an das Säugetier in Kombination mit einer zusätzlichen Therapie umfasst, wobei die zusätzliche Therapie ein Gewebeplasminogenaktivator, ein Neuroprotektivum oder eine Rekanalisation ist.

19. Die pharmazeutische Zusammensetzung zur Verwendung nach irgendeinem der Ansprüche 13-18, wobei das Säugetier ein Mensch ist.

20. Pharmazeutische Zusammensetzung zur Verwendung nach irgendeinem der Ansprüche 13-19, wobei das Verfahren die Verabreichung der pharmazeutischen Zusammensetzung durch orale, topische, intraokulare, intrathekale, intrapulmonale, intratracheale oder Aerosol-Verabreichung; oder durch transdermale oder Schleimhaut-Adsorption; oder durch subkutane, intravenöse, intraarterielle, intravesikuläre oder intramuskuläre Injektion umfasst.

## Revendications

1. Composition pharmaceutique comprenant un mélange :
(i) d'une protéine H-NOX liée de manière covalente au polyéthylène glycol (une protéine H-NOX PEGylatée), et
(ii) d'une protéine H-NOX non liée de manière covalente au polyéthylène glycol (une protéine H-NOX non PEGylatée),
dans laquelle à la fois la protéine H-NOX PEGylatée et la protéine H-NOX non PEGylatée sont chacune une protéine H-NOX trimérique comprenant trois monomères de H-NOX, dans laquelle chaque monomère de H-NOX comprend un domaine de H-NOX de *T. tengcongensis* et un domaine de trimérisation et
dans laquelle le domaine de H-NOX dans chaque monomère de H-NOX comprend une substitution de L144F.

2. Composition pharmaceutique selon la revendication 1, dans laquelle le domaine de H-NOX de *T. tengcongensis* dans chaque monomère de H-NOX comprend la séquence d'acides aminés SEQ ID NO : 6.

3. Composition pharmaceutique selon l'une quelconque des revendications 1-2, dans laquelle chaque monomère de H-NOX comprend le domaine de H-NOX de *T*. *tengcongensis* lié de manière covalente au domaine de trimérisation.

4. Composition pharmaceutique selon la revendication 3, dans laquelle, dans chaque monomère de H-NOX, l'extrémité C du domaine de H-NOX de *T. tengcongensis* est lié de manière covalente au domaine de trimérisation.

5. Composition pharmaceutique selon l'une quelconque des revendications 1-4, dans laquelle le domaine de trimérisation dans chaque monomère de H-NOX est un domaine foldon de la fibritine du bactériophage T4.

6. Composition pharmaceutique selon la revendication 5, dans laquelle le domaine foldon de la fibritine du bactériophage T4 comprend la séquence d'acides aminés SEQ ID NO : 4.

7. Composition pharmaceutique selon l'une quelconque des revendications 1-6, dans laquelle le domaine NOX est lié au domaine de trimérisation par le biais d'un lieur d'acides aminés dans chaque monomère de H-NOX.

8. Composition pharmaceutique selon la revendication 7, dans laquelle le lieur d'acides aminés fait trois, quatre, cinq, six, sept, huit, neuf ou dix acides aminés en longueur.

9. Composition pharmaceutique selon la revendication 1, dans laquelle chaque monomère de H-NOX comprend la séquence d'acides aminés SEQ ID NO : 8.

10. Composition pharmaceutique selon l'une quelconque des revendications 1-9, dans laquelle la protéine H-NOX PEGylatée comprend trois molécules de polyéthylène glycol (PEG) par monomère.

11. Composition pharmaceutique selon la revendication 10, dans laquelle chaque molécule de PEG présente un poids moléculaire de 5 KDa.

12. Composition pharmaceutique selon l'une quelconque des revendications 1-11, dans laquelle la composition pharmaceutique comprend en outre un excipient ou un diluant pharmaceutiquement acceptable.

13. Composition pharmaceutique telle que définie dans l'une quelconque des revendications 1-12 destinée à être utilisée dans un procédé pour traiter un coup, un accident ischémique transitoire ou un tissu hypoxique associé à une lésion à un organe ou à un membre chez un mammifère.

14. Composition pharmaceutique telle que définie dans l'une quelconque des revendications 1-12 destinée à être utilisée dans un procédé d'administration d'oxygène à un tissu hypoxique associé à une lésion à un organe ou à un membre chez un mammifère ou dans un procédé d'administration d'oxygène après un coup ou un accident ischémique transitoire chez un mammifère.

15. Composition pharmaceutique destinée à être utilisée selon la revendication 13 ou 14, dans laquelle la composition pharmaceutique est destinée à traiter ou à administrer de l'oxygène à un tissu hypoxique associé à une lésion à un organe ou à un membre et dans laquelle l'organe ou le membre est un cerveau, un cœur, un foie, un rein, un poumon, une rate, des intestins ou un pancréas.

16. Composition pharmaceutique destinée à être utilisée selon la revendication 13 ou 14, dans laquelle la composition pharmaceutique est destinée à traiter un coup ou à administrer de l'oxygène après un coup, dans laquelle le coup est un coup ischémique ou un coup hémorragique.

17. Composition pharmaceutique selon l'une quelconque des revendications 1-12 destinée à être utilisée dans un procédé de traitement d'une lésion cérébrale hypoxique chez un mammifère ou dans un procédé d'administration d'oxygène après une lésion cérébrale hypoxique chez un mammifère.

18. Composition pharmaceutique destinée à être utilisée selon l'une quelconque des revendications 13-17, dans laquelle le procédé comprend l'administration de la composition pharmaceutique au mammifère en combinaison avec une thérapie additionnelle, dans laquelle la thérapie additionnelle est un activateur tissulaire du plasminogène, un neuroprotecteur ou une recanalisation.

19. Composition pharmaceutique destinée à être utilisée selon l'une quelconque des revendications 13-18, dans laquelle le mammifère est un être humain.

20. Composition pharmaceutique destinée à être utilisée selon l'une quelconque des revendications 13-19, dans laquelle le procédé comprend l'administration de la composition pharmaceutique par administration orale, topique, intra-oculaire, intrathécale, intrapulmonaire, intratrachéale ou par aérosol ; ou par adsorption transdermique ou par les muqueuses ; ou par injection sous-cutanée, intraveineuse, intra-artérielle, intravésiculaire ou intramusculaire.
